# EUROPEAN PATENT SPECIFICATION

(11) **EP 1 305 434 B1**
(45) Date of publication and mention of the grant of the patent: **14.03.2007**
(21) Application number: 01959270.8
(22) Date of filing: 27.07.2001
(51) Int. Cl.: C12N 15/57, C12N 9/64, C07K 16/40, G01N 33/68, A61K 38/48, G06F 15/00, G06F 19/00

(54) **A HUMAN DISINTEGRIN PROTEIN**
HUMANE DISINTEGRINPROTEINE
PROTEINE DESINTEGRINE HUMAINE

(30) Priority: 28.07.2000 US 221838 P; 09.04.2001 US 282550 P
(43) Date of publication of application: 02.05.2003
(73) Proprietor: IMMUNEX CORPORATION, Thousand Oaks, CA 91320-1799 (US)
(72) Inventor: BLACK, Roy, A., Seattle, WA 98115 (US); POINDEXTER, Kurt, Seattle, WA 98103 (US); MOSLEY, Bruce, A., Seattle, WA 98155 (US); DUBOSE, Robert, F., Bellevue, WA 98006 (US); WILEY, Steven, R., Seattle, WA 98119 (US)
(74) Representative: Dörries, Hans Ulrich
(86) International application number: PCT/US2001/023709
(87) International publication number: WO 2002/010405

(56) References cited:
- WO-A-01/08396
- WO-A-01/66557
- WO-A-01/93983
- DATABASE EMBL [Online] Sequence ID HS388301, 20 March 1996 (1996-03-20) BOUILLAUD F: "Study of expressed sequences tags in adipose tissue 1995," Database accession no. N73388 XP002203867
- DATABASE EMBL [Online] 7 March 2000 (2000-03-07) STRAUSBERG R: "Transmembrane protein TMDC I precursor" Database accession no. AW511202 XP002203868
- DATABASE MEDLINE [Online] June 2000 (2000-06) ZHOU Q ET AL: "Contortrostatin, a dimeric disintegrin from Agkistrodon contortrix contortrix, inhibits breast cancer progression." Database accession no. NLM10966001 XP002203869 & BREAST CANCER RESEARCH AND TREATMENT. NETHERLANDS JUN 2000, vol. 61, no. 3, June 2000 (2000-06), pages 249-260, ISSN: 0167-6806
- NATH D ET AL: "Meltrin gamma(ADAM-9) mediates cellular adhesion through alpha(6)beta(1 )integrin, leading to a marked induction of fibroblast cell motility." JOURNAL OF CELL SCIENCE. ENGLAND JUN 2000, vol. 113 ( Pt 12), June 2000 (2000-06), pages 2319-2328, XP002203866 ISSN: 0021-9533
- KUNTZ I D ET AL: "STRUCTURE-BASED MOLECULAR DESIGN" ACCOUNTS OF CHEMICAL RESEARCH, AMERICAN CHEMICAL SOCIETY. WASHINGTON, US, vol. 27, no. 5, May 1994 (1994-05), pages 117-123, XP000885741 ISSN: 0001-4842

### SEQUENCE LISTING

<110> Immunex Corporation
   Poindexter, Kurt
   Black, Roy A.
   Mosley, Bruce
   Dubose, Robert F
   Wiley, Steve R
<120> A HUMAN DISINTEGRIN PROTEIN
<130> IMNX 3199-WO
<140> TO BE ASSIGNED
   <141> 2001-07-27
<150> US 60/221,838
   <151> 2000-07-28
<150> US 60/282,550
   <151> 2001-04-09
<160> 25
<170> PatentIn version 3.1
<210> 1
   <211> 66
   <212> PRT
   <213> Homo sapien
<400> 1
   Ile Leu Gln Ser Gly Val Glu Cys Arg Pro Lys Ala His Pro Glu Cys
   1               5                   10                  15
   Asp Ile Ala Glu Asn Cys Asn Gly Ser Ser Pro Glu Cys Gly Pro Asp
               20                  25                  30
   Ile Thr Leu Ile Asn Gly Leu Ser Cys Lys Asn Asn Lys Phe Ile Cys
           35                  40                  45
   Tyr Asp Gly Asp Cys His Asp Leu Asp Ala Arg Cys Glu Ser Val Phe
       50                  55                  60
   Gly Lys
   65
<210> 2
   <211> 156
   <212> DNA
   <213> homo sapiens
<220>
   <221> CDS
   <222> (2)..(154)
   <223>
<400> 2
   c gga gca aaa tgt tat aaa gga ctg tgc tgc aaa gac tgt caa att tta      49
     Gly Ala Lys Cys Tyr Lys Gly Leu Cys Cys Lys Asp Cys Gln Ile Leu
     1               5                   10                  15
   caa tca ggc gtt gaa tgt agg ccg aaa gca cat cct gaa tgt gac atc        97
   Gln Ser Gly Val Glu Cys Arg Pro Lys Ala His Pro Glu Cys Asp Ile
               20                  25                  30
   gct gaa aat tgt aat gga agc tca cca gaa tgt ggt cct gac ata act       145
   Ala Glu Asn Cys Asn Gly Ser Ser Pro Glu Cys Gly Pro Asp Ile Thr
           35                  40                  45
   tta atc aat gg                                                        156
   Leu Ile Asn
       50
<210> 3
   <211> 51
   <212> PRT
   <213> homo sapiens
<400> 3
   Gly Ala Lys Cys Tyr Lys Gly Leu Cys Cys Lys Asp Cys Gln Ile Leu
   1 5 10 15
   Gln Ser Gly Val Glu Cys Arg Pro Lys Ala His Pro Glu Cys Asp Ile
   20 25 30
   Ala Glu Asn Cys Asn Gly Ser Ser Pro Glu Cys Gly Pro Asp Ile Thr
   35 40 45
   Leu Ile Asn
   50
<210> 4
   <211> 96
   <212> PRT
   <213> homo spiens
<400> 4
   Cys Gly Pro Ala Ser Cys Cys Asp Phe Arg Thr Cys Val Leu Lys Asp
   1 5 10 15
   Gly Ala Lys Cys Tyr Lys Gly Leu Cys Cys Lys Asp Cys Gln Ile Leu
   20 25 30
   Gln Ser Gly Val Glu Cys Arg Pro Lys Ala His Pro Glu Cys Asp Ile
   35 40 45
   Ala Glu Asn Cys Asn Gly Ser Ser Pro Glu Cys Gly Pro Asp Ile Thr
   50 55 60
   Leu Ile Asn Gly Leu Ser Cys Lys Asn Asn Lys Phe Ile Cys Tyr Asp
   65 70 75 80
   Gly Asp Cys His Asp Leu Asp Ala Arg Cys Glu Ser Val Phe Gly Lys
   85 90 95
<210> 5
   <211> 1532
   <212> DNA
   <213> homo sapiens
<220>
   <221> CDS
   <222> (32)..(1432)
   <223>
<400> 5
   tttagcggcc gcgaattcgc ccttcaccca g atg ctg gca ctc agt ctg gga         52
                                      Met Leu Ala Leu Ser Leu Gly
                                      1               5
   ata tca tat gac gac cca aag aaa tgt caa tgt tca gaa tcc acc tgt       100
   Ile Ser Tyr Asp Asp Pro Lys Lys Cys Gln Cys Ser Glu Ser Thr Cys
           10                  15                  20
   ata atg aat cca gaa gtt gtg caa tcc aat ggt gtg aag act ttt agc       148
   Ile Met Asn Pro Glu Val Val Gln Ser Asn Gly Val Lys Thr Phe Ser
       25                  30                  35
   agt tgc agt ttg agg agc ttt caa aat ttc att tca aat gtg ggt gtc       196
   Ser Cys Ser Leu Arg Ser Phe Gln Asn Phe Ile Ser Asn Val Gly Val
   40                  45                  50                  55
   aaa tgt ctt cag aat aag cca caa atg caa aaa aaa tct ccg aaa cca       244
   Lys Cys Leu Gln Asn Lys Pro Gln Met Gln Lys Lys Ser Pro Lys Pro
                    60                  65                  70
   gtc tgt ggc aat ggc aga ttg gag gga aat gaa atc tgt gat tgt ggt      292
   Val Cys Gly Asn Gly Arg Leu Glu Gly Asn Glu Ile Cys Asp Cys Gly
               75                  80                  85
   act gag gct caa tgt gga cct gca agc tgt tgt gat ttt cga act tgt      340
   Thr Glu Ala Gln Cys Gly Pro Ala Ser Cys Cys Asp Phe Arg Thr Cys
           90                  95                  100
   gta ctg aaa gac gga gca aaa tgt tat aaa gga ctg tgc tgc aaa gac      388
   Val Leu Lys Asp Gly Ala Lys Cys Tyr Lys Gly Leu Cys Cys Lys Asp
       105                 110                 115
   tgt caa att tta caa tca ggc gtt gaa tgt agg ccg aaa gca cat cct      436
   Cys Gln Ile Leu Gln Ser Gly Val Glu Cys Arg Pro Lys Ala His Pro
   120                 125                 130                 135
   gaa tgt gac atc gct gaa aat tgt aat gga agc tca cca gaa tgt ggt      484
   Glu Cys Asp Ile Ala Glu Asn Cys Asn Gly Ser Ser Pro Glu Cys Gly
                   140                 145                 150
   cct gac ata act tta atc aat gga ctt tca tgc aaa aat aat aag ttt      532
   Pro Asp Ile Thr Leu Ile Asn Gly Leu Ser Cys Lys Asn Asn Lys Phe
               155                 160                 165
   att tgt tat gac gga gac tgc cat gat ctc gat gca cgt tgt gag agt      580
   Ile Cys Tyr Asp Gly Asp Cys His Asp Leu Asp Ala Arg Cys Glu Ser
           170                 175                 180
   gta ttt gga aaa ggt tca aga aat gct cca ttt gcc tgc tat gaa gaa      628
   Val Phe Gly Lys Gly Ser Arg Asn Ala Pro Phe Ala Cys Tyr Glu Glu
       185                 190                 195
   ata caa tct caa tca gac aga ttt ggg aac tgt ggt agg gat aga aat      676
   Ile Gln Ser Gln Ser Asp Arg Phe Gly Asn Cys Gly Arg Asp Arg Asn
   200                 205                 210                 215
   aac aaa tat gtg ttc tgt gga tgg agg aat ctt ata tgt gga aga tta      724
   Asn Lys Tyr Val Phe Cys Gly Trp Arg Asn Leu Ile Cys Gly Arg Leu
                   220                 225                 230
   gtt tgt acc tac cct act cga aag cct ttc cat caa gaa aat ggt gat      772
   Val Cys Thr Tyr Pro Thr Arg Lys Pro Phe His Gln Glu Asn Gly Asp
               235                 240                 245
   gtg att tat gct ttc gta cga gat tct gta tgc ata act gta gac tac      820
   Val Ile Tyr Ala Phe Val Arg Asp Ser Val Cys Ile Thr Val Asp Tyr
           250                 255                 260
   aaa ttg cct cga aca gtt cca gat cca ctg gct gtc aaa aat ggc tct      868
   Lys Leu Pro Arg Thr Val Pro Asp Pro Leu Ala Val Lys Asn Gly Ser
       265                 270                 275
   cag tgt gat att ggg agg gtt tgt gta aat cgt gaa tgt gta gaa tca      916
   Gln Cys Asp Ile Gly Arg Val Cys Val Asn Arg Glu Cys Val Glu Ser
   280                 285                 290                 295
   agg ata att aag gct tca gca cat gtt tgt tca caa cag tgt tct gga      964
   Arg Ile Ile Lys Ala Ser Ala His Val Cys Ser Gln Gln Cys Ser Gly
                   300                 305                 310
   cat gga gtg tgt gat tcc aga aac aag tgc cat tgt tcg cca ggc tat     1012
   His Gly Val Cys Asp Ser Arg Asn Lys Cys His Cys Ser Pro Gly Tyr
               315                 320                 325
   aag cct cca aac tgc caa ata cgt tcc aaa gga ttt tcc ata ttt cct     1060
   Lys Pro Pro Asn Cys Gln Ile Arg Ser Lys Gly Phe Ser Ile Phe Pro
           330                 335                 340
   gag gaa gat atg ggt tca atc atg gaa aga gca tct ggg aag act gaa     1108
   Glu Glu Asp Met Gly Ser Ile Met Glu Arg Ala Ser Gly Lys Thr Glu
       345                 350                 355
   aac acc tgg ctt cta ggt ttc ctc att gct ctt cct att ctc att gta      1156
   Asn Thr Trp Leu Leu Gly Phe Leu Ile Ala Leu Pro Ile Leu Ile Val
   360                 365                 370                 375
   aca acc gca ata gtt ttg gca agg aaa cag ttg aaa aag tgg ttc gcc      1204
   Thr Thr Ala Ile Val Leu Ala Arg Lys Gln Leu Lys Lys Trp Phe Ala
                   380                 385                 390
   aag gaa gag gaa ttc cca agt agc gaa tct aaa tcg gaa ggt agc aca      1252
   Lys Glu Glu Glu Phe Pro Ser Ser Glu Ser Lys Ser Glu Gly Ser Thr
               395                 400                 405
   cag aca tat gcc agc caa tcc agc tca gaa ggc agc act cag aca tat      1300
   Gln Thr Tyr Ala Ser Gln Ser Ser Ser Glu Gly Ser Thr Gln Thr Tyr
           410                 415                 420
   gcc agc caa acc aga tca gaa agc agc agt caa gct gat act agc aaa      1348
   Ala Ser Gln Thr Arg Ser Glu Ser Ser Ser Gln Ala Asp Thr Ser Lys
       425                 430                 435
   tcc aaa tca gaa gat agt gct gaa gca tat act agc aga tcc aaa tca      1396
   Ser Lys Ser Glu Asp Ser Ala Glu Ala Tyr Thr Ser Arg Ser Lys Ser
   440                 445                 450                 455
   cag gac agt acc caa aca caa agc agt agt aac tag tgatccttca           1442
   Gln Asp Ser Thr Gln Thr Gln Ser Ser Ser Asn
                   460                 465
   gaaggcaacg gataacatcg agaagggcga attcgtttaa acctgcagga ctagtccctt    1502
   tagtgagggt taattctgag cttggcgtaa                                     1532
<210> 6
   <211> 466
   <212> PRT
   <213> homo sapiens
<400> 6
   Met Leu Ala Leu Ser Leu Gly Ile Ser Tyr Asp Asp Pro Lys Lys Cys
   1               5                   10                  15
   Gln Cys Ser Glu Ser Thr Cys Ile Met Asn Pro Glu Val Val Gln Ser
               20                  25                  30
   Asn Gly Val Lys Thr Phe Ser Ser Cys Ser Leu Arg Ser Phe Gln Asn
           35                  40                  45
   Phe Ile Ser Asn Val Gly Val Lys Cys Leu Gln Asn Lys Pro Gln Met
       50                  55                  60
   Gln Lys Lys Ser Pro Lys Pro Val Cys Gly Asn Gly Arg Leu Glu Gly
   65                  70                  75                  80
   Asn Glu Ile Cys Asp Cys Gly Thr Glu Ala Gln Cys Gly Pro Ala Ser
                   85                  90                  95
   Cys Cys Asp Phe Arg Thr Cys Val Leu Lys Asp Gly Ala Lys Cys Tyr
               100                 105                 110
   Lys Gly Leu Cys Cys Lys Asp Cys Gln Ile Leu Gln Ser Gly Val Glu
           115                 120                 125
   Cys Arg Pro Lys Ala His Pro Glu Cys Asp Ile Ala Glu Asn Cys Asn
       130                 135                 140
   Gly Ser Ser Pro Glu Cys Gly Pro Asp Ile Thr Leu Ile Asn Gly Leu
   145                 150                 155                 160
   Ser Cys Lys Asn Asn Lys Phe Ile Cys Tyr Asp Gly Asp Cys His Asp
                   165                 170                 175
   Leu Asp Ala Arg Cys Glu Ser Val Phe Gly Lys Gly Ser Arg Asn Ala
               180                 185                   190
   Pro Phe Ala Cys Tyr Glu Glu Ile Gln Ser Gln Ser Asp Arg Phe Gly
           195                 200                 205
   Asn Cys Gly Arg Asp Arg Asn Asn Lys Tyr Val Phe Cys Gly Trp Arg
       210                 215                 220
   Asn Leu Ile Cys Gly Arg Leu Val Cys Thr Tyr Pro Thr Arg Lys Pro
   225                 230                 235                 240
   Phe His Gln Glu Asn Gly Asp Val Ile Tyr Ala Phe Val Arg Asp Ser
                   245                 250                 255
   Val Cys Ile Thr Val Asp Tyr Lys Leu Pro Arg Thr Val Pro Asp Pro
               260                 265                 270
   Leu Ala Val Lys Asn Gly Ser Gln Cys Asp Ile Gly Arg Val Cys Val
           275                 280                 285
   Asn Arg Glu Cys Val Glu Ser Arg Ile Ile Lys Ala Ser Ala His Val
       290                 295                 300
   Cys Ser Gln Gln Cys Ser Gly His Gly Val Cys Asp Ser Arg Asn Lys
   305                 310                 315                 320
   Cys His Cys Ser Pro Gly Tyr Lys Pro Pro Asn Cys Gln Ile Arg Ser
                   325                 330                 335
   Lys Gly Phe Ser Ile Phe Pro Glu Glu Asp Met Gly Ser Ile Met Glu
               340                 345                 350
   Arg Ala Ser Gly Lys Thr Glu Asn Thr Trp Leu Leu Gly Phe Leu Ile
           355                 360                 365
   Ala Leu Pro Ile Leu Ile Val Thr Thr Ala Ile Val Leu Ala Arg Lys
       370                 375                 380
   Gln Leu Lys Lys Trp Phe Ala Lys Glu Glu Glu Phe Pro Ser Ser Glu
   385                 390                 395                 400
   Ser Lys Ser Glu Gly Ser Thr Gln Thr Tyr Ala Ser Gln Ser Ser Ser
                   405                 410                 415
   Glu Gly Ser Thr Gln Thr Tyr Ala Ser Gln Thr Arg Ser Glu Ser Ser
               420                 425                 430
   Ser Gln Ala Asp Thr Ser Lys Ser Lys Ser Glu Asp Ser Ala Glu Ala
           435                 440                 445
   Tyr Thr Ser Arg Ser Lys Ser Gln Asp Ser Thr Gln Thr Gln Ser Ser
       450                 455                 460
   Ser Asn
   465
<210> 7
   <211> 1240
   <212> DNA
   <213> homo sapiens
<220>
   <221> CDS
   <222> (82)..(1140)
   <223>
<400> 7
   attgaattta gcggccgcga attcgccctt cacccagatg ctggcactca gtctgggaat      60
   atcatatgac gacccaaaga a atg tca atg ttc aga atc cac ctg tat aat       111
                           Met Ser Met Phe Arg Ile His Leu Tyr Asn
                           1               5                   10
   gaa tcc aga agt tgt caa tgt gga cct gca agc tgt tgt gat ttt cga      159
   Glu Ser Arg Ser Cys Gln Cys Gly Pro Ala Ser Cys Cys Asp Phe Arg
                   15                  20                  25
   act tgt gta ctg aaa gac gga gca aaa tgt tat aaa gga ctg tgc tgc      207
   Thr Cys Val Leu Lys Asp Gly Ala Lys Cys Tyr Lys Gly Leu Cys Cys
               30                  35                  40
   aaa gac tgt caa att tta caa tca ggc gtt gaa tgt agg ccg aaa gca      255
   Lys Asp Cys Gln Ile Leu Gln Ser Gly Val Glu Cys Arg Pro Lys Ala
           45                  50                  55
   cat cct gaa tgt gac atc gct gaa aat tgt aat gga agc tca cca gaa      303
   His Pro Glu Cys Asp Ile Ala Glu Asn Cys Asn Gly Ser Ser Pro Glu
       60                  65                  70
   tgt ggt cct gac ata act tta atc aat gga ctt tca tgc aaa aat aat      351
   Cys Gly Pro Asp Ile Thr Leu Ile Asn Gly Leu Ser Cys Lys Asn Asn
   75                  80                  85                  90
   aag ttt att tgt tat gac gga gac tgc cat gat ctc gat gca cgt tgt      399
   Lys Phe Ile Cys Tyr Asp Gly Asp Cys His Asp Leu Asp Ala Arg Cys
                   95                  100                 105
   gag agt gta ttt gga aaa ggt tca aga aat gct cca ttt gcc tgc tat      447
   Glu Ser Val Phe Gly Lys Gly Ser Arg Asn Ala Pro Phe Ala Cys Tyr
               110                 115                 120
   gaa gaa ata caa tct caa tca gac aga ttt ggg aac tgt ggt agg gat      495
   Glu Glu Ile Gln Ser Gln Ser Asp Arg Phe Gly Asn Cys Gly Arg Asp
           125                 130                 135
   aga aat aac aaa tat gtg ttc tgt gga tgg agg aat ctt ata tgt gga      543
   Arg Asn Asn Lys Tyr Val Phe Cys Gly Trp Arg Asn Leu Ile Cys Gly
       140                 145                 150
   aga tta gtt tgt acc tac cct act cga aag cct ttc cat caa gaa aat      591
   Arg Leu Val Cys Thr Tyr Pro Thr Arg Lys Pro Phe His Gln Glu Asn
   155                 160                 165                 170
   ggt gat gtg att tat gct ttc gta cga gat tct gta tgc ata act gta      639
   Gly Asp Val Ile Tyr Ala Phe Val Arg Asp Ser Val Cys Ile Thr Val
                   175                 180                 185
   gac tac aaa ttg cct cga aca gtt cca gat cca ctg gct gtc aaa aat      687
   Asp Tyr Lys Leu Pro Arg Thr Val Pro Asp Pro Leu Ala Val Lys Asn
               190                 195                 200
   ggc tct cag tgt gat att ggg agg gtt tgt gta aat cgt gaa tgt gta      735
   Gly Ser Gln Cys Asp Ile Gly Arg Val Cys Val Asn Arg Glu Cys Val
           205                 210                 215
   gaa tca agg ata att aag gct tca gca cat gtt tgt tca caa cag tgt      783
   Glu Ser Arg Ile Ile Lys Ala Ser Ala His Val Cys Ser Gln Gln Cys
       220                 225                 230
   tct gga cat gga gtg tgt gat tcc aga aac aag tgc cat tgt tcg cca      831
   Ser Gly His Gly Val Cys Asp Ser Arg Asn Lys Cys His Cys Ser Pro
   235                 240                 245                 250
   ggc tat aag cct cca aac tgc caa ata cgt tcc aaa gga ttt tcc ata      879
   Gly Tyr Lys Pro Pro Asn Cys Gln Ile Arg Ser Lys Gly Phe Ser Ile
                   255                 260                 265
   ttt cct gag gaa gat atg ggt tca atc atg gaa aga gca tct ggg aag      927
   Phe Pro Glu Glu Asp Met Gly Ser Ile Met Glu Arg Ala Ser Gly Lys
               270                 275                 280
   act gaa aac acc tgg ctt cta ggt ttc ctc att gct ctt cct att ctc      975
   Thr Glu Asn Thr Trp Leu Leu Gly Phe Leu Ile Ala Leu Pro Ile Leu
           285                 290                 295
   att gta aca acc gca ata gtt ttg gca agg aaa cag ttg aaa aag tgg      1023
   Ile Val Thr Thr Ala Ile Val Leu Ala Arg Lys Gln Leu Lys Lys Trp
       300                 305                 310
   ttc gcc aag gaa gag gaa ttc cca agt agc gaa tct aaa tcg gaa gat      1071
   Phe Ala Lys Glu Glu Glu Phe Pro Ser Ser Glu Ser Lys Ser Glu Asp
   315                 320                 325                 330
   agt gct gaa gca tat act agc aga tcc aaa tca cag gac agt acc caa      1119
   Ser Ala Glu Ala Tyr Thr Ser Arg Ser Lys Ser Gln Asp Ser Thr Gln
                   335                 340                 345
   aca caa agc agt agt aac tag tgatccttca gaaggcaacg gataacatcg         1170
   Thr Gln Ser Ser Ser Asn
               350
   agaagggcga attcgtttaa acctgcagga ctagtccctt tagtgagggt taattctgag   1230
   cttggcgtaa                                                           1240
<210> 8
   <211> 352
   <212> PRT
   <213> homo sapiens
<400> 8
   Met Ser Met Phe Arg Ile His Leu Tyr Asn Glu Ser Arg Ser Cys Gln
   1 5 10 15
   Cys Gly Pro Ala Ser Cys Cys Asp Phe Arg Thr Cys Val Leu Lys Asp
   20 25 30
   Gly Ala Lys Cys Tyr Lys Gly Leu Cys Cys Lys Asp Cys Gln Ile Leu
   35 40 45
   Gln Ser Gly Val Glu Cys Arg Pro Lys Ala His Pro Glu Cys Asp Ile
   50 55 60
   Ala Glu Asn Cys Asn Gly Ser Ser Pro Glu Cys Gly Pro Asp Ile Thr
   65 70 75 80
   Leu Ile Asn Gly Leu Ser Cys Lys Asn Asn Lys Phe Ile Cys Tyr Asp
   85 90 95
   Gly Asp Cys His Asp Leu Asp Ala Arg Cys Glu Ser Val Phe Gly Lys
   100 105 110
   Gly Ser Arg Asn Ala Pro Phe Ala Cys Tyr Glu Glu Ile Gln Ser Gln
   115 120 125
   Ser Asp Arg Phe Gly Asn Cys Gly Arg Asp Arg Asn Asn Lys Tyr Val
   130 135 140
   Phe Cys Gly Trp Arg Asn Leu Ile Cys Gly Arg Leu Val Cys Thr Tyr
   145 150 155 160
   Pro Thr Arg Lys Pro Phe His Gln Glu Asn Gly Asp Val Ile Tyr Ala
   165 170 175
   Phe Val Arg Asp Ser Val Cys Ile Thr Val Asp Tyr Lys Leu Pro Arg
   180 185 190
   Thr Val Pro Asp Pro Leu Ala Val Lys Asn Gly Ser Gln Cys Asp Ile
   195 200 205
   Gly Arg Val Cys Val Asn Arg Glu Cys Val Glu Ser Arg Ile Ile Lys
   210 215 220
   Ala Ser Ala His Val Cys Ser Gln Gln Cys Ser Gly His Gly Val Cys
   225 230 235 240
   Asp Ser Arg Asn Lys Cys His Cys Ser Pro Gly Tyr Lys Pro Pro Asn
   245 250 255
   Cys Gln Ile Arg Ser Lys Gly Phe Ser Ile Phe Pro Glu Glu Asp Met
   260 265 270
   Gly Ser Ile Met Glu Arg Ala Ser Gly Lys Thr Glu Asn Thr Trp Leu
   275 280 285
   Leu Gly Phe Leu Ile Ala Leu Pro Ile Leu Ile Val Thr Thr Ala Ile
   290 295 300
   Val Leu Ala Arg Lys Gln Leu Lys Lys Trp Phe Ala Lys Glu Glu Glu
   305 310 315 320
   Phe Pro Ser Ser Glu Ser Lys Ser Glu Asp Ser Ala Glu Ala Tyr Thr
   325 330 335
   Ser Arg Ser Lys Ser Gln Asp Ser Thr Gln Thr Gln Ser Ser Ser Asn
   340 345 350
<210> 9
   <211> 1290
   <212> DNA
   <213> homo sapiens
<220>
   <221> CDS
   <222> (32)..(1021)
   <223>
<400> 9
   tttagcggcc gcgaattcgc ccttcaccca g atg ctg gca ctc agt ctg gga       52
                                      Met Leu Ala Leu Ser Leu Gly
                                      1               5
   ata tca tat gac gac cca aag aaa tgt caa tgt tca gaa tcc acc tgt     100
   Ile Ser Tyr Asp Asp Pro Lys Lys Cys Gln Cys Ser Glu Ser Thr Cys
           10                  15                  20
   ata atg aat cca gaa gtt gtg caa tcc aat ggt gtg aag act ttt agc     148
   Ile Met Asn Pro Glu Val Val Gln Ser Asn Gly Val Lys Thr Phe Ser
       25                  30                  35
   agt tgc agt ttg agg agc ttt caa aat ttc att tca aat gtg ggt gtc     196
   Ser Cys Ser Leu Arg Ser Phe Gln Asn Phe Ile Ser Asn Val Gly Val
   40                  45                  50                  55
   aaa tgt ctt cag aat aag cca caa atg caa aaa aaa tct ccg aaa cca     244
   Lys Cys Leu Gln Asn Lys Pro Gln Met Gln Lys Lys Ser Pro Lys Pro
                   60                  65                  70
   gtc tgt ggc aat ggc aga ttg gag gga aat gaa atc tgt gat tgt ggt     292
   Val Cys Gly Asn Gly Arg Leu Glu Gly Asn Glu Ile Cys Asp Cys Gly
               75                  80                  85
   act gag gct caa tgt gga cct gca agc tgt tgt gat ttt cga act tgt     340
   Thr Glu Ala Gln Cys Gly Pro Ala Ser Cys Cys Asp Phe Arg Thr Cys
           90                  95                  100
   gta ctg aaa gac gga gca aaa tgt tat aaa gga ctg tgc tgc aaa gac     388
   Val Leu Lys Asp Gly Ala Lys Cys Tyr Lys Gly Leu Cys Cys Lys Asp
       105                 110                 115
   tgt caa att tta caa tca ggc gtt gaa tgt agg ccg aaa gca cat cct     436
   Cys Gln Ile Leu Gln Ser Gly Val Glu Cys Arg Pro Lys Ala His Pro
   120                 125                 130                 135
   gaa tgt gac atc gct gaa aat tgt aat gga agc tca cca gaa tgt ggt     484
   Glu Cys Asp Ile Ala Glu Asn Cys Asn Gly Ser Ser Pro Glu Cys Gly
                   140                 145                 150
   cct gac ata act tta atc aat gga ctt tca tgc aaa aat aat aag ttt     532
   Pro Asp Ile Thr Leu Ile Asn Gly Leu Ser Cys Lys Asn Asn Lys Phe
               155                 160                 165
   att tgt tat gac gga gac tgc cat gat ctc gat gca cgt tgt gag agt      580
   Ile Cys Tyr Asp Gly Asp Cys His Asp Leu Asp Ala Arg Cys Glu Ser
           170                 175                 180
   gta ttt gga aaa ggt tca aga aat gct cca ttt gcc tgc tat gaa gaa      628
   Val Phe Gly Lys Gly Ser Arg Asn Ala Pro Phe Ala Cys Tyr Glu Glu
       185                 190                 195
   ata caa tct caa tca gac aga ttt ggg aac tgt ggt agg gat aga aat      676
   Ile Gln Ser Gln Ser Asp Arg Phe Gly Asn Cys Gly Arg Asp Arg Asn
   200                 205                 210                 215
   aac aaa tat gtg ttc tgt gga tgg agg aat ctt ata tgt gga aga tta      724
   Asn Lys Tyr Val Phe Cys Gly Trp Arg Asn Leu Ile Cys Gly Arg Leu
                   220                 225                 230
   gtt tgt acc tac cct act cga aag cct ttc cat caa gaa aat ggt gat      772
   Val Cys Thr Tyr Pro Thr Arg Lys Pro Phe His Gln Glu Asn Gly Asp
               235                 240                 245
   gtg att tat gct ttc gta cga gat tct gta tgc ata act gta gac tac      820
   Val Ile Tyr Ala Phe Val Arg Asp Ser Val Cys Ile Thr Val Asp Tyr
           250                 255                 260
   aaa ttg cct cga aca gtt cca gat cca ctg gct gtc aaa aat ggc tct      868
   Lys Leu Pro Arg Thr Val Pro Asp Pro Leu Ala Val Lys Asn Gly Ser
       265                 270                 275
   cag tgt gat att ggg agg gtt tgt gta aat cgt gaa tgt gta gaa tca      916
   Gln Cys Asp Ile Gly Arg Val Cys Val Asn Arg Glu Cys Val Glu Ser
   280                 285                 290                 295
   agg ata att aag gct tca gca cat gtt tgt tca caa cag tgt tct gga      964
   Arg Ile Ile Lys Ala Ser Ala His Val Cys Ser Gln Gln Cys Ser Gly
                   300                 305                 310
   cat gga gtg caa tca tgg aaa gag cat ctg gga aga ctg aaa gca cct      1012
   His Gly Val Gln Ser Trp Lys Glu His Leu Gly Arg Leu Lys Ala Pro
               315                 320                 325
   ggc ttc tag gtttcctcat tgctcttcct attctcattg taacaaccgc              1061
   Gly Phe
   aatagttttg gcaaggaaac agttgaaaaa gtggttcgcc aaggaagagg aattcccaag   1121
   tagcgaatcc aaatcagaag atagtgctga agcatatact agcagatcca aatcacagga   1181
   cagtacccaa acacaaagca gtagtaacta gtgatccttc agaaggcaac ggataacatc   1241
   gagaagggcg aattcgttta aacctgcagg actagtccct ttagtgagg                1290
<210> 10
   <211> 329
   <212> PRT
   <213> homo sapiens
<400> 10
   Met Leu Ala Leu Ser Leu Gly Ile Ser Tyr Asp Asp Pro Lys Lys Cys
   1 5 10 15
   Gln Cys Ser Glu Ser Thr Cys Ile Met Asn Pro Glu Val Val Gln Ser
   20 25 30
   Asn Gly Val Lys Thr Phe Ser Ser Cys Ser Leu Arg Ser Phe Gln Asn
   35 40 45
   Phe Ile Ser Asn Val Gly Val Lys Cys Leu Gln Asn Lys Pro Gln Met
   50 55 60
   Gln Lys Lys Ser Pro Lys Pro Val Cys Gly Asn Gly Arg Leu Glu Gly
   65 70 75 80
   Asn Glu Ile Cys Asp Cys Gly Thr Glu Ala Gln Cys Gly Pro Ala Ser
   85 90 95
   Cys Cys Asp Phe Arg Thr Cys Val Leu Lys Asp Gly Ala Lys Cys Tyr
   100 105 110
   Lys Gly Leu Cys Cys Lys Asp Cys Gln Ile Leu Gln Ser Gly Val Glu
   115 120 125
   Cys Arg Pro Lys Ala His Pro Glu Cys Asp Ile Ala Glu Asn Cys Asn
   130 135 140
   Gly Ser Ser Pro Glu Cys Gly Pro Asp Ile Thr Leu Ile Asn Gly Leu
   145 150 155 160
   Ser Cys Lys Asn Asn Lys Phe Ile Cys Tyr Asp Gly Asp Cys His Asp
   165 170 175
   Leu Asp Ala Arg Cys Glu Ser Val Phe Gly Lys Gly Ser Arg Asn Ala
   180 185 190
   Pro Phe Ala Cys Tyr Glu Glu Ile Gln Ser Gln Ser Asp Arg Phe Gly
   195 200 205
   Asn Cys Gly Arg Asp Arg Asn Asn Lys Tyr Val Phe Cys Gly Trp Arg
   210 215 220
   Asn Leu Ile Cys Gly Arg Leu Val Cys Thr Tyr Pro Thr Arg Lys Pro
   225 230 235 240
   Phe His Gln Glu Asn Gly Asp Val Ile Tyr Ala Phe Val Arg Asp Ser
   245 250 255
   Val Cys Ile Thr Val Asp Tyr Lys Leu Pro Arg Thr Val Pro Asp Pro
   260 265 270
   Leu Ala Val Lys Asn Gly Ser Gln Cys Asp Ile Gly Arg Val Cys Val
   275 280 285
   Asn Arg Glu Cys Val Glu Ser Arg Ile Ile Lys Ala Ser Ala His Val
   290 295 300
   Cys Ser Gln Gln Cys Ser Gly His Gly Val Gln Ser Trp Lys Glu His
   305 310 315 320
   Leu Gly Arg Leu Lys Ala Pro Gly Phe
   325
<210> 11
   <211> 5
   <212> PRT
   <213> Artificial linker moiety
<400> 11
   Gly Gly Gly Gly Ser
   1 5
<210> 12
   <211> 6
   <212> PRT
   <213> Artificial linker moiety
<220>
   <221> MISC_FEATURE
   <222> (6)..(6)
   <223> X is one or more repeats of GGGGS
<400> 12
   Gly Gly Gly Gly Ser Xaa
   1 5
<210> 13
   <211> 12
   <212> PRT
   <213> Artificial linker moiety
<400> 13
   Gly Lys Ser Ser Gly Ser Gly Ser Glu Ser Lys Ser
   1 5 10
<210> 14
   <211> 14
   <212> PRT
   <213> Artificial linker moiety
<400> 14
   Gly Ser Thr Ser Gly Ser Gly Lys Ser Ser Glu Gly Lys Gly
   1 5 10
<210> 15
   <211> 18
   <212> PRT
   <213> Artificial linker moiety
<400> 15
   Gly Ser Thr Ser Gly Ser Gly Lys Ser Ser Glu Gly Ser Gly Ser Thr
   1 5 10 15
   Lys Gly
<210> 16
   <211> 18
   <212> PRT
   <213> Artificial linker moiety
<400> 16
   Gly Ser Thr Ser Gly Ser Gly Lys Pro Gly Ser Gly Glu Gly Ser Thr
   1               5                   10                  15
   Lys Gly
<210> 17
   <211> 14
   <212> PRT
   <213> Artificial linker moiety
<400> 17
   Glu Gly Lys Ser Ser Gly Ser Gly Ser Glu Ser Lys Glu Phe
   1               5                   10
<210> 18
   <211> 5
   <212> PRT
   <213> Artificial localization sequence
<400> 18
   Lys Lys Lys Arg Lys
   1               5
<210> 19
   <211> 26
   <212> PRT
   <213> Artificial localization sequence
<400> 19
   Met Leu Arg Thr Ser Ser Leu Phe Thr Arg Arg Val Gln Pro Ser Leu
   1               5                   10                  15
   Phe Arg Asn Ile Leu Arg Leu Gln Ser Thr
               20                  25
<210> 20
   <211> 4
   <212> PRT
   <213> Artificial localization sequence
<400> 20
   Lys Asp Glu Leu
   1
<210> 21
   <211> 4
   <212> PRT
   <213> Artificial localization sequence
<220>
   <221> MISC_FEATURE
   <222> (4)..(4)
   <223> X at residue 4 is any amino acid
<400> 21
   Cys Ala Ala Xaa
   1
<210> 22
   <211> 4
   <212> PRT
   <213> Artificial localization sequence
<220>
   <221> MISC_FEATURE
   <222> (3)..(4)
   <223> X at residue 3 and 4 can be any amino acid
<400> 22
   Cys Cys Xaa Xaa
   1
<210> 23
   <211> 819
   <212> PRT
   <213> homo sapien
<400> 23
   Met Gly Ser Gly Ala Arg Phe Pro Ser Gly Thr Leu Arg Val Arg Trp
   1               5                   10                  15
   Leu Leu Leu Leu Gly Leu Val Gly Pro Val Leu Gly Ala Ala Arg Pro
               20                  25                  30
   Gly Phe Gln Gln Thr Ser His Leu Ser Ser Tyr Glu Ile Ile Thr Pro
           35                  40                  45
   Trp Arg Leu Thr Arg Glu Arg Arg Glu Ala Pro Arg Pro Tyr Ser Lys
       50                  55                  60
   Gln Val Ser Tyr Val Ile Gln Ala Glu Gly Lys Glu His Ile Ile His
   65                  70                  75                  80
   Leu Glu Arg Asn Lys Asp Leu Leu Pro Glu Asp Phe Val Val Tyr Thr
                   85                  90                  95
   Tyr Asn Lys Glu Gly Thr Leu Ile Thr Asp His Pro Asn Ile Gln Asn
               100                 105                 110
   His Cys His Tyr Arg Gly Tyr Val Glu Gly Val His Asn Ser Ser Ile
           115                 120                 125
   Ala Leu Ser Asp Cys Phe Gly Leu Arg Gly Leu Leu His Leu Glu Asn
       130                 135                 140
   Ala Ser Tyr Gly Ile Glu Pro Leu Gln Asn Ser Ser His Phe Glu His
   145                 150                 155                 160
   Ile Ile Tyr Arg Met Asp Asp Val Tyr Lys Glu Pro Leu Lys Cys Gly
   165 170 175
   Val Ser Asn Lys Asp Ile Glu Lys Glu Thr Ala Lys Asp Glu Glu Glu
   180 185 190
   Glu Pro Pro Ser Met Thr Gln Leu Leu Arg Arg Arg Arg Ala Val Leu
   195 200 205
   Pro Gln Thr Arg Tyr Val Glu Leu Phe Ile Val Val Asp Lys Glu Arg
   210 215 220
   Tyr Asp Met Met Gly Arg Asn Gln Thr Ala Val Arg Glu Glu Met Ile
   225 230 235 240
   Leu Leu Ala Asn Tyr Leu Asp Ser Met Tyr Ile Met Leu Asn Ile Arg
   245 250 255
   Ile Val Leu Val Gly Leu Glu Ile Trp Thr Asn Gly Asn Leu Ile Asn
   260 265 270
   Ile Val Gly Gly Ala Gly Asp Val Leu Gly Asn Phe Val Gln Trp Arg
   275 280 285
   Glu Lys Phe Leu Ile Thr Arg Arg Arg His Asp Ser Ala Gln Leu Val
   290 295 300
   Leu Lys Lys Gly Phe Gly Gly Thr Ala Gly Met Ala Phe Val Gly Thr
   305 310 315 320
   Val Cys Ser Arg Ser His Ala Gly Gly Ile Asn Val Phe Gly Gln Ile
   325 330 335
   Thr Val Glu Thr Phe Ala Ser Ile Val Ala His Glu Leu Gly His Asn
   340 345 350
   Leu Gly Met Asn His Asp Asp Gly Arg Asp Cys Ser Cys Gly Ala Lys
   355 360 365
   Ser Cys Ile Met Asn Ser Gly Ala Ser Gly Ser Arg Asn Phe Ser Ser
   370 375 380
   Cys Ser Ala Glu Asp Phe Glu Lys Leu Thr Leu Asn Lys Gly Gly Asn
   385 390 395 400
   Cys Leu Leu Asn Ile Pro Lys Pro Asp Glu Ala Tyr Ser Ala Pro Ser
   405 410 415
   Cys Gly Asn Lys Leu Val Asp Ala Gly Glu Glu Cys Asp Cys Gly Thr
   420 425 430
   Pro Lys Glu Cys Glu Leu Asp Pro Cys Cys Glu Gly Ser Thr Cys Lys
   435 440 445
   Leu Lys Ser Phe Ala Glu Cys Ala Tyr Gly Asp Cys Cys Lys Asp Cys
   450 455 460
   Arg Phe Leu Pro Gly Gly Thr Leu Cys Arg Gly Lys Thr Ser Glu Cys
   465 470 475 480
   Asp Val Pro Glu Tyr Cys Asn Gly Ser Ser Gln Phe Cys Gln Pro Asp
   485 490 495
   Val Phe Ile Gln Asn Gly Tyr Pro Cys Gln Asn Asn Lys Ala Tyr Cys
   500 505 510
   Tyr Asn Gly Met Cys Gln Tyr Tyr Asp Ala Gln Cys Gln Val Ile Phe
   515 520 525
   Gly Ser Lys Ala Lys Ala Ala Pro Lys Asp Cys Phe Ile Glu Val Asn
   530 535 540
   Ser Lys Gly Asp Arg Phe Gly Asn Cys Gly Phe Ser Gly Asn Glu Tyr
   545 550 555 560
   Lys Lys Cys Ala Thr Gly Asn Ala Leu Cys Gly Lys Leu Gln Cys Glu
   565 570 575
   Asn Val Gln Glu Ile Pro Val Phe Gly Ile Val Pro Ala Ile Ile Gln
   580 585 590
   Thr Pro Ser Arg Gly Thr Lys Cys Trp Gly Val Asp Phe Gln Leu Gly
   595 600 605
   Ser Asp Val Pro Asp Pro Gly Met Val Asn Glu Gly Thr Lys Cys Gly
   610 615 620
   Ala Gly Lys Ile Cys Arg Asn Phe Gln Cys Val Asp Ala Ser Val Leu
   625 630 635 640
   Asn Tyr Asp Cys Asp Val Gln Lys Lys Cys His Gly His Gly Val Cys
   645 650 655
   Asn Ser Asn Lys Asn Cys His Cys Glu Asn Gly Trp Ala Pro Pro Asn
   660 665 670
   Cys Glu Thr Lys Gly Tyr Gly Gly Ser Val Asp Ser Gly Pro Thr Tyr
   675 680 685
   Asn Glu Met Asn Thr Ala Leu Arg Asp Gly Leu Leu Val Phe Phe Phe
   690 695 700
   Leu Ile Val Pro Leu Ile Val Cys Ala Ile Phe Ile Phe Ile Lys Arg
   705 710 715 720
   Asp Gln Leu Trp Arg Ser Tyr Phe Arg Lys Lys Arg Ser Gln Thr Tyr
   725 730 735
   Glu Ser Asp Gly Lys Asn Gln Ala Asn Pro Ser Arg Gln Pro Gly Ser
   740 745 750
   Val Pro Arg His Val Ser Pro Val Thr Pro Pro Arg Glu Val Pro Ile
   755 760 765
   Tyr Ala Asn Arg Phe Ala Val Pro Thr Tyr Ala Ala Lys Gln Pro Gln
   770 775 780
   Gln Phe Pro Ser Arg Pro Pro Pro Pro Gln Pro Lys Val Ser Ser Gln
   785 790 795 800
   Gly Asn Leu Ile Pro Ala Arg Pro Ala Pro Ala Pro Pro Leu Tyr Ser
   805 810 815
   Ser Leu Thr
<210> 24
   <211> 12
   <212> PRT
   <213> Artificial consensus sequence
<220>
   <221> MISC_FEATURE
   <222> (1)..(12)
   <223> x at residues 3, 6, 7, 9, and 10 can be any amino acid
<400> 24
   His Glu Xaa Gly His Xaa Xaa Gly Xaa Xaa His Asp
   1 5 10
<210> 25
   <211> 542
   <212> PRT
   <213> Artificial Sequence
<220>
   <223> Fc Disintegrin Construct
<400> 25
   Met Glu Thr Asp Thr Leu Leu Leu Trp Val Leu Leu Leu Trp Val Pro
   1               5                   10                  15
   Gly Ser Thr Gly Thr Ser Cys Gly Asn Gly Arg Leu Glu Gly Asn Glu
               20                  25                  30
   Ile Cys Asp Cys Gly Thr Glu Ala Gln Cys Gly Pro Ala Ser Cys Cys
           35                  40                  45
   Asp Phe Arg Thr Cys Val Leu Lys Asp Gly Ala Lys Cys Tyr Lys Gly
       50                  55                  60
   Leu Cys Cys Lys Asp Cys Gln Ile Leu Gln Ser Gly Val Glu Cys Arg
   65                  70                  75                  80
   Pro Lys Ala His Pro Glu Cys Asp Ile Ala Glu Asn Cys Asn Gly Ser
                   85                  90                  95
   Ser Pro Glu Cys Gly Pro Asp Ile Thr Leu Ile Asn Gly Leu Ser Cys
               100                 105                 110
   Lys Asn Asn Lys Phe Ile Cys Tyr Asp Gly Asp Cys His Asp Leu Asp
           115                 120                 125
   Ala Arg Cys Glu Ser Val Phe Gly Lys Gly Ser Arg Asn Ala Pro Phe
       130                 135                 140
   Ala Cys Tyr Glu Glu Ile Gln Ser Gln Ser Asp Arg Phe Gly Asn Cys
   145                 150                 155                 160
   Gly Arg Asp Arg Asn Asn Lys Tyr Val Phe Cys Gly Trp Arg Asn Leu
                   165                 170                 175
   Ile Cys Gly Arg Leu Val Cys Thr Tyr Pro Thr Arg Lys Pro Phe His
               180                 185                 190
   Gln Glu Asn Gly Asp Val Ile Tyr Ala Phe Val Arg Asp Ser Val Cys
           195                 200                 205
   Ile Thr Val Asp Tyr Lys Leu Pro Arg Thr Val Pro Asp Pro Leu Ala
       210                 215                 220
   Val Lys Asn Gly Ser Gln Cys Asp Ile Gly Arg Val Cys Val Asn Arg
   225                 230                 235                 240
   Glu Cys Val Glu Ser Arg Ile Ile Lys Ala Ser Ala His Val Cys Ser
                   245                 250                 255
   Gln Gln Cys Ser Gly His Gly Val Cys Asp Ser Arg Asn Lys Cys His
               260                 265                 270
   Cys Ser Pro Gly Tyr Lys Pro Pro Asn Cys Gln Ile Arg Ser Lys Gly
           275                 280                 285
   Phe Ser Ile Phe Pro Glu Glu Asp Met Gly Ser Ile Met Glu Arg Ala
       290                 295                 300
   Ser Gly Lys Thr Glu Asn Thr Trp Arg Ser Cys Asp Lys Thr His Thr
   305                 310                 315                 320
   Cys Pro Pro Cys Pro Ala Pro Glu Ala Glu Gly Ala Pro Ser Val Phe
                   325                 330                 335
   Leu Phe Pro Pro Lys Pro Lys Asp Thr Leu Met Ile Ser Arg Thr Pro
               340                 345                 350
   Glu Val Thr Cys Val Val Val Asp Val Ser His Glu Asp Pro Glu Val
           355                 360                 365
   Lys Phe Asn Trp Tyr Val Asp Gly Val Glu Val His Asn Ala Lys Thr
       370                 375                 380
   Lys Pro Arg Glu Glu Gln Tyr Asn Ser Thr Tyr Arg Val Val Ser Val
   385                 390                 395                 400
   Leu Thr Val Leu His Gln Asp Trp Leu Asn Gly Lys Glu Tyr Lys Cys
                   405                 410                 415
   Lys Val Ser Asn Lys Ala Leu Pro Ala Pro Ile Glu Lys Thr Ile Ser
               420                 425                 430
   Lys Ala Lys Gly Gln Pro Arg Glu Pro Gln Val Tyr Thr Leu Pro Pro
           435                 440                 445
   Ser Arg Glu Glu Met Thr Lys Asn Gln Val Ser Leu Thr Cys Leu Val
       450                 455                 460
   Lys Gly Phe Tyr Pro Ser Asp Ile Ala Val Glu Trp Glu Ser Asn Gly
   465                 470                 475                 480
   Gln Pro Glu Asn Asn Tyr Lys Thr Thr Pro Pro Val Leu Asp Ser Asp
                   485                 490                 495
   Gly Ser Phe Phe Leu Tyr Ser Lys Leu Thr Val Asp Lys Ser Arg Trp
               500                 505                 510
   Gln Gln Gly Asn Val Phe Ser Cys Ser Val Met His Glu Ala Leu His
           515                 520                 525
   Asn His Tyr Thr Gln Lys Ser Leu Ser Leu Ser Pro Gly Lys
       530                 535                 540

## Description

### CROSS REFERENCE TO RELATED APPLICATIONS

This application claims the benefit under 35 U.S.C. §119 of U.S. Provisional Application Serial Number 60/221,838, filed 28 July 2000, and U.S. Provisional Application Serial Number 60/282,550, filed 9 April 2001.

### FIELD OF THE INVENTION

This invention relates to polypeptides having homology to a human metalloproteinase-disintegrin polypeptide family, to polynucleotides encoding such polypeptides, and to methods of making and use thereof.

### BACKGROUND

A metalloproteinase-disintegrin polypeptides, also referred to herein as ADAM ("A Disintegrin And Metalloproteinase domain") polypeptides or "ADAMs," are a related group of multi-domain, type I membrane polypeptides. Certain members of the ADAM family of polypeptides are highly expressed in some cell types including, for example, reproductive tissue or muscle cells. In addition, members of the ADAM family of polypeptides are generally constitutively expressed throughout development.

A number of ADAM genes have now been identified, including fertilin α and β (involved in the integrin mediated binding and fusion of egg and sperm; previously known as PH-30 α and β), epididymal apical protein I, cyritestin, MDC (a candidate for tumor suppressor in human breast cancer), meltrin-α (mediates fusion of myoblast in the process of myotube formation), MS2 (a macrophage surface antigen), and metargidin. In addition, a new ADAM family gene, named ADAMTS-1, containing a disintegrin and metalloproteinase domain with thrombospondin (TSP) motifs, has been shown to be closely associated with various inflammatory processes, as well as development of cancer cachexia (Kuno, K. *et al*., J. Biol. Chem. 272:556-562 (1997)). A new member of ADAM in Drosophila, called the kuzbanian gene ("KUZ"), was found to be involved in Drosophila neurogenesis (Rooke, J. *et al*., Science 273:1227-1231 (August 1996)).

Typical ADAM family polypeptides are cell surface polypeptides that consist of pro-, metalloprotease-like, disintegrin-like, cysteine-rich, epidermal growth factor-like repeat, transmembrane and cytoplasmic domains. In some ADAMs the metalloproteinase domain is believed to be involved in protein processing functions such as release of growth factors, adhesion proteins, and inflammatory factors. The disintegrin domain may play a role in integrin-mediated cell adhesion (cell to cell and cell to matrix) interactions, such as platelet aggregation, migration of tumor cells or neutrophils, and angiogenesis. These activities of the ADAM family of polypeptides are most likely mediated through interactions with the substrates of the metalloproteinase and with integrins, with the substrates of the metalloproteinase binding to the metalloproteinase catalytic domain and integrins binding to the disintegrin domain of the ADAM family of polypeptides. Because of their suspected roles in mediation of protein processing functions such as release of growth factors, adhesion proteins, and inflammatory factors and cell adhesion, the ADAM family of polypeptides are suspected of being associated with inflammation, cancer, allergy, reproductive, and vascular conditions. Characteristics and activities of the ADAM polypeptide family are described further in Black, R.A. and White, J.M., 1998, Curr. Opin. in Cell Biol. 10: 654-659; and in Schlondorff, J. and Blobel, C.P, 1999, J. Cell Sci. 112: 3603-3617. Nath et al., J. Cell Sci. 113: 2319-2328 (2000) report that meltrin y (ADAM-9) may play arde in regulating the mobility of cells by binding to α₆β₁ integrin.

The entry regarding Sequence ID HS388301 (March 20, 1996; Bouillaud, F.) of the EMBL Database (Accession No. N73388) describes a sequence of 688 nucleotides of a human EST clone derived from adipose tissue that is indicated to be similar to a membrane protein with disintegrin and metalloproteinase domain of the MDC or ADAM family.

### SUMMARY OF THE INVENTION

Provided herein for the first time are polynucleotide and polypeptide sequences having homology to the ADAM polypeptide family, termed herein as "ADAM-H9," for ("A Disintegrin And Metalloproteinase with Homology to ADAM9") as well as methods of making and methods of use thereof.

The invention provides a polypeptide selected from the group consisting of (a) a polypeptide comprising an amino acid sequence of SEQ ID NO:8 or SEQ ID NO:10; (b) soluble fragments of the polypeptide of SEQ ID NO:6, SEQ ID NO:8, or SEQ ID NO:10 having at least one activity selected from the group consisting of integrin binding activity, inhibition of endothelial cell migration, and inhibition of angiogenesis; (c) fragments of the polypeptide of SEQ ID NO:6, SEQ ID NO:8, or SEQ ID NO:10 comprising a disintegrin domain amino acid sequence, said sequence corresponding to the sequence from amino acid residue 73±5 to any one of amino acid residues 360±5 to 362±5 of SEQ ID NO:6, from any one of amino acid residues 1 to 16±5 to any one of amino acid residues 285±5 to 287±5 of SEQ ID NO:8, and from any one of amino acid residues 1 to 73±5 to any one of amino acid residues 314±5 to 329±5, respectively; (d) SEQ ID NO:6 from amino acid residue 73±5 to any one of amino acid residues 360±5 to 362±5; (e) SEQ ID NO:8 from any one of amino acid residues 1 to 16±5 to any one of amino acid residues 285±5 to 287±5; (f) SEQ ID NO:10 from any one of amino acid residues 1 to 73±5 to any one of amino acid residues 314±5 to 329±5; (g) polypeptides having amino acid sequences sharing amino acid identity across the length of the amino acid sequences of SEQ ID NO:8 or SEQ ID NO:10, wherein the percent amino acid identity is selected from the group consisting of at least 97.5%, at least 99%, and at least 99.5%; and (h) a polypeptide comprising an amino acid sequence of SEQ ID NO:25.

The invention further provides a polypeptide as described above linked to a second polypeptide, wherein the second polypeptide is a leucine zipper polypeptide, an Fc polypeptide, or a peptide linker.

The invention also provides an isolated polynucleotide encoding a polypeptide as described above.

Also provided by the invention is an isolated polynucleotide selected from the group consisting of (a) a polynucleotide comprising a sequence of SEQ ID NO:7 or SEQ ID NO:9; (b) SEQ ID NO:5 from nucleotide 248 to nucleotide 1111 of SEQ ID NO:5; (c) a polynucleotide comprising a sequence of SEQ ID NO:7 from a nucleotide between 82 and 127 to nucleotide 936; d) a polynucleotide comprising a sequence of SEQ ID NO:9 from a nucleotide between 32 and 248 to a nucleotide between 973 and 1018; (e) a nucleotide sequence complementary to a sequence of SEQ ID NO:7 or SEQ ID NO:9; and (f) any of nucleotide sequences of (a) to (e) wherein T can also be U. An isolated polynucleotide comprising a sequence described above operably linked to a polynucleotide encoding a polypeptide of interest is further provided by the invention.

The invention provides an isolated polynucleotide encoding any of the polypeptides above. In one embodiment, the polynucleotide comprises a sequence set forth in SEQ ID Nos:5, 7, or 9, complements thereof. Described herein are furthermore polynucleotides that hybridize to a polynucleotide having a sequence of SEQ ID Nos:2, 5, 7, or 9. The polynucleotide can be DNA or RNA.

Also provided by the invention is an isolated polynucleotide comprising a sequence as set forth in SEQ ID Nos:5, 7, or 9 operably linked to a polynucleotide encoding a polypeptide of interest (*e.g*., a sequence encoding a leucine zipper, Fc polypeptide, or peptide linker sequence). Preferably the polynucleotide of SEQ ID NO:5, 7, or 9 encodes a fragment having disintegrin activity and encodes a polypeptide as set forth in SEQ ID Nos:6 from about amino acid number 73 to about 360, SEQ ID NO:8 from amino acid 1 or 16 to 285 and/or SEQ ID NO:10 from amino acid 1 or 73 to 314 or 329.

The invention provides an expression vector having a polynucleotide of the invention as well as host cells comprising such an expression vector or a recombinant polynucleotide of the invention.

The invention further provides a method for producing a polypeptide, comprising culturing a recombinant host cell comprising a polynucleotide of the invention under conditions promoting expression of the polypeptide encoded by the polynucleotide and purifying the polypeptide.

The invention provides a substantially purified antibody that specifically binds to a polypeptide of the invention. The antibody can be monoclonal or polyclonal. In some embodiments, the antibody is human or humanized.

Described herein is also a method of designing an inhibitor or binding agent of a polypeptide of the invention. The method includes determining the three-dimensional structure of an ADAM-H9 polypeptide of the invention, analyzing the three-dimensional structure of the polypeptide for binding sites of ligands, designing a molecule that is predicted to interact with the polypeptide, and determining the inhibitory or binding activity of the molecule.

The invention also provides a method for identifying an agent that modulates ADAM-H9 activity or expression. The method includes contacting the agent with an ADAM-H9 polypeptide or polynucleotide under conditions such that the agent and polypeptide or polynucleotide interact and determining the ADAM-H9 activity or expression in the presence of the agent compared to a control wherein a change in activity or expression is indicative of an agent that modulates ADAM -H9 activity or expression.

Described herein is furthermore a method for modulating angiogenesis in a tissue, comprising contacting the tissue with an ADAM-H9 polypeptide of the invention. The contacting may be *in vitro* or *in vivo*. Also provided are methods for modulating endothelial cell migration, by contacting an endothelial cell with an ADAM-H9 polypeptide or an antibody that specifically binds to an ADAM-H9 polypeptide. The contacting may be *in vitro* or *in vivo.*

The invention also provides a method of inhibiting the binding of an integrin to a ligand comprising contacting a cell that expresses the integrin with an effective amount of an ADAM-H9 disintegrin domain. In some embodiments the ADAM-H9 disintegrin domain comprises a sequence as set forth in SEQ ID NO:6 from about amino acid number 73 to about 360, SEQ ID NO:8 from amino acid 1 or 16 (or residues therebetween) to 285, or SEQ ID NO:10 from amino acid 1 or 73 (or residues therebetween) to 314 or 329 (or residues therebetween).

Also described is a method of modulating the binding of an integrin to a ligand in a mammal comprising administering an effective amount of a soluble polypeptide comprising an ADAM-H9 disintegrin domain. In some embodiments the mammal is afflicted with a condition selected from the group consisting of ocular disorders; malignant or metastatic conditions; inflammatory diseases; osteoporosis and other conditions mediated by accelerated bone resorption; restenosis; inappropriate platelet activation, recruitment, or aggregation; thrombosis; or a condition requiring tissue repair or wound healing.

Described herein is further a method of inhibiting angiogenesis in a mammal, comprising administering to the mammal an inhibition-effective amount of a soluble polypeptide comprising an ADAM-H9 disintegrin domain. In one embodiment, the ADAM-H9 disintegrin domain comprises a sequence as set forth in SEQ ID NO:6 from about amino acid number 73 to 360, SEQ ID NO:8 from about amino acid 1 or 16 to 285, or SEQ ID NO:10 from about amino acid 1 or 73 to 314 or 329. The soluble ADAM-H9 disintegrin domain can be in the form of a multimer (*e.g*., a dimer, trimer, or fusion polypeptide). In one embodiment, the multimer comprises an Fc polypeptide or a leucine zipper. In another embodiment, the polypeptide comprises a sequence as set forth in SEQ ID NO:25.

Also described is a computer readable medium having contained thereon computer readable data of a sequence as set forth in SEQ ID Nos: 1, 2, 3, 4, 5, 6, 7, 8, 9, 10, or a combination thereof.

The invention also provides the use of a soluble ADAM-H9 disintegrin domain polypeptide comprising a fragment of the polypeptide of SEQ ID NO:6, SEQ ID NO:8, or SEQ ID NO:10 having at least one activity selected from the group consisting of integrin binding activity, inhibition of endothelial cell migration, and inhibition of angiogenesis, for the preparation of a pharmaceutical composition for inhibiting angiogenesis in a mammal in need of such treatment.

The invention further provides the use of a soluble ADAM-H9 disintegrin domain polypeptide comprising a fragment of the polypeptide of SEQ ID NO:6, SEQ ID NO:8, or SEQ ID NO:10 having at least one activity selected from the group consisting of integrin binding activity, inhibition of endothelial cell migration, and inhibition of angiogenesis, for the preparation of a pharmaceutical composition for treating a condition selected from the group consisting of ocular disorders; malignant and metastatic conditions; inflammatory diseases; osteoporosis, accelerated bone resorption disorders; restenosis; inappropriate platelet activation, recruitment, or aggregation; thrombosis; and a condition requiring tissue repair or wound healing.

### BRIEF DESCRIPTION OF THE DRAWINGS

**Figure 1** shows an alignment of ADAM9 (SEQ ID NO:23) with ADAM-H9 sequences set forth in SEQ ID Nos: 6, 8, and 10. Identified in the figure are domains predicted by homology to the ADAM family of polypeptides.
**Figure 2** shows a table depicting the amino acids sequences of polypeptides according to the invention.
**Figure 3** shows a table depicting nucleotide sequences of polynucleotides according to the invention.

### DETAILED DESCRIPTION OF THE INVENTION

The typical structural elements common to various members of the ADAM family of polypeptides include, in N-to-C order, a signal sequence, a prodomain, a metalloproteinase domain, a disintegrin domain, a cysteine-rich domain, a transmembrane domain, and a cytoplasmic domain. There are certain key residues within the metalloproteinase domains/motifs (*e.g.*, the HexGHxxGxxHD motif (SEQ ID NO:24)) such that substitutions of those extremely conserved residues are likely to be associated with an altered function or lack of function for the polypeptide. ADAMs with the conserved metalloprotease active site sequence of SEQ ID NO:24 include ADAMs 1, 8-10, 12-13, 15-17, 19-21, 24-26, 28, and 30. The metalloproteinase catalytic domains also contain four conserved cysteines that may be required for the formation of a functional polypeptide structure through disulfide bonds. There are 31 highly conserved cysteines in the disintegrin and cysteine rich region; almost all of the ADAM family of polypeptides have these 31 cysteines. The skilled artisan will recognize that the boundaries of these regions within the polypeptides are approximate and that the precise boundaries of such domains (which can be predicted by using computer programs available for that purpose) can differ from member to member within the ADAM family of polypeptides.

The ADAM family of polypeptides is reasonably well conserved, with the human family members similar to each other and to ADAM family members from other species such as mouse, rat, and even *Drosophila melanogaster* and *Caenorhabditis elegans* (see, *e.g.*, Yamamoto et al., Immunol. Today, 20(6):278, 1999; and the following Internet websites for more information (www): gene.ucl.ac.uk/users/hester/metalo.html; uta.fi/~loiika/ADAMs/HADAMs.html; and people.Virginia.EDU/~jag6n/Table_of_the_ ADAMs.html). However, subfamilies of the ADAM family of polypeptides can be defined on the basis of sequence similarity and related biological activities. One such subfamily comprises the ADAM10 and ADAM17/TACE polypeptides, which show greater sequence similarity to each other compared to other members identified so far within the ADAM family of polypeptides. ADAMs 10 and 17 have 21 cysteines in the disintegrin-cysteine rich region in contrast to the 31 conserved cysteines in this region among the other ADAMs. Accordingly, ADAMs may have from 20 to 31 conserved cysteines (*e.g.,* 20, 21, 22, 23, 24, 25, 26, 27, 28, 29, 30, or 31 conserved cysteines). ADAM17/TACE and ADAM-10 are also "sheddases," meaning they are believed to cleave and release the extracellular domains of other membrane proteins. The major function of another subfamily may be to bind integrins or other proteins. ADAM-2, for example, is processed to remove both the prodomain and the metalloproteinase catalytic domain so as to expose the disintegrin domain and allow it to bind to its cognate. Another subfamily that can be defined are the ADAMs that appear to be testis-specific; these polypeptides are ADAMs 2-3, 16, 18, 20-21, 24-26, and 29-30; with ADAMs 5 and 6 being primarily testis-specific.

Polypeptides of the ADAM family are expressed in many cell types including, for example, uritogenital tissues (*e.g.*, kidney tissue and reproductive tissue), neurologic tissue, and muscle cells. Some binding partners for ADAM polypeptides are expressed, for example, by endothelial cells and T cells, as displayed by the disintegrin-cysteine rich domains of several ADAM family polypeptides binding to endothelial cells, at least partly through interaction with integrins, and to T cells. The interactions between members of the ADAM family of polypeptides and their binding partners are likely involved in mediating interactions between cell types including reproductive tissue, neurologic tissue, and muscle cells, and binding-partner-expressing endothelial cells and T cells.

The disintegrin domain of some ADAM family polypeptides can interact with binding partners such as cell surface integrins (see, *e.g.*, co-pending International Application Serial No. PCT/US01/05701, the disclosure of which is incorporated herein by reference in its entirety). By binding to one or more binding partners, the disintegrin domain polypeptide can inhibit the biological activities (*e.g.,* angiogenesis) mediated via binding of ADAM polypeptides to its binding partner. Because some ADAM family polypeptides exhibit integrin-binding activities via the disintegrin domain, modulation of disintegrin activity will modulate adhesion, *e.g.*, the role of ADAMs 1 and 2 in sperm binding to egg and the role of ADAM9 in interactions of glomerular and tubular epithelial cells with the basal laminae in renal tissue. The degree to which individual members of the ADAM family bf polypeptides and fragments and other derivatives of these polypeptides exhibit these activities can be determined by standard assay methods, such as inhibition of endothelial cell migration by disintegrin-Fc constructs, and the like. Particularly suitable assays to detect or measure the binding between ADAM polypeptides and their binding partners are FACS analysis. Additional assays for evaluating the biological activities and partner-binding properties of ADAM family polypeptides are described below (see, *e.g.*, Examples 5-7). Although ADAM-H9 lacks a metalloproteinase domain, it may act as a dominant negative with respect to the metalloproteinase activity of other ADAM family polypeptides.

Polypeptides of the ADAM family are involved in inflammation, cancer, allergy, reproductive, neural disorders and diseases, angiogenesis and vascular diseases or conditions that share as a common feature integrin-associated interactions. Examples of inflammation, cancer, allergy, reproductive, neural disorders and diseases, angiogenesis and vascular conditions that are known or are likely to involve the biological activities of ADAM polypeptides are rheumatoid arthritis, septic shock, glomerular diseases, acute renal failure, Alzheimer's disease, and inappropriate bone resorption. Blocking or inhibiting the interactions between members of the ADAM family of polypeptides and their substrates, ligands, receptors, binding partners, or other interacting polypeptides is an aspect of the invention and provides methods for treating, modulating, or ameliorating these diseases and conditions through the use of inhibitors or modulators of ADAM polypeptide activity. In one embodiment, interaction between members of the ADAM family of polypeptides and their cognates is affected by contacting a sample containing an ADAM family polypeptide or its cognate with an ADAM-H9 polypeptide or antibody. In another embodiment, the ADAM family polypeptide is ADAM-H9.

For certain conditions involving too little disintegrin activity, methods of treating or ameliorating these conditions comprise increasing the amount or activity of, for example, ADAM-H9 polypeptides by providing such polypeptides or active fragments or fusion polypeptides thereof, or by providing agents that activate endogenous or exogenous ADAM polypeptides. Additional uses for ADAM polypeptide family members include diagnostic reagents for inflammation, cancer, allergy, reproductive, neural disorders, and vascular diseases; research reagents for investigation of integrin polypeptides and fertilization processes, purification and processing of integrins and/or endothelial cells or T cells; or as a carrier/targeting polypeptide to deliver therapeutic agents to cells.

As used herein, both "protein" and "polypeptide" mean any chain of amino acids, regardless of length or post-translational modification (*e.g.*, glycosylation or phosphorylation), and include natural proteins, synthetic or recombinant polypeptides and peptides as well as a recombinant molecule consisting of a hybrid with one portion, for example, having all or part of an ADAM-H9 amino acid sequence and a second portion being encoded by all or part of a second nucleotide sequence. Typically the protein or polypeptide is substantially pure of other components from which it is normally present in nature. The term "substantially pure" or "purified" when referring to a polypeptide, means a polypeptide that is at least 30% free from the proteins and naturally-occurring organic molecules with which it is naturally associated. Preferably the substantially pure polypeptide of the invention is at least 35-50%; preferably 60-70%; more preferably at least 75% to 90%; and most preferably at least 99% by weight purified from other naturally occurring molecules. A substantially pure polypeptide of the invention can be obtained, for example, by extraction from a natural source, by expression of a recombinant polynucleotide encoding the polypeptide, or by chemically synthesizing the polypeptide. Purity can be measured by any appropriate method, *e.g.,* chromatography, PAGE, or HPLC analysis.

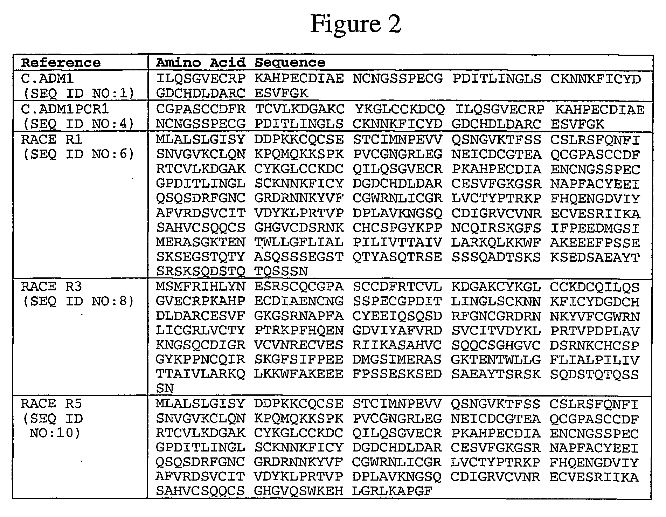

As used herein an "ADAM-H9 polypeptide" means a polypeptide that contains or comprises an amino acid sequence as set forth in Figure 2; polypeptides having substantial homology or substantial identity to the sequences set forth in SEQ ID, Nos:1, 3, 4, 6, 8, or 10; fragments of the foregoing sequences; and conservative variants of the foregoing. Described and/or claimed herein are polypeptides having a sequence as set forth in SEQ ID Nos: 1, 3, 4, 6, 8, and 10. The polypeptides have been shown to have a high degree of homology to the ADAM9 polypeptide and thus have a predicted function/activity of an ADAM polypeptide. Accordingly, described and/or claimed herein is an ADAM-H9 polypeptide comprising a sequence selected from the group consisting of SEQ ID Nos:1, 3, 4, 6, 8, and 10. In one embodiment, an ADAM-H9 polypeptide has disintegrin activity or metalloproteinase inhibitory activity or a combination thereof. Methods of determining whether a polypeptide of the invention has a desired disintegrin activity or metalloproteinase inhibitory activity can be accomplished by assaying the polypeptide by any of the methods described herein below. For example, ADAM-H9 disintegrin activity can be measured using, the methods of Examples 5-7, below.

A polypeptide of the invention also encompasses an amino acid sequence that has a sufficient or a substantial degree of identity or similarity to a sequence set forth in Figure 2. Substantially identical sequences can be identified by those of skill in the art as having structural domains and/or having biological activity in common with an ADAM-H9 polypeptide. Methods of determining similarity or identity may employ computer algorithms such as, *e.g.,* BLAST, FASTA, and the like.

The phrase "substantially identical," in the context of two nucleic acids or polypeptides, refers to sequences or subsequences that have at least 60%, preferably 80% or 85%, most preferably 90-95% nucleotide or amino acid residue identity when aligned for maximum correspondence over a comparison window as measured by, for example, a sequence comparison algorithm or by manual alignment and visual inspection. This definition also refers to the complement of a test sequence, which has substantial sequence or subsequence complementarity when the test sequence has substantial identity to a reference sequence. A "comparison window", as used herein, includes reference to a segment of any one of the number of contiguous positions selected from the group consisting of from 20 to 1800, usually about 50 to 200, more usually about 70 to 150 in which a sequence may be compared to a reference sequence of the same number of contiguous positions after the two sequences are optimally aligned.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'1. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection.

One example of a useful algorithm is PILEUP. PILEUP creates a multiple sequence alignment from a group of related sequences using progressive, pairwise alignments to show relationship and percent sequence identity. PILEUP uses a simplification of the progressive alignment method of Feng & Doolittle, J. Mol. Evol. 35:351 (1987), and is similar to the method described by Higgins & Sharp, CABIOS 5:151 (1989). The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. For example, a reference sequence can be compared to other test sequences to determine the percent sequence identity relationship using the following parameters: default gap weight (3.00), default gap length weight (0.10), and weighted end gaps.

Another example of algorithm that is suitable for determining percent sequence identity and sequence similarity is the BLAST algorithm, as described in Altschul *et al.,* J. Mol. Biol. 215:403 (1990). Software for performing BLAST analyses is publicly available through the National Center for Biotechnology Information (www-ncbi.nlm.nih.gov/). This algorithm involves first identifying high scoring sequence pairs (HSPs) by identifying short words of length W in the query sequence, which either match or satisfy a positive-valued threshold score T when aligned with a word of the same length in a database sequence. T is referred to as the neighborhood word score threshold. These initial neighborhood word hits act as seeds for initiating searches to find longer HSPs containing them. The word hits are extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST program uses as defaults a wordlength (W) of 11, the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands. One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

Alternatively, the percent identity of two amino acid or two nucleic acid sequences can be determined by comparing sequence information using the GAP computer program, version 6.0 described by Devereux *et al.* (*Nucl. Acids Res.* 12:387, 1984) and available from the University of Wisconsin Genetics Computer Group. The preferred default parameters for the GAP program include: (1) a unary comparison matrix (containing a value of 1 for identities and 0 for non-identities) for nucleotides, and the weighted comparison matrix of Gribskov and Burgess, *Nucl. Acids Res.* 14:6745, 1986, as described by Schwartz and Dayhoff, eds., *Atlas of Polypeptide Sequence and Structure,* National Biomedical Research Foundation, pp. 353-358, 1979; (2) a penalty of 3.0 for each gap and an additional 0.10 penalty for each symbol in each gap; and (3) no penalty for end gaps.

One of skill will recognize that individual substitutions, deletions or additions to a nucleic acid sequence, peptide, or polypeptide sequence that alters, adds or deletes a single amino acid or a small percentage of amino acids in the encoded sequence is a "conservatively modified variant" where the alteration results in a molecule having substantially the same biological activity (*e.g*., disintegrin activity). For example, an alteration that results in the substitution of an amino acid with a chemically similar amino acid is a conservatively modified variant. Conservative substitution tables providing functionally similar amino acids are known in the art. The following six groups each contain amino acids that are conservative substitutions for one another 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W) (see, *e.g.,* Creighton, Proteins (1984)).

One indication that two polynucleotides or polypeptides are substantially identical is that the polypeptide encoded by a first polynucleotide is immunologically cross reactive with the antibodies raised against the polypeptide encoded by a second polynucleotide. Another indication that two polynucleotides are substantially identical is that the two molecules or their complements hybridize to each other under stringent conditions.

Polypeptides derived from the ADAM-H9 polypeptides of the invention by any type of alteration (*e.g.,* insertions, deletions, or substitutions of amino acids; changes in the state of glycosylation of the polypeptide; refolding or isomerization to change its three-dimensional structure or self-association state; and changes to its association with other polypeptides or molecules) are also encompassed by the invention. Therefore, the polypeptides provided by the invention include polypeptides characterized by amino acid sequences similar to those as set forth in Figure 2, but into which modifications are naturally provided or deliberately engineered. A polypeptide that shares biological activities in common with a polypeptide comprising a sequence as set forth in SEQ ID Nos 6, 8, or 10 having disintegrin activity and/or metalloproteinase inhibitory activity are encompassed by the invention.

The present invention encompasses various forms of ADAM-H9 disintegrin domains that retain at least one activity ("disintegrin activity") selected from the group consisting of integrin binding activity, inhibition of endothelial cell migration, and inhibition of angiogenesis. The term "ADAM-H9 disintegrin domain polypeptide" (ADAM-H9dis) is intended to encompass polypeptides containing all or part of a ADAM-H9 disintegrin domain, with or without other ADAM domains (such as the cysteine-rich region), as well as related forms including, but not limited to: (a) fragments, (b) variants, (c) derivatives, (d) fusion polypeptides, and (e) multimeric forms (multimers). The ability of these related forms to inhibit integrin binding, endothelial cell migration, and/or inhibition of angiogenesis may be determined *in vitro* or *in vivo* by using methods such as those exemplified below or by using other assays known in the art.

One of skill in the art can easily assay for activity using the methods described herein. Such methods measure, for example, biological activities exhibited by members of the ADAM family of polypeptides including, without limitation, cell adhesion. For example, anti-ADAM-H9 antibodies, which neutralize ADAM-H9 activity, can be used to assay for similar polypeptides by contacting an anti-ADAM-H9 antibody with a polypeptide of interest and determining if the activity associated with the polypeptide of interest is neutralized. In addition, the cross-reactivity of an antibody that specifically binds to an ADAM-H9 polypeptide of the invention is indicative of a polypeptide that shares structural characteristics (*e.g.*, primary, secondary, or tertiary protein characteristics) with an ADAM-H9 polypeptide of the invention.

The invention provides both full-length and mature forms of ADAM-H9 polypeptides. Full-length polypeptides are those having the complete primary amino acid sequence of the polypeptide as initially translated. The amino acid sequences of full-length polypeptides can be obtained, for example, by translation of the complete open reading frame ("ORF") of a cDNA molecule. Several full-length polypeptides may be encoded by a single genetic locus if multiple mRNA forms are produced from that locus by alternative splicing or by the use of multiple translation initiation sites. The "mature form" of a polypeptide refers to a polypeptide that has undergone post-translational processing steps, if any, such as, for example, cleavage of the signal sequence or proteolytic cleavage to remove a prodomain. Multiple mature forms of a particular full-length polypeptide may be produced, for example, by imprecise cleavage of the signal sequence, or by differential regulation of proteases that cleave the polypeptide. The mature form(s) of such polypeptide may be obtained by expression, in a suitable mammalian cell or other host cell, of a polynucleotide that encodes the full-length polypeptide. The sequence of the mature form of the polypeptide may also be determinable from the amino acid sequence of the full-length form, through identification of signal sequences or protease cleavage sites (*e.g.,* S68 or P71 of SEQ ID Nos:6 and 10 are possible processing sites). The ADAM-H9 polypeptides of the invention also include polypeptides that result from post-transcriptional or post-translational processing events such as alternate mRNA processing which can yield a truncated but biologically active polypeptide, for example, a naturally occurring soluble form of the polypeptide. Also encompassed within the invention are variations attributable to proteolysis such as differences in the N- or C-termini upon expression in different types of host cells, due to proteolytic removal of one or more terminal amino acids from the polypeptide (generally from 1-5 terminal amino acids).

A polypeptide of the invention may be prepared by culturing transformed or recombinant host cells under culture conditions suitable to express a polypeptide of the invention. The resulting expressed polypeptide may then be purified from such culture using known purification processes, such as gel filtration and ion exchange chromatography. The purification of the polypeptide may also include an affinity column containing agents which will bind to the polypeptide; one or more column steps over such affinity resins as concanavalin A-agarose, heparin-toyopearl® or Cibacrom blue 3GA Sepharose®; one or more steps involving hydrophobic interaction chromatography using such resins as phenyl ether, butyl ether, or propyl ether; or immunoaffinity chromatography. Alternatively, the polypeptide of the invention may also be expressed in a form that will facilitate purification. For example, it may be expressed as a fusion polypeptide, such as those of maltose binding polypeptide (MBP), glutathione-S-transferase (GST) or thioredoxin (TRX). Kits for expression and purification of such fusion polypeptides are commercially available from New England BioLab (Beverly, MA), Pharmacia (Piscataway, NJ), and InVitrogen, respectively. The polypeptide can also be tagged with an epitope and subsequently purified by using a specific antibody directed to such epitope. One such epitope ("Flag") is commercially available from Kodak (New Haven, Conn.). Finally, one or more reverse-phase high performance liquid chromatography (RP-HPLC) steps employing hydrophobic RP-HPLC media, *e.g.*, silica gel having pendant methyl or other aliphatic groups, can be employed to further purify the polypeptide. Some or all of the foregoing purification steps, in various combinations, can also be employed to provide a substantially homogeneous recombinant polypeptide. The polypeptide thus purified is substantially free of other mammalian polypeptides and is defined in accordance with the invention as an "substantially purified polypeptide"; such purified polypeptides of include antibodies that specifically bind to an ADAM-H9 polypeptide, fragment, variant, and the like. A polypeptide of the invention may also be expressed as a product of transgenic animals, *e.g.*, as a component of the milk of transgenic cows, goats, pigs, or sheep which are characterized by somatic or germ cells containing a polynucleotide encoding a polypeptide of the invention.

It is also possible to utilize an affinity column such as a monoclonal antibody generated against polypeptides of the invention, to affinity-purify expressed polypeptides. These polypeptides can be removed from an affinity column using conventional techniques, *e.g.*, in a high salt elution buffer and then dialyzed into a lower salt buffer for use or by changing pH or other components depending on the affinity matrix utilized, or be competitively removed using the naturally occurring substrate of the affinity moiety, such as a polypeptide derived from the invention. In this aspect of the invention, proteins that bind a polypeptide of the invention (*e.g.*, an anti-ADAM-H9 antibody of the invention) can be bound to a solid phase support or a similar substrate suitable for identifying, separating, or purifying cells that express polypeptides of the invention on their surface. Adherence of, for example, an anti-ADAM-H9 antibody of the invention to a solid phase surface can be accomplished by any means, for example, magnetic microspheres can be coated with these polypeptide-binding proteins and held in the incubation vessel through a magnetic field. Suspensions of cell mixtures are contacted with the solid phase that has such polypeptide-binding proteins thereon. Anti-ADAM-H9 antibodies bind cells having polypeptides of the invention on their surface (*e.g.*, an extracellular domain of ADAM-H9). Unbound cells *(e.g.,* cell lacking and ADAM-H9 polypeptide) are washed away from the bound cells. This affinity-binding method is useful for purifying, screening, or separating such polypeptide-expressing cells from solution. Methods of releasing positively selected cells from the solid phase are known in the art and encompass, for example, the use of enzymes. Such enzymes are preferably non-toxic and non-injurious to the cells and are preferably directed to cleaving the cell-surface binding partner. Alternatively, mixtures of cells suspected of containing polypeptide-expressing cells of the invention are first incubated with a biotinylated binding polypeptide of the invention. Incubation periods are typically at least one hour in duration to ensure sufficient binding to polypeptides of the invention. The resulting mixture then is passed through a column packed with avidin-coated beads, whereby the high affinity of biotin for avidin provides the binding of the cells to the beads. Use of avidin-coated beads is known in the art (see, Berenson, *et al. J. Cell. Biochem.,* 10D:239, 1986). Wash of unbound material and the release of the bound cells is performed using conventional methods.

A polypeptide of the invention may also be produced by known conventional chemical synthesis. Methods for constructing the polypeptides of the invention by synthetic means are known to those skilled in the art. The synthetically-constructed polypeptide sequences, by virtue of sharing primary, secondary or tertiary structural and/or conformational characteristics with a native polypeptides may possess biological properties in common therewith, including biological activity. Thus, the synthesized polypeptides may be employed as biologically active or immunological substitutes for natural, purified polypeptides in screening of therapeutic compounds and in immunological processes for the development of antibodies.

The desired degree of purity depends on the intended use of the polypeptide. A relatively high degree of purity is desired when the polypeptide is to be administered *in vivo,* for example. In such a case, the polypeptides are purified such that no polypeptide bands corresponding to other polypeptides are detectable upon analysis by SDS-polyacrylamide gel electrophoresis (SDS-PAGE). It will be recognized by one skilled in the pertinent field that multiple bands corresponding to the polypeptide can be visualized by SDS-PAGE, due to differential glycosylation, differential post-translational processing, and the like. Most preferably, the polypeptide of the invention is purified to substantial homogeneity, as indicated by a single polypeptide band upon analysis by SDS-PAGE. The polypeptide band can be visualized by silver staining, Coomassie blue staining, or (if the polypeptide is radiolabeled) by autoradiography.

Species homologues of ADAM-H9 polypeptides and polynucleotides encoding the polypeptides are also provided by the invention. As used herein, a "species homologue" is a polypeptide or polynucleotide with a different species of origin from that of a given polypeptide or polynucleotide, but with significant sequence similarity to the given polypeptide or polynucleotide. Species homologues may be isolated and identified by making suitable probes or primers from polynucleotides encoding the polypeptides provided herein and screening a suitable nucleic acid source from the desired species. Alternatively, homologues may be identified by screening a genome database containing sequences from one or more species utilizing a sequence (*e.g.*, nucleic acid or amino acid sequence) of an ADAM-H9 of the invention. Such genome databases are readily available for a number of species (*e.g.*, on the world wide web (www) at tigr.org/tdb; genetics.wisc.edu; stanford.edu/-ball; hiv-web.1an1.gov; ncbi.nlm.nig.gov; ebi.ac.uk; and pasteur.fr/other/biology). The invention also encompasses allelic variants of ADAM-H9 polypeptides and nucleic acids encoding them that are naturally-occurring alternative forms of such polypeptides and polynucleotides in which differences in amino acid or nucleotide sequence are attributable to genetic polymorphism.

Intermediate Sequence Search (ISS) can be used to identify closely related as well as distant homologs by connecting two proteins through one or more intermediate sequences. ISS repetitively uses the results of the previous query as new search seeds. Saturated BLAST is a package that performs ISS. Starting with a protein sequence, Saturated BLAST runs a BLAST search and identifies representative sequences for the next generation of searches. The procedure is run until convergence or until some predefined criteria are met. Saturated BLAST is available on the world wide web (www) at: bioinformatics.burnham-inst.org/xblast (see also, Li *et al.* Bioinformatics 16(12):1105, 2000).

Fragments of the ADAM-H9 polypeptides of the invention are encompassed by the invention and may be in linear form or cyclized using known methods (see, *e.g.,* H.U. Saragovi, *et al*., Bio/Technology 10, 773 (1992); and R. S. McDowell, *et al.,* J. Amer. Chem. Soc. 114:9245 (1992), both of which are incorporated by reference herein). Peptide fragments of ADAM-H9 polypeptides of the invention, and polynucleotides encoding such fragments include amino acid or nucleotide sequence lengths that are at least 25% (more preferably at least 50%, 60%, or 70%, and most preferably at least 80%) of the length of an ADAM-H9 polypeptide or polynucleotide. Preferably such sequences will have at least 60% sequence identity (more preferably at least 70%-75%, 80%-85%, 90%-95%, at least 97.5%, or at least 99%, and most preferably at least 99.5%) with an ADAM-H9 polypeptide or polynucleotide when aligned so as to maximize overlap and identity while minimizing sequence gaps. Also included in the invention are polypeptides, peptide fragments, and polynucleotides encoding them, that contain or encode a segment preferably comprising at least 8 to 10, or more preferably at least 20, or still more preferably at least 30, or most preferably at least 40 contiguous amino acids. Such polypeptides and fragments may also contain a segment that shares at least 70% (at least 75%, 80%-85%, 90%-95%, at least 97.5%, or at least 99%, and most preferably at least 99.5%) with any such segment of any of the ADAM family polypeptides, when aligned so as to maximize overlap and identity while minimizing sequence gaps. Visual inspection, mathematical calculation, or computer algorithms can determine the percent identity.

The invention also provides for soluble forms of ADAM-H9 polypeptides comprising certain fragments or domains of these polypeptides. Soluble fragments having disintegrin activity are of particular interest. For example, an amino acid sequence beginning with a highly conserved CGN sequence at residue 73 and continuing to about residue 361 which lacks a transmembrane region of SEQ ID NO:6 has disintegrin activity. Other soluble forms include polypeptides comprising SEQ ID NO:8 beginning at an amino acid between and including residues 1 and 16 to residue 285, or SEQ ID NO: 10 beginning at an amino acid between and including residues 1 and 73 to a residue between 314 and 329. In such forms part or all of the intracellular and transmembrane domains of the polypeptide are deleted such that the polypeptide is fully secreted from the cell in which it is expressed. The intracellular and transmembrane domains of polypeptides of the invention can be identified in accordance with known techniques for determination of such domains from sequence information. For example, alignment of the polypeptide sequences of the invention with other members of the ADAM family of polypeptides having known domains will provide information regarding the domains of the polypeptides of the invention. For example, SEQ ID NO:1 3, 6, 8, and 10 have been identified based upon their homology with ADAM9 and have domains predicted to belong to a disintegrin domain, a transmembrane domain and a cytoplasmic domain. A polypeptide having, for example, a sequence set forth in SEQ ID Nos:6, 8, and 10 beginning with a highly conserved CGN sequence at, for example, residue 73 of SEQ ID NO:6 and continuing to about residue 360 has a number of conserved cysteine residues when compared to ADAM9 and includes a predicted disintegrin domain of an ADAM family polypeptide. One of skill in the art will recognize that slight modifications in the range of sequences of a particular domain can be made without affecting the molecule's biological activity. Accordingly, changes in the identified sequences of 1, 2, 3, 4, or 5 amino acids in either direction of the particular domain are encompassed by the present invention (*e.g.*, the disintegrin domain of SEQ ID NO:6 may include residues 68, 69, 70, 71, 72, 74, 75, 76, 77, or 78 to about residues 355, 356, 357, 358, 359, 361, 362, 363, 364, or 365).

In another aspect of the invention, a polypeptide may comprise various combinations of ADAM polypeptide domains, such as a metalloproteinase domain, a disintegrin domain, or a cytoplasmic domain. Accordingly, polypeptides of the invention and polynucleotides include those comprising or encoding two or more copies of a domain such as the metalloproteinase domain, two or more copies of a domain such as the disintegrin domain, or at least one copy of each domain, and these domains may be presented in any order within such polypeptides. Also included are recombinant polypeptides and the polynucleotides encoding the polypeptides wherein the recombinant polypeptides are "chimeric polypeptides" or "fusion polypeptides" and comprise an ADAM-H9 sequence as set forth in SEQ ID NO:1, 3, 6, 8 or 10 operatively linked to a second polypeptide. The second polypeptide can be any polypeptide of interest having an activity or function independent of, or related to, the function of an ADAM-H9 polypeptide. For example, the second polypeptide can be a domain of a related but distinct member of the ADAM family of polypeptides such as, for example, an extracellular, cytoplasmic, metalloprotease, or transmembrane domain of a related ADAM polypeptide. The term "operatively linked" is intended to indicate that the ADAM-H9 sequence and the second polypeptide sequence are fused in-frame to each other. The second polypeptide can be fused to the N-terminus or C-terminus of an ADAM-H9 sequence as set forth in Figure 2. For example, in one embodiment the fusion polypeptide is a GST-ADAM-H9 fusion polypeptide in which the ADAM-H9 sequences are fused to the C-terminus of the GST sequences. Such fusion polypeptides can facilitate the purification of recombinant ADAM-H9 sequences. In another embodiment, the fusion polypeptide is an ADAM-H9 sequence comprising a heterologous signal sequence at its N-terminus. In certain host cells (*e.g.*, mammalian host cells), expression and/or secretion of an ADAM-H9 polypeptide can be increased through use of a heterologous signal sequence. As another example, an ADAM-H9 polypeptide or fragment thereof may be fused to a hexa-histidine tag to facilitate purification of bacterially expressed protein, or to a hemagglutinin tag to facilitate purification of protein expressed in eukaryotic cells. Further, fusion polypeptides can comprise, for example, poly-His or the antigenic identification peptides described in U.S. Patent No. 5,011,912 and in Hopp *et al*., *Bio*/*Technology* 6:1204, 1988. One such peptide is the FLAG^{®} peptide, which is highly antigenic and provides an epitope reversibly bound by a specific monoclonal antibody, enabling rapid assay and facile purification of expressed recombinant polypeptide. A murine hybridoma designated 4E11 produces a monoclonal antibody that binds the FLAG^{®} peptide in the presence of certain divalent metal cations, as described in U.S. Patent 5,011,912, hereby incorporated by reference. The 4E11 hybridoma cell line has been deposited with the ATCC under accession no. HB9259. Monoclonal antibodies that bind the FLAG^{®} peptide are available from Eastman Kodak Co., Scientific Imaging Systems Division, New Haven, Connecticut.

Encompassed by the invention are oligomers or fusion polypeptides that contain an ADAM-H9 polypeptide. Oligomers that can be used as fusion partners can be in the form of covalently linked or non-covalently-linked multimers, including dimers, trimers, or higher oligomers. In one aspect of the invention, the oligomers maintain the binding ability of the polypeptide components and provide therefor, bivalent, trivalent, and the like, binding sites. In an alternative embodiment the invention is directed to oligomers comprising multiple polypeptides joined *via* covalent or non-covalent interactions between peptide moieties fused to the polypeptides. Such peptides can be peptide linkers (spacers), or peptides that have the property of promoting oligomerization. Leucine zippers and certain polypeptides derived from antibodies are among the peptides that can promote oligomerization of the polypeptides attached thereto, as described in more detail below. Particularly preferred oligomers comprise a disintegrin domain of ADAM-H9. Such preferred oligomers are exemplified by an ADAM-H9dis operably linked to an Fc domain or leucine zipper domain.

Typically a linker will be a peptide linker moiety. The length of the linker moiety is chosen to optimize the biological activity of the polypeptide having an ADAM-H9 sequence and can be determined empirically without undue experimentation. The linker moiety should be long enough and flexible enough to allow an ADAM-H9 moiety to freely interact with a substrate or ligand. The preferred linker moiety is a peptide between about one and 30 amino acid residues in length, preferably between about two and 15 amino acid residues. Preferred linker moieties are --Gly-Gly-, GGGGS (SEQ ID NO:11), (GGGGS)ₙ (SEQ ID NO:12), GKSSGSGSESKS (SEQ ID NO:13), GSTSGSGKSSEGKG (SEQ ID NO:14), GSTSGSGKSSEGSGSTKG (SEQ ID NO:15), GSTSGSGKPGSGEGSTKG (SEQ ID NO: 16), or EGKSSGSGSESKEF (SEQ ID NO:17). Linking moieties are described, for example, in Huston; J. S., *et al.,* PNAS 85:5879 (1988), Whitlow, M., *et al.,* Protein Engineering 6:989 (1993), and Newton, D. L., *et al.,* Biochemistry 35:545 (1996). Other suitable peptide linkers are those described in U.S. Patents 4,751,180 and 4,935,233, which are hereby incorporated by reference. A DNA sequence encoding a desired peptide linker can be inserted between, and in the same reading frame as, DNA sequences of the invention, using any suitable conventional technique. For example, a chemically synthesized oligonucleotide encoding the linker can be ligated between the sequences. In particular embodiments, a fusion polypeptide comprises from two to four soluble ADAM polypeptides, separated by peptide linkers.

In embodiments where variants of an ADAM-H9 polypeptide are constructed to include a membrane-spanning domain, they will form a Type I membrane polypeptide. In such embodiments, it is preferable to link the fusion partner to the C-terminus of the ADAM-H9 polypeptide. Alternatively, the membrane-spanning polypeptides can be fused with known extracellular receptor domain polypeptides, for which the ligand is also known. Such fusion polypeptides can then be manipulated to control the intracellular signaling pathways triggered by the bound ADAM-H9 polypeptide. Polypeptides that span the cell membrane can also be fused with agonists or antagonists of cell-surface receptors, or cellular adhesion molecules to further modulate ADAM-H9 intracellular effects. In another aspect of the invention, interleukins can be situated between the preferred ADAM-H9 polypeptide fragment and other fusion polypeptide domains.

The ADAM-H9 polypeptides of the invention can also include a localization sequence to direct the polypeptide to particular cellular sites by fusion to appropriate organellar targeting signals or localized host proteins. A polynucleotide encoding a localization sequence, or signal sequence, can be ligated or fused at the 5' terminus of a polynucleotide encoding an ADAM-H9 polypeptide such that the signal peptide is located at the amino terminal end of the resulting fusion polynucleotide/polypeptide. In eukaryotes, the signal peptide functions to transport a polypeptide across the endoplasmic reticulum. The secretory protein is then transported through the Golgi apparatus, into secretory vesicles and into the extracellular space or the external environment. Signal peptides include pre-pro peptides that contain a proteolytic enzyme recognition site.

The localization sequence can be a nuclear-, an endoplasmic reticulum-, a peroxisome-, or a mitochondrial-localization sequence, or a localized protein. Localization sequences can be targeting sequences that are described, for example, in "Protein Targeting", chapter 35 of Stryer, L., Biochemistry (4th ed.). W. H. Freeman, 1995. Some important localization sequences include those targeting the nucleus (*e.g.*, KKKRK (SEQ ID NO:18)), mitochondria (MLRTSSLFTRRVQPSLFRNI LRLQST (SEQ ID NO:19)), endoplasmic reticulum (KDEL (SEQ ID NO:20)), peroxisome (SKF), plasma membrane (CAAX (SEQ ID NO:21), CC, CXC, or CCXX (SEQ ID NO:22)), cytoplasmic side of plasma membrane (fusion to SNAP-25), or the Golgi apparatus (fusion to furin).

In another embodiment, a polypeptide of the invention or fragments thereof may be fused to carrier molecules such as immunoglobulins for a variety of purposes including increasing the valency of polypeptide binding sites. As an example, fragments of the polypeptide may be fused through linker sequences to the Fc portion of an immunoglobulin. For a bivalent form of the polypeptide, such a fusion could be to the Fc portion of an IgG molecule. Other immunoglobulin isotypes may also be used to generate such fusions. For example, a polypeptide-IgM fusion would generate a decavalent form of the polypeptide of the invention. In one embodiment, the invention provides a fusion polypeptide having an Fc polypeptide domain and an ADAM-H9 polypeptide sequence as set forth in SEQ ID NO:6 from about amino acid number 73 to about 360, SEQ ID NO:8 from amino acid 1 or 16 to 285, or SEQ ID NO: 10 from amino acid 1 or 73 to 314 or 329.

The term "Fc polypeptide" as used herein includes native and mutein forms of polypeptides made up of the Fc region of an antibody comprising any or all of the CH domains of the Fc region. Truncated forms of such polypeptides containing the hinge region that promotes dimerization are also included. Preferred polypeptides comprise an Fc polypeptide derived from a human IgG1 antibody. As one alternative, an oligomer is prepared using polypeptides derived from immunoglobulins. Preparation of fusion polypeptides comprising certain heterologous polypeptides fused to various portions of antibody-derived polypeptides (including the Fc domain) has been described, *e.g.,* by Ashkenazi *et al.* (*PNAS USA* 88:10535, 1991); Byrn *et al.* (*Nature* 344:677, 1990); and Hollenbaugh and Aruffo ("Construction of Immunoglobulin Fusion Polypeptides", in *Current Protocols in Immunology,* Suppl. 4, pages 10.19.1 - 10.19.11, 1992). Methods for preparation and use of immunoglobulin-based oligomers are known in the art. One embodiment of the invention is directed to a dimer comprising two fusion polypeptides created by fusing a polypeptide of the invention to an Fc polypeptide derived from an antibody. A gene fusion encoding the polypeptide/Fc fusion polypeptide is inserted into an appropriate expression vector. Polypeptide/Fc fusion polypeptides are expressed in host cells transformed or transfected with the recombinant expression vector or recombinant polynucleotide encoding the fusion polypeptide, and allowed to assemble much like antibody molecules, whereupon interchain disulfide bonds form between the Fc moieties to yield divalent molecules. One suitable Fc polypeptide, described in PCT application WO 93/10151 (hereby incorporated by reference), is a single chain polypeptide extending from the N-terminal hinge region to the native C-terminus of the Fc region of a human IgG1 antibody. Another useful Fc polypeptide is the Fc mutein described in U.S. Patent 5,457,035 and in Baum *et al.,* (*EMBO J.* 13:3992, 1994) incorporated herein by reference. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors. The above-described fusion polypeptides comprising Fc moieties (and oligomers formed therefrom) offer the advantage of facile purification by affinity chromatography over Polypeptide A or Polypeptide G columns. In other embodiments, the polypeptides of the invention can be substituted for the variable portion of an antibody heavy or light chain. If fusion polypeptides are made with both heavy and light chains of an antibody, it is possible to form an oligomer with as many as four ADAM-H9 and/or ADAM extracellular regions.

Another method for preparing the oligomers of the invention involves use of a leucine zipper. Leucine zipper domains are peptides that promote oligomerization (dimers and trimers) of the polypeptides in which they are found. Leucine zippers were originally identified in several DNA-binding polypeptides (Landschulz *et al., Science* 240:1759, 1988), and have since been found in a variety of different polypeptides. The zipper domain comprises a repetitive heptad repeat, often with four or five leucine residues interspersed with other amino acids.

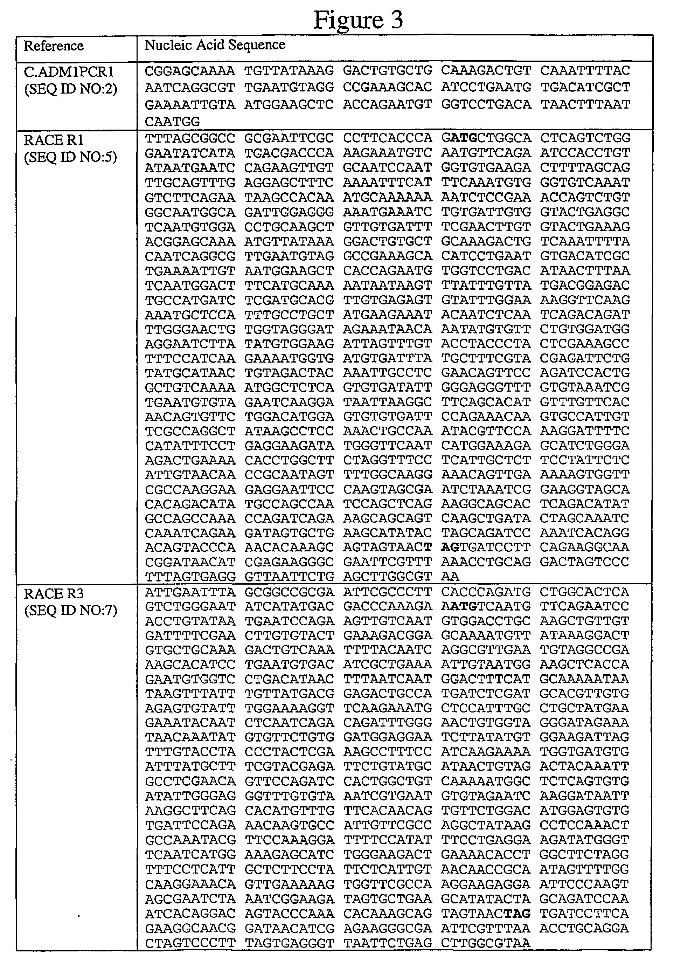

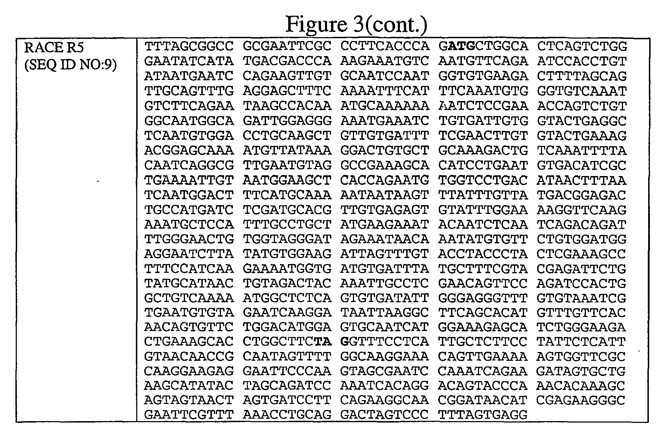

A chimeric or fusion polypeptide of the invention can be produced by standard recombinant DNA techniques. In one embodiment, polynucleotide fragments coding for the different polypeptide sequences are ligated together in-frame in accordance with conventional techniques, for example, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. Examples of polynucleotides encoding all or portions of the ADAM-H9 polypeptides are set forth in Figure 3. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Current Protocols in Molecular Biology, eds. Ausubel *et al.* John Wiley & Sons: 1992). Moreover, many expression vectors are commercially available that already encode a fusion moiety (*e.g.*, a GST polypeptide).

The invention further includes polypeptides with or without associated native-pattern glycosylation. Polypeptides expressed in yeast or mammalian expression systems (*e.g.*, COS-1 or CHO cells) can be similar to or significantly different from a native polypeptide in molecular weight and glycosylation pattern, depending upon the choice of expression system. Expression of polypeptides of the invention in bacterial expression systems, such as *E. coli,* provides non-glycosylated molecules. Further, a given preparation can include multiple differentially glycosylated species of the polypeptide. Glycosyl groups can be removed through conventional methods, in particular those utilizing glycopeptidase.

In another embodiment, modifications in the polypeptide or polynucleotide can be made using known techniques. Modifications of interest in the polypeptide sequences may include the alteration, substitution, replacement, insertion, or deletion of a selected amino acid residue in the coding sequence. For example, one or more of the cysteine residues may be deleted or replaced with another amino acid to alter the conformation of the molecule, an alteration which may involve preventing formation of incorrect intramolecular disulfide bridges upon folding or renaturation. Techniques for such alteration, substitution, replacement, insertion, or deletion are known to those skilled in the art (see, *e.g.,* U.S. Pat. No. 4,518,584). As another example, N-glycosylation sites in a polypeptide's extracellular domain can be modified to preclude glycosylation, allowing expression of a reduced carbohydrate analog in mammalian and yeast expression systems. N-glycosylation sites in eukaryotic polypeptides are characterized by an amino acid triplet Asn-X-Y, wherein X is any amino acid except Pro, and Y is Ser or Thr. Appropriate substitutions, additions, or deletions to the nucleotide sequence encoding these triplets will result in prevention of attachment of carbohydrate residues at the Asn side chain. Alteration of a single nucleotide, chosen so that Asn is replaced by a different amino acid, for example, is sufficient to inactivate an N-glycosylation site. Alternatively, the Ser or Thr can by replaced with another amino acid, such as Ala. Known procedures for inactivating N-glycosylation sites in polypeptides include those described in U.S. Patent 5,071,972 and EP 276,846, hereby incorporated by reference. Putative N-glycosylation sites include N23 of SEQ ID NO:1; N37 of SEQ ID NO:3; N53 of SEQ ID NO:4; N144 and N277 of SEQ ID NO:6; N10, N69, and N202 of SEQ ID NO:8; and N144, N277 of SEQ ID NO:10.

Additional variants within the scope of the invention include polypeptides that can be modified to create derivatives thereof by forming covalent or aggregative conjugates with other chemical moieties, such as glycosyl groups, lipids, phosphate, acetyl groups and the like. Covalent derivatives can be prepared by linking the chemical moieties to functional groups on amino acid side chains or at the N-terminus or C-terminus of a polypeptide. Conjugates comprising diagnostic (detectable) or therapeutic agents attached thereto are contemplated herein. Preferably, such alteration, substitution, replacement, insertion or deletion retains the desired activity of the polypeptide.

The invention also provides polynucleotides encoding ADAM-H9 polypeptides. The term "polynucleotide" refers to a polymeric form of nucleotides of at least 10 bases in length. The nucleotides can be ribonucleotides, deoxyribonucleotides, or modified forms of either type of nucleotide. The term includes single and double stranded forms of DNA or RNA. DNA includes, for example, cDNA, genomic DNA, chemically synthesized DNA. DNA amplified by PCR, and combinations thereof. The polynucleotides of the invention include full-length genes and cDNA molecules as well as a combination of fragments thereof. The polynucleotides of the invention are preferentially derived from human sources, but the invention includes those derived from non-human species, as well.

By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. The term therefore includes, for example, a recombinant polynucleotide molecule, which is incorporated into a vector, *e.g.*, an expression vector; into an autonomously replicating plasmid or virus; or into the genomic DNA of a prokaryote or eukaryote, or which exists as a separate molecule (*e.g.,* a cDNA) independent of other sequences.

An ADAM-H9 polynucleotide (1) described and/or claimed herein encodes a polypeptide comprising a sequence as set forth in SEQ ID Nos: 1, 3, 4, 6, 8, or 10; (2) has a sequence as set forth in SEQ ID Nos:2, 5, 7, or 9 (see, *e.g.*, Figure 3); (3) has sequences complementary to a sequence as set forth in SEQ ID Nos:2, 5, 7, or 9; (4) fragments of SEQ ID Nos:2, 5, 7, or 9 or their complements that specifically hybridize to the polynucleotide of (2) or (3) under moderate to highly stringent conditions; (5) polynucleotides of (1), (2), (3), or (4) wherein T can also be U (*e.g.,* RNA sequences). Also described are homologs of an ADAM-H9 polynucleotide of the invention. These polynucleotides can be identified in several ways, including isolation of genomic or cDNA molecules from a suitable source, or computer searches of available sequence databases. Oligonucleotides or polynucleotides corresponding to the amino acid sequences described herein can be used as probes or primers for the isolation of polynucleotide homologs or as query sequences for database searches. Degenerate oligonucleotide sequences can be obtained by "back-translation" from the amino acid sequences of the invention. The polymerase chain reaction (PCR) procedure can be employed to isolate and amplify a DNA sequence encoding an ADAM polypeptide or a desired combination of ADAM polypeptide fragments. Oligonucleotides that define the desired termini of a target DNA molecule are employed as 5' and 3' primers. Accordingly, fragments of the polynucleotides of the invention are useful as probes and primers to identify or amplify related sequence or obtain full-length sequences of an ADAM-H9 of the invention. The oligonucleotides can additionally contain recognition sites for restriction endonucleases, to facilitate insertion of the amplified combination of DNA fragments into an expression vector. PCR techniques are known in the art (see, *e.g., PCR Protocols: A Guide to Methods and Applications,* Innis *et. al.*, eds., Academic Press, Inc. (1990)).

The invention also includes polynucleotides and oligonucleotides that hybridize under reduced stringency conditions, more preferably moderately stringent conditions, and most preferably highly stringent conditions, to ADAM-H9 polynucleotides described herein. The basic parameters affecting the choice of hybridization conditions and guidance for devising suitable conditions are set forth by Sambrook, J., E. F. Fritsch, and T. Maniatis (1989, *Molecular Cloning: A Laboratory Manual,* Cold Spring Harbor Laboratory Press, Cold Spring Harbor, N.Y., chapters 9 and 11; and *Current Protocols in Molecular Biology,* 1995, F. M. Ausubel *et al.,* eds., John Wiley & Sons, Inc., sections 2.10 and 6.3-6.4, incorporated herein by reference), and can be readily determined by those having ordinary skill in the art based on, for example, the length and/or base composition of the polynucleotide. One way of achieving moderately stringent conditions involves the use of a prewashing solution containing 5 x SSC, 0.5% SDS, 1.0 mM EDTA (pH 8.0), hybridization buffer of about 50% formamide, 6 x SSC, and a hybridization temperature of about 55 °C (or other similar hybridization solutions, such as one containing about 50% formamide, with a hybridization temperature of about 42 °C), and washing conditions of about 60 °C, in 0.5 x SSC, 0.1% SDS. Generally, highly stringent conditions are defined as hybridization conditions as above, but with washing at approximately 68 °C, 0.2 x SSC, 0.1% SDS. SSPE (1xSSPE is 0.15M NaCl. 10 mM NaH₂PO₄, and 1.25 mM EDTA, pH 7.4) can be substituted for SSC (1xSSC is 0.15M NaCl and 15 mM sodium citrate) in the hybridization and wash buffers; washes are performed for 15 minutes after hybridization is complete. It should be understood that the wash temperature and wash salt concentration can be adjusted as necessary to achieve a desired degree of stringency by applying the basic principles that govern hybridization reactions and duplex stability, as known to those skilled in the art and described further below (see, *e.g.,* Sambrook *et al.,* 1989). When hybridizing a nucleic acid to a target polynucleotide of unknown sequence, the hybrid length is assumed to be that of the hybridizing nucleic acid. When nucleic acids of known sequence are hybridized, the hybrid length can be determined by aligning the sequences of the nucleic acids and identifying the region or regions of optimal sequence complementarity. The hybridization temperature for hybrids anticipated to be less than 50 base pairs in length should be 5 to 10 °C less than the melting temperature (Tₘ) of the hybrid, where Tₘ is determined according to the following equations. For hybrids less than 18 base pairs in length, Tₘ (°C) = 2(# of A + T bases) + 4(# of G + C bases). For hybrids above 18 base pairs in length, Tₘ (°C) = 81.5 + 16.6(log₁₀ [Na⁺]) + 0.41(% G + C) - (600/N), where N is the number of bases in the hybrid, and [Na⁺] is the concentration of sodium ions in the hybridization buffer ([Na⁺] for 1xSSC = 0.165M). Preferably, each such hybridizing nucleic acid has a length that is at least 25% (more preferably at least 50%, 60%, or 70%, and most preferably at least 80%) of the length of a polynucleotide of the invention to which it hybridizes, and has at least 60% sequence identity (more preferably at least 70%, 75%, 80%, 85%, 90%, 95%, 97.5%, or at least 99%, and most preferably at least 99.5%) with a polynucleotide of the invention to which it hybridizes.

"Conservatively modified variants" applies to both polypeptide and polynucleotide. With respect to particular polynucleotide, conservatively modified variants refer to codons in the polynucleotide which encode identical or essentially identical amino acids. Because of the degeneracy of the genetic code, a large number of functionally identical polynucleotides encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such variations are "silent variations," which are one species of conservatively modified variations. Every polynucleotide sequence herein that encodes a polypeptide also describes every possible silent variation of the nucleic acid. One of skill will recognize that each codon in a polynucleotide (except AUG, which is ordinarily the only codon for methionine) can be modified to yield a functionally identical molecule. Accordingly, each silent variation of a nucleic acid that encodes a polypeptide is implicit in each described sequence.

The invention also provides methodology for analysis of polynucleotides of the invention on "DNA chips" as described in Hacia *et al.,* Nature Genetics, 14:441-447 (1996). For example, high-density arrays of oligonucleotides comprising a sequence as set forth in SEQ ID Nos:2, 5, 7, 9, or a variant or mutant thereof are applied and immobilized to the chip and can be used to detect sequence variations in a population. Polynucleotides in a test sample are hybridized to the immobilized oligonucleotides. The hybridization profile of the target polynucleotide to the immobilized probe is quantitated and compared to a reference profile. The resulting genetic information can be used in molecular diagnosis. The density of oligonucleotides on DNA chips can be modified as needed.

The invention also provides genes corresponding to the polynucleotides disclosed herein. "Corresponding genes" are the regions of the genome that are transcribed to produce the mRNAs from which cDNA molecules are derived and may include contiguous regions of the genome necessary for the regulated expression of such genes. Corresponding genes may therefore include but are not limited to coding sequences, 5' and 3' untranslated regions, alternatively spliced exons, introns, promoters, enhancers, and silencer or suppressor elements. The corresponding genes can be isolated in accordance with known methods using the sequence information disclosed herein. Such methods include the preparation of probes or primers from the disclosed sequence information for identification and/or amplification of genes in appropriate genomic libraries or other sources of genomic materials.

Expression, isolation, and purification of the polypeptides and fragments of the invention can be accomplished by any suitable technique, including but not limited to the following methods.

The isolated polynucleotides of the invention may be operably linked to an expression control sequence such as the pMT2 or pED expression vectors disclosed in Kaufman *et al.,* Nucleic Acids Res. 19:4485 (1991); and Pouwels *et al. Cloning Vectors: A Laboratory Manual,* Elsevier, New York, (1985, and Supplements), in order to produce a polypeptide of the invention recombinantly. Many suitable expression control sequences are known in the art. General methods of expressing recombinant polypeptides are also known and are exemplified in R. Kaufman, Methods in Enzymology 185:537 (1990). As defined herein "operably linked" means that an isolated polynucleotide of the invention and an expression control sequence are situated within a vector or cell in such a way that the polypeptide encoded by the polynucleotide is expressed by a host cell which has been transformed (transfected) with the vector or polynucleotide operably linked to the control sequence.

In addition, a sequence encoding an appropriate signal peptide (native or heterologous) can be incorporated into expression vectors. The choice of signal peptide or leader can depend on factors such as the type of host cells in which the recombinant polypeptide is to be produced. Examples of heterologous signal peptides that are functional in mammalian host cells include the signal sequence for interleukin (IL)-7 (see, U.S. Patent 4,965,195); the signal sequence for IL-2 receptor (see, Cosman *et al., Nature* 312:768, 1984); the IL-4 receptor signal peptide (see, EP 367,566); the type I IL-1 receptor signal peptide (see, U.S. Patent 4,968,607); and the type II IL-1 receptor signal peptide (see, EP 460,846). A signal peptide that is functional in the intended host cells promotes extracellular secretion of the polypeptide. The signal peptide is cleaved from the polypeptide upon secretion of a polypeptide from the cell. A polypeptide preparation can include a mixture of polypeptide molecules having different N-terminal amino acids, resulting from cleavage of the signal peptide at more than one site.

Established methods for introducing DNA into mammalian cells have been described (Kaufman, R.J., *Large Scale Mammalian Cell Culture,* 1990, pp. 15-69). Additional protocols using commercially available reagents, such as Lipofectamine or Lipofectamine-Plus lipid reagent (Gibco/BRL), can be used to transfect cells (Felgner *et al., Proc. Natl. Acad Sci. USA* 84:7413, 1987). In addition, electroporation can be used to transfect mammalian cells using conventional procedures, such as those in Sambrook *et al.* (*Molecular Cloning: A Laboratory Manual,* 2 ed. Vol. 1-3, Cold Spring Harbor Laboratory Press, 1989). Selection of stable transformants can be performed using methods known in the art, such as, for example, resistance to cytotoxic drugs. Kaufman *et al., Meth. in Enzymology* 185:487, 1990, describes several selection schemes, such as dihydrofolate reductase (DHFR) resistance. A suitable strain for DHFR selection can be CHO strain DX-B11, which is deficient in DHFR (Urlaub *et al., Proc. Natl. Acad. Sci. USA* 77:4216, 1980). A plasmid expressing the DHFR cDNA can be introduced into strain DX-B11, and only cells that contain the plasmid can grow in the appropriate selective media. Other examples of selectable markers that can be incorporated into an expression vector include cDNAs conferring resistance to antibiotics, such as G418 and hygromycin B. Cells harboring the vector are selected on the basis of resistance to these compounds.

Alternatively, gene products can be obtained via homologous recombination, or "gene targeting" techniques. Such techniques employ the introduction of exogenous transcription control elements (such as the CMV promoter or the like) in a particular predetermined site on the genome, to induce expression of an endogenous ADAM-H9 of the invention. The location of integration into a host chromosome or genome can be easily determined by one of skill in the art, given the known location and sequence of the gene. In a preferred embodiment, the invention also contemplates the introduction of exogenous transcriptional control elements in conjunction with an amplifiable gene, to produce increased amounts of the gene product. The practice of homologous recombination or gene targeting is explained by Chappel in U.S. Patent No. 5,272,071 (see also Schimke, *et al. "Amplification of Genes in Somatic Mammalian cells,"* Methods in Enzymology 151:85 (1987), and by Capecchi, *et al., "The New Mouse Genetics: Altering the Genome by Gene Targeting,"* TIG 5:70 (1989)).

Suitable host cells for expression of the polypeptide include eukaryotic and prokaryotic cells. Mammalian host cells include, for example, the COS-7 line of monkey kidney cells (ATCC CRL 1651) (Gluzman *et al*., *Cell 23*:175, 1981), L cells, C127 cells, 3T3 cells (ATCC CCL 163), Chinese hamster ovary (CHO) cells, HeLa cells, BHK (ATCC CRL 10) cell lines, the CV1/EBNA cell line derived from the African green monkey kidney cell line CV1 (ATCC CCL 70) (see, McMahan *et al*. *EMBO J*. 10: 2821, 1991), human kidney 293 cells, human epidermal A431 cells, human Colo205 cells, other transformed primate cell lines, normal diploid cells, cell strains derived from *in vitro* culture of primary tissue, primary explants, HL-60, U937, HaK or Jurkat cells. Alternatively, it may be possible to produce the polypeptide in lower eukaryotes such as yeast or in prokaryotes such as bacteria. Potentially suitable yeast strains include *Saccharomyces cerevisiae, Schizosaccharomyces pombe, Kluyveromyces* strains, *Candida,* or any yeast strain capable of expressing heterologous polypeptides. Potentially suitable bacterial strains include, for example, *Escherichia coli, Bacillus subtilis, Salmonella typhimurium,* or any bacterial strain capable of expressing heterologous polypeptides. If the polypeptide is made in yeast or bacteria, it may be necessary to modify the polypeptide produced therein, for example by phosphorylation or glycosylation of the appropriate sites, in order to obtain the functional polypeptide. Such covalent attachments may be accomplished using known chemical or enzymatic methods. The polypeptide may also be produced by operably linking a polynucleotide of the invention to suitable control sequences in one or more insect expression vectors, and employing an insect expression system. Materials and methods for baculovirus/insect cell expression systems are commercially available in kit form from, *e.g.*, Invitrogen, San Diego, CA, U.S.A. (the MaxBac® kit), as well as methods described in Summers and Smith, Texas Agricultural Experiment Station Bulletin No. 1555 (1987), and Luckow and Summers, *Bio*/*Technology* 6:47 (1988), incorporated herein by reference. Cell-free translation systems could also be employed to produce polypeptides using RNAs derived from nucleic acid constructs disclosed herein. A host cell that comprises an isolated polynucleotide of the invention, preferably operably linked to at least one expression control sequence, is a "recombinant host cell".

In some instances it may be desirable to reduce the amount or activity of an ADAM-H9 polypeptide where overexpression or aberrant activity of ADAM-H9 is associated with a disorder or disease. Any method which neutralizes ADAM-H9 polypeptides or inhibits expression (either transcription or translation) of an ADAM-H9 polynucleotide can be used to reduce the biological activities of ADAM-H9 polypeptides.

Antagonists can be designed to reduce the level of endogenous ADAM-H9 expression, *e.g.,* using known antisense or ribozyme approaches to inhibit or prevent translation of ADAM-H9 mRNA transcripts; triple helix approaches to inhibit transcription of ADAM-H9 genes; or targeted homologous recombination to inactivate or "knock out" the ADAM-H9 genes or their endogenous promoters or enhancer elements. Such antisense, ribozyme, and triple helix antagonists may be designed to reduce or inhibit either unimpaired or, if appropriate, mutant ADAM-H9 activity.

Antisense RNA and DNA molecules act to directly block the translation of mRNA by hybridizing to targeted mRNA and preventing polypeptide translation. Antisense approaches involve the design of oligonucleotides (either DNA or RNA) that are complementary to a mRNA having an ADAM-H9 polynucleotide sequence. Absolute complementarity, although preferred, is not required. Oligonucleotides that are complementary to the 5' end of the message, *e.g.*, the 5' untranslated sequence up to, and including, the AUG initiation codon, should work most efficiently at inhibiting translation. Antisense nucleic acids are preferably oligonucleotides ranging from 6 to about 50 nucleotides in length. The oligonucleotides can be DNA, RNA, chimeric mixtures, derivatives or modified versions thereof, single-stranded or double-stranded. The oligonucleotide can be modified at the base moiety, sugar moiety, or phosphate backbone, for example, to improve stability of the molecule, hybridization, and the like. The oligonucleotide may include other appended groups such as peptides (*e.g.,* for targeting host cell receptors *in vivo*), or agents facilitating transport across the cell membrane (see, *e.g.*, Letsinger *et al.,* Proc. Natl. Acad. Sci. U.S.A. 86:6553,1989; Lemaitre *et al.,* Proc. Natl. Acad. Sci. 84:648, 1987; PCT Publication No. WO88/09810), or hybridization-triggered cleavage agents or intercalating agents (see, *e.g.,* Zon, Pharm. Res. 5:539, 1988). The antisense molecules are delivered to cells, which express a transcript having an ADAM-H9 polynucleotide sequence *in vivo* by, for example, direct injection into the tissue or cell derivation site, or modified antisense molecules, designed to target the desired cells (*e.g.,* antisense linked to peptides or antibodies that specifically bind receptors or antigens expressed on the target cell surface) can be administered systemically. Preferred approach utilizes a recombinant DNA construct in which the antisense oligonucleotide is placed under the control of a strong pol III or pol II promoter.

Ribozyme molecules designed to catalytically cleave mRNA transcripts having an ADAM-H9 polynucleotide sequence prevent translation of ADAM-H9 mRNA (see, *e.g.*, PCT International Publication WO90/11364 US Patent No. 5,824,519). Ribozymes are RNA molecules possessing the ability to specifically cleave other single-stranded RNA. Because ribozymes are sequence-specific, only mRNAs with particular sequences are inactivated. There are two basic types of ribozymes namely, tetrahymena-type (Hasselhoff, Nature, 334:585, 1988) and "hammerhead"-type. Tetrahymena-type ribozymes recognize sequences, which are four bases in length, while "hammerhead"-type ribozymes recognize base sequences 11-18 bases in length. The longer the recognition sequence, the greater the likelihood that the sequence will occur exclusively in the target mRNA species. Consequently, hammerhead-type ribozymes are preferable to tetrahymena-type ribozymes. As in the antisense approach, ribozymes can be composed of modified oligonucleotides and delivered using a DNA construct "encoding" the ribozyme under the control of a strong constitutive pol III or pol II promoter.

Alternatively, endogenous ADAM-H9 expression can be reduced by targeting DNA sequences complementary to a regulatory region of the target gene (*e.g.*, the target gene promoter and/or enhancers) to form triple helical structures that prevent transcription of the target gene (see generally, Helene, Anticancer Drug Des., 6(6), 569,1991; Helene, *et al*., Ann. N.Y. Acad. Sci., 660:27,1992; and Maher, Bioassays 14(12), 807,1992).

Antisense, ribozyme, and triple helix molecules may be prepared by any method known in the art for the synthesis of DNA and RNA molecules and include techniques for chemically synthesizing oligodeoxyribonucleotides and oligoribonucleotides such as, for example, solid phase phosphoramidite chemical synthesis using an automated DNA synthesizer available from Biosearch, Applied Biosystems. Phosphorothioate oligonucleotides may be synthesized by the method of Stein *et al.,* Nucl. Acids Res. 16:3209, 1988. Methylphosphonate oligonucleotides can be prepared by use of controlled pore glass polymer supports (Sarin *et al.*, Proc. Natl. Acad. Sci. U.S.A. 85:7448,1988). Alternatively, RNA molecules may be generated by *in vitro* and *in vivo* transcription of DNA sequences encoding the antisense RNA molecule.

Endogenous gene expression can also be reduced by inactivating or "knocking out" the target gene or its promoter using targeted homologous recombination (see, *e.g.,* Smithies, *et al.,* Nature 317:230,1985; Thomas and Capecchi, Cell 51, 503, 1987; Thompson, *et al.,* Cell 5, 313, 1989; each of which is incorporated by reference herein in its entirety). For example, a mutant non-functional target gene (or a completely unrelated DNA sequence) flanked by DNA homologous to the endogenous target gene can be used, with or without a selectable marker and/or a negative selectable marker. Insertion of the DNA construct, via targeted homologous recombination, results in inactivation of the target gene. Such approaches are particularly suited where modifications to embryonic stem cells can be used to generate non-human animal offspring with an inactive target gene (*e.g.*, see Thomas and Capecchi, 1987 and Thompson, 1989, *supra*; see also the "RNA interference" ("RNAi") technique of Grishok *et al.,* Science 287 (5462): 2494, 2000), and Dernburg *et al.,* Genes Dev. 14 (13): 1578, 2000).

As used herein, a "transgenic animal" is an animal that includes a transgene that is inserted into an embryonal cell and becomes a part of the genome of the animal that develops from that cell, or an offspring of such an animal. Any non-human animal that can be produced by transgenic technology is included in the invention, although mammals are preferred. Preferred mammals include non-human primates, sheep, goats, horses, cattle, pigs, rabbits, and rodents, such as, guinea pigs, hamsters, rats, gerbils, and mice.

A "transgene" is a polynucleotide that comprises one or more selected sequences (*e.g.*, encoding ribozymes that cleave ADAM-H9 mRNA, encoding an antisense molecule to an ADAM-H9 mRNA, encoding a mutant ADAM-H9 sequence, and the like) to be expressed in a transgenic animal. The polynucleotide is partly or entirely heterologous, *i.e.,* foreign, to the transgenic animal, or homologous to an endogenous gene of the transgenic animal, but which is designed to be inserted into the animal's genome at a location which differs from that of the natural gene. A transgene may include one or more promoters and any other DNA sequences, such as introns, necessary for expression of the selected DNA, all operably linked to the selected DNA, and may include an enhancer sequence.

The transgenic animal can be used in order to identify the impact of increased or decreased ADAM-H9 levels on a particular pathway or phenotype. Protocols useful in producing such transgenic animals are known in the art (see, *e.g.,* Brinster, *et al.,* Proc. Natl. Acad Sci. USA 82:4438, 1985; Jaenisch, Proc. Natl. Acad. Sci. USA 73:1260, 1976; Hogan, *et al.,* 1986, Manipulating the Mouse Embryo, Cold Spring Harbor Laboratory Press, Cold Spring Harbor, N.Y.; Jahner, *et al.,* Proc. Natl. Acad. Sci. USA 82:6927, 1985; Van der Putten, *et al.,* Proc Natl. Acad. Sci. USA 82:6148; Steward, *et al.,* EMBO J., 6:383, 1987; Jahner, *et al.,* Nature, 298:623, 1982).

In another embodiment, antibodies that are immunoreactive with the polypeptides of the invention are provided herein. The ADAM-H9 polypeptides, fragments, variants, fusion polypeptides, and the like, as set forth above, can be employed as "immunogens" in producing antibodies immunoreactive therewith. Such antibodies specifically bind to the polypeptides via the antigen-binding sites of the antibody. Specifically binding antibodies are those that will specifically recognize and bind with ADAM-H9 family polypeptides, homologues, and variants, but not with other molecules. In one preferred embodiment, the antibodies are specific for polypeptides having an ADAM-H9 amino acid sequence of the invention and do not cross-react with other polypeptides.

More specifically, the polypeptides, fragment, variants, fusion polypeptides, and the like contain antigenic determinants or epitopes that elicit the formation of antibodies. These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon polypeptide folding. Epitopes can be identified by any of the methods known in the art. Additionally, epitopes from the polypeptides of the invention can be used as research reagents, in assays, and to purify specific binding antibodies from substances such as polyclonal sera or supernatants from cultured hybridomas. Such epitopes or variants thereof can be produced using techniques known in the art such as solid-phase synthesis, chemical or enzymatic cleavage of a polypeptide, or using recombinant DNA technology.

Both polyclonal and monoclonal antibodies to the polypeptides of the invention can be prepared by conventional techniques. See, for example, *Monoclonal Antibodies, Hybridomas: A New Dimension in Biological Analyses,* Kennet *et al.* (eds.), Plenum Press, New York (1980); and *Antibodies: A Laboratory Manual,* Harlow and Land (eds.), Cold Spring Harbor Laboratory Press, Cold Spring Harbor, NY, (1988); Kohler and Milstein, (U.S. Pat. No. 4,376,110); the human B-cell hybridoma technique (Kosbor *et al.,* Immunology Today 4:72, 1983; Cole *et al.,* Proc. Natl. Acad. Sci. USA 80:2026, 1983); and the EBV-hybridoma technique (Cole *et al.,* 1985, Monoclonal Antibodies And Cancer Therapy, Alan R. Liss, Inc., pp. 77-96). Hybridoma cell lines that produce monoclonal antibodies specific for the polypeptides of the invention are also contemplated herein. Such hybridomas can be produced and identified by conventional techniques. For the production of antibodies, various host animals may be immunized by injection with an ADAM-H9 polypeptide, fragment, variant, or mutants thereof. Such host animals may include, but are not limited to, rabbits, mice, and rats, to name a few. Various adjutants may be used to increase the immunological response. Depending on the host species, such adjutants include, but are not limited to, Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjutants such as BCG (bacille Calmette-Guerin) and Corynebacterium parvum. The monoclonal antibodies can be recovered by conventional techniques. Such monoclonal antibodies may be of any immunoglobulin class including IgG, IgM, IgE, IgA, IgD, and any subclass thereof.

In addition, techniques developed for the production of "chimeric antibodies" (Takeda *et al.,* Nature, 314:452, 1985) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a porcine mAb and a human immunoglobulin constant region. The monoclonal antibodies of the invention also include humanized versions of murine monoclonal antibodies. Such humanized antibodies can be prepared by known techniques and offer the advantage of reduced immunogenicity when the antibodies are administered to humans. For example, transgenic mice into which genetic material encoding one or more human immunoglobulin chains has been introduced may be employed. Such mice may be genetically altered in a variety of ways. The genetic manipulation may result in human immunoglobulin polypeptide chains replacing endogenous immunoglobulin chains in at least some (preferably virtually all) antibodies produced by the animal upon immunization. Procedures for the production of chimeric and further engineered monoclonal antibodies include those described in Riechmann *et al.* (*Nature* 332:323, 1988), Liu *et al.* (*PNAS* 84:3439, 1987), Larrick *et al.* (*Bio*/*Technology* 7:934, 1989), and Winter and Harris (*TIPS* 14:139, Can, 1993). Procedures to generate antibodies transgenically can be found in GB 2,272,440, US Patent Nos. 5,569,825 and 5,545,806 and related patents claiming priority therefrom, all of which are incorporated by reference herein. Preferably, for use in humans, the antibodies are human or humanized; techniques for creating such human antibodies are also known. Transgenic animals for making human antibodies are available from, for example, Medarex Inc. (Princeton, NJ) and Abgennix Inc. (Fremont, CA).

Expression of a humanized immunoglobulin sequences in bacterial hosts may be used to select higher affinity humanized immunoglobulin sequences by mutagenizing the CDR regions and producing bacteriophage display libraries which may be screened for humanized immunoglobulin CDR variants which possess high affinity and/or high specificity binding to an ADAM-H9 polypeptide or fragment thereof. One potential advantage of such affinity sharpening is the generation of humanized immunoglobulin CDR variants that have improved binding affinity and/or reduced cross-reactivity with molecules other than an ADAM-H9 polypeptide or fragment thereof. Methods for producing phage display libraries having immunoglobulin variable region sequences are provided in the art (see, *e.g.,* Cesareni, FEBS Lett 307:66, 1992; Swimmer *et al.,* Proc. Natl. Acad. Sci. USA 89:3756, 1992; Gram *et al.,* Proc. Natl. Acad. Sci. USA 89:3576, 1992; Clackson *et al.,* Nature 352:624, 1991; Scott & Smith, Science 249:386, 1990; Garrard *et al.,* Bio/Techniques 9:1373, 1991. The resultant affinity sharpened CDR variant humanized immunoglobulin sequences are subsequently expressed in a suitable host.

Antibody fragments, which recognize specific epitopes, may be generated by known techniques. For example, such fragments include but are not limited to: the F(ab')₂ fragments which can be produced by pepsin digestion of the antibody molecule and the Fab fragments which can be generated by reducing the disulfide bridges of the (ab')₂ fragments. Alternatively, Fab expression libraries may be constructed (Huse *et al.,* Science, 246:1275, 1989) to allow rapid and easy identification of monoclonal Fab fragments with the desired specificity. Techniques described for the production of single chain antibodies (U.S. Pat. No. 4,946,778; Bird, Science 242:423, 1988; Huston *et al.,* Proc. Natl. Acad. Sci. USA 85:5879, 1988; and Ward *et al.,* Nature 334:544, 1989) can also be adapted to produce single chain antibodies against polypeptides containing ADAM-H9 amino acid sequences. In addition, antibodies to the ADAM-H9 polypeptide can, in turn, be utilized to generate anti-idiotype antibodies that "mimic" an ADAM-H9 polypeptide and that may bind to the ADAM-H9 polypeptide using techniques known to those skilled in the art. (See, *e.g.,* Greenspan & Bona, FASEB J 7(5):437, 1993; and Nissinoff, J. Immunol. 147(8):2429, 1991).

Screening procedures to identify such antibodies are known, and can involve immunoaffinity chromatography, for example. Antibodies can be screened for agonistic (*i.e.*, ligand-mimicking) properties. Such antibodies, upon binding to an ADAM-H9 polypeptide on the cell surface, can induce biological effects (*e.g.,* transduction of biological signals) similar to the biological effects induced when the naturally occurring ADAM-H9 binding partner binds to the polypeptide on the cell surface. Agonistic antibodies can be used to induce ADAM-H9 mediated co-stimulatory pathways or intercellular communication.

In addition, antibodies that block binding of a polypeptide having an ADAM-H9 sequence of the invention to its binding partner can be used to inhibit ADAM-H9 mediated intercellular communication or co-stimulation that results from such binding and/or to identify integrin cognates of ADAM-H9. Such blocking antibodies can be identified using any suitable assay procedure, such as by testing antibodies for the ability to inhibit binding of an ADAM-H9 polypeptide to certain cells expressing a binding partner (*e.g.*, an integrin) to the polypeptide. Alternatively, blocking antibodies can be identified in assays for the ability to inhibit a biological effect that results from binding of an ADAM-H9 polypeptide to target cells. In one embodiment, a flow cytometric integrin mAb based binding inhibition assay is used to show binding of ADAM-H9dis-Fc polypeptides to integrins expressed on the surface of endothelial cells. Human endothelial cells can be used in such assay. Human endothelial cells express αᵥβ₃, αᵥβ₅, β₁, β₄, α₁, α₂, α₃, α₄, α₅, and α₆ integrins. An ADAM-H9dis-Fc polypeptide is contacted with the endothelial cells. Monoclonal antibodies specific for human integrins α_{ν}β₃ (LM609, anti-CD51/61, Chemicon, Temecula, CA; Brooks *et al.,* Science 264:569, 1994), α₂β₁ (BHA2.1, anti-CD49b, Chemicon; Wang *et al.,* Mol. Biol. of the Cell 9:865, 1998), α₅β₁ (SAM-1, anti-CD49e, Biodesign; A. te Velde *et al.,* J. Immunol. 140:1548, 1988), (α_{3β1}, (ASC-6, anti-CD49c, Chemicon; Pattaramalai *et al.,* Exp. Cell. Res. 222: 281, 1996), α₄β₁, (HP2/1, anti-CD49d, Immunotech, Marseilles, France; Workshop of the 4^{th} International Conference on Human Leukocyte Differentiation Antigens, Vienna Austria, 1989, workshop number p091), α₆β₁ (GoH3, anti-CD49f, Immunotech; Workshop 4^{th} International Conference on Human Leukocyte Differentiation Antigens, workshop number p055), α₆β₄ (439-9B, anti-CD104, Pharmingen, San Diego, CA; Schlossman *et al.,* 1995 Leukocyte Typing V: White Cell Differentiation Antigens. Oxford University Press, New York), and αᵥβ₅ (MAB 1961, Chemicon; Weinaker, *et al,,* J. Biol. Chem. 269:6940, 1994) can bind specifically to HMVEC-d. Each of these antibodies is known to specifically block binding of the indicated integrin to its ligands (*e.g.*, fibronectin, vitronectin, fibrinogen). The ability of integrin mAbs to inhibit the binding of ADAM-H9dis-Pc polypeptides reveals which integrins the disintegrin domains bind and, indirectly, which integrin binding activities the disintegrin domains are able to antagonize. ADAM-H9dis-Fc polypeptides that bind to select integrins are further tested for the ability to disrupt integrin-ligand interactions and to modulate endothelial cell function, angiogenesis, and other biological activities *in vitro* and *in vivo.*

Disorders caused or exacerbated (directly or indirectly) by the interaction of ADAM-H9 with a cell surface-binding partner can thus be treated. A therapeutic method involves *in vivo* administration of a blocking antibody to a subject in an amount effective to inhibit ADAM-H9 binding-mediated biological activity. As used herein, a "subject" can be any animal, preferably a mammal (*e.g.,* canine, feline, bovine, porcine, equine, primates, and the like), and most preferably a human. Monoclonal antibodies are generally preferred for use in such therapeutic methods. In one embodiment, an antigen-binding antibody fragment is employed. Compositions comprising an antibody against an ADAM-H9 polypeptide, and a physiologically acceptable diluent, excipient, or carrier, are provided herein.

Also provided herein are conjugates comprising a detectable (*e.g.*, diagnostic) or therapeutic agent attached to the antibody. The conjugates find use in *in vitro* or *in vivo* procedures. The antibodies of the invention can also be used in assays to detect the presence of the polypeptides or fragments of the invention, either *in vitro* or *in vivo.* The antibodies also can be employed in purifying polypeptides or fragments of the invention by immunoaffinity chromatography.

Rational drug design is used to produce structural analogs of biologically active polypeptides of interest or of small molecules with which they interact, *e.g*., substrates, binding agents, inhibitors, agonists, antagonists, and the like. The methods provided herein can be used to fashion or identify agents which are more active or stable forms of the polypeptide or which enhance or interfere with the function of a polypeptide *in vivo* (Hodgson J, Biotechnology 9:19, 1991). The three-dimensional structure of a polypeptide of the invention, a ligand or binding partner, or of a polypeptide-binding partner complex, is determined by x-ray crystallography, by nuclear magnetic resonance, or by computer homology modeling or, most typically, by a combination of these approaches. Relevant structural information is used to design analogous molecules, to identify efficient inhibitors, or to identify small molecules that may bind to a polypeptide of the invention. The use of ADAM polypeptide structural information, preferably ADAM-H9 structural information, in molecular modeling software systems provides for the design of inhibitors or binding agents useful in modulating ADAM-H9 activity. A particular method comprises analyzing the three dimensional structure of ADAM-H9 polypeptides for likely binding sites of substrates or ligands, synthesizing a new molecule that incorporates a predictive reactive site, and assaying the new molecule as described further herein. Examples of algorithms, software, and methods for modeling substrates or binding agents based upon the three-dimensional structure of a protein are described in PCT publication WO107579A2.

It is also possible to isolate a target-specific antibody, selected by a functional assay, as described further herein, and then to solve its crystal structure thus yielding a pharmacore upon which subsequent drug design can be based. It is possible to bypass polypeptide crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

The invention provides methods for identifying agents that modulate ADAM-H9 activity or expression. Such methods included contacting a sample containing an ADAM-H9 polypeptide or polynucleotide with a test agent under conditions that allow for the test agent and the polypeptide or polynucleotide to interact and measuring the expression or activity of an ADAM-H9 polypeptide in the presence or absence of the test agent.

In one embodiment, a cell containing an ADAM-H9 polynucleotide is contacted with a test agent under conditions such that the cell and test agent are allowed to interact. Such conditions typically include normal cell culture conditions consistent with the particular cell type being utilized and which are known in the art. It may be desirable to allow the test agent and cell to interact under conditions associated with increased temperature or in the presence of regents that facilitate the uptake of the test agent by the cell. A control is treated similarly but in the absence of the test agent. Alternatively, the ADAM-H9 activity or expression may be measured prior to contact with the test agent (*e.g.*, the standard or control measurement) and then again following contact with the test agent. The treated cell is then compared to the control and a difference in the expression or activity of ADAM-H9 compared to the control is indicative of an agent that modulates ADAM-H9 activity or expression.

When ADAM-H9 expression is being measured, detecting the amount of mRNA encoding an ADAM-H9 polypeptide in the cell can be quantified by, for example, PCR or Northern blot. Where a change in the amount of ADAM-H9 polypeptide in the sample is being measured, detecting ADAM-H9 by use of anti-ADAM-H9 antibodies can be used to quantify the amount of ADAM-H9 polypeptide in the cell using known techniques.

A test agent can be any molecule typically used in the modulation of protein activity or expression and includes, for example, small molecules, chemicals, peptidomimetics, antibodies, peptides, polynucleotides (*e.g.*, antisense or ribozyme molecules), and the like. Accordingly, agents developed by computer based drug design can be tested in the laboratory using the assay and methods described herein to determine the activity of the agent on the modulation of ADAM-H9 activity or expression. Modulation of ADAM-H9 includes an increase or decrease in activity or expression.

An ADAM-H9 polypeptide of the invention (including fragments, variants, oligomers, and other forms) are useful in a variety of assays. For example, an ADAM-H9 of the invention can be used to identify binding partners of members of the ADAM family of polypeptides, which can also be used to modulate intercellular communication, co-stimulation, or immune cell activity. Alternatively, they can be used to identify non-binding-partner molecules or substances that modulate intercellular communication, co-stimulatory pathways, or immune cell activity.

ADAM-H9 polypeptides and fragments thereof can be used to identify binding partners. For example, they can be tested for the ability to bind a candidate-binding partner in any suitable assay, such as a conventional binding assay. To illustrate, an ADAM-H9 polypeptide or fragment thereof can be labeled with a detectable molecule (*e.g.*, a radionuclide, a chromophore, and an enzyme that catalyzes a colorimetric or fluorometric reaction and the like). The labeled polypeptide is contacted with cells expressing the candidate-binding partner. The cells then are washed to remove unbound-labeled polypeptide, and the presence of cell-bound label is determined by a suitable technique, chosen according to the nature of the label.

In one embodiment, a binding partner integrin is identified by the use of anti-integrin antibodies. The ability of integrin mAbs to inhibit the binding of ADAM-H9dis-Fc polypeptides reveals which integrin the disintegrin domain binds and, indirectly, which integrin binding activities the disintegrin domain is able to antagonize. ADAM-H9dis-Fc polypeptides that bind to select integrins are further tested for the ability to disrupt integrin-ligand interactions and to modulate endothelial cell function, angiogenesis, and other biological activities *in vitro* and *in vivo.*

In another example of a binding assay a recombinant expression vector containing the candidate binding partner cDNA is transfected into CVI-EBNA-1 cells. The cells are incubated for 1 hour at 37°C with various concentrations of, for example, a soluble ADAM-H9 polypeptide/Fc fusion polypeptide. Cells are washed and incubated with a constant saturating concentration of a ¹²⁵I-mouse anti-human IgG. After washing, cells are released *via* trypsinization. The mouse anti-human IgG employed above is directed against the Fc region of human IgG and can be obtained from Jackson Immunoresearch Laboratories, Inc., West Grove, PA. The antibody will bind to the Fc portion of any Fc polypeptide that has bound to the cells. Cell-bound ¹²⁵I-antibody is quantified on a Packard Autogamma counter.

Where an ADAM-H9 polypeptide binds or potentially binds to another polypeptide (*e.g.*, in a receptor-ligand interaction), the ADAM-H9 polynucleotide can also be used in interaction trap assays (see, *e.g.*, Gyuris *et al.,* Cell 75:791, 1993) to identify polynucleotides encoding the other polypeptide with which binding occurs or to identify inhibitors of the binding interaction. Polypeptides involved in these binding interactions can also be used to screen for peptide or small molecule inhibitors or agonists of the binding interaction.

Another type of suitable binding assay is a competitive binding assay. To illustrate, biological activity of a variant can be determined by assaying for the variant's ability to compete with the native polypeptide for binding to the candidate-binding partner. Competitive binding assays can be performed by conventional methodology. Reagents that can be employed in competitive binding assays include a radiolabeled ADAM-H9 fragment or variant and intact cells expressing ADAM-H9 (endogenous or recombinant) on the cell surface. Instead of intact cells, one could substitute a soluble binding partner/Fc fusion polypeptide bound to a solid phase through the interaction of Polypeptide A or Polypeptide G (on the solid phase) with the Fc moiety. Chromatography columns that contain Polypeptide A and G include those available from Pharmacia Biotech, Inc., Piscataway, NJ.

The influence of ADAM-H9 polypeptides, ADAM-H9 fragments and antibodies on intercellular communication, co-stimulation, integrin binding, endothelial cell migration, angiogenesis or immune cell activity can be assayed by contacting a cell or a group of cells with a polynucleotide, polypeptide, agonist or antagonist, to induce, enhance, suppress, or arrest cellular communication, costimulation, integrin binding, endothelial cell migration, angiogenesis or activity in the target cells. Identification of ADAM-H9 polypeptides, agonists or antagonists can be carried out *via* a variety of assays known to those skilled in the art. Included in such assays are those that evaluate the ability of an ADAM-H9 polypeptide to influence intercellular communication, co-stimulation, integrin binding, endothelial cell migration, or angiogenesis. Such an assay would involve, for example, the analysis of cell-cell interactions (*e.g.,* through integrin-related binding) in the presence of an ADAM-H9 polypeptide or soluble disintegrin fragment thereof. In such an assay, one would determine a rate of cell-cell interaction, cell matrix interaction, or integrin associated binding in the presence of a polypeptide having an ADAM-H9 sequence and then determine if such binding or interaction is altered in the presence of, *e.g.,* a soluble disintegrin ADAM-H9 (ADAM-H9dis) sequence. Exemplary assays for this aspect of the invention includes endothelial migration assays. Other assays are known in the art.

In another aspect, the invention provides a method of detecting the ability of a test agent to affect the cell-cell interaction, cell-matrix interaction, integrin-associated binding activity, endothelial cell migratory activity, or angiogenic activity of the test agent on a cell or culture. In this aspect, the method comprises: (1) contacting a first group of target cells with a test agent including a polypeptide having an ADAM-H9 sequence *(e.g.,* SEQ ID Nos:1, 3, 4, 6, 8, or 10; or a soluble ADAM-H9 disintegrin moiety), a ligand or receptor for an ADAM-H9 polypeptide, or fragment thereof, under conditions appropriate to the particular assay being used; (2) measuring the net rate of cell-cell interaction, cell-matrix interaction, integrin-associated binding activity, endothelial cell migratory activity, or angiogenic activity among the target cells; and (3) observing the net rate of cell-cell interaction, cell-matrix interaction, integrin-associated binding activity, endothelial cell migratory activity, or angiogenic activity among control cells containing an ADAM-H9 polypeptide ligand or fragments thereof, in the absence of a test agent, under otherwise identical conditions as the first group of cells. In this embodiment, the net rate of intercellular communication or co-stimulation in the control cells is compared to that of the cells treated with both an ADAM-H9 molecule as well as a test agent. The comparison will provide a difference in the net rate of cell-cell interaction, cell-matrix interaction, integrin-associated binding activity, endothelial cell migratory activity, or angiogenic activity indicative of an agent that modulates ADAM-H9 activity. The test agent can function as an effector by either activating or up-regulating, or by inhibiting or down-regulating cell-cell interaction, cell-matrix interaction, integrin-associated binding, endothelial cell migratory activity, or angiogenic activity.

A polypeptide of the invention may exhibit cytokine production or inhibition activity, cell proliferation (either inducing or inhibiting), or cell differentiation (either inducing or inhibiting) activity. Many polypeptide factors discovered to date, including all known cytokines, have exhibited activity in one or more cell proliferation assays, and hence the assays serve as a convenient confirmation of cytokine activity. The activity of a polypeptide of the invention is evidenced by any one of a number of routine factor dependent cell proliferation assays for cell lines including, without limitation, 32D, DA2, DA1G, T10, B9, B9/11, BaF3, MC9/G, M+ (preB M+), 2E8, RB5, DA1, 123, T1165, HT2, CTLL2, TF-1, Mo7e and CMK. The activity of an ADAM-H9 polypeptide of the invention may be measured by the following methods:
Assays for T-cell or thymocyte proliferation include, without limitation, those described in: Current Protocols in Immunology, Ed. by Coligan *et al.,* Pub. Greene Publishing Associates and Wiley-Interscience (Chapter 3, *In vitro* assays for Mouse Lymphocyte Function 3.1-3.19; Chapter 7, Immunologic studies in Humans); Takai *et al.,* J. Immunol. 137:3494, 1986; Bertagnolli *et al.,* J. Immunol. 145:1706, 1990; Bertagnolli *et al.,* Cell. Immunol. 133:327, 1991; Bertagnolli, *et al.,* J. Immunol. 149:3778, 1992; Bowman *et al.,* J. Immunol. 152: 1756, 1994.
Assays for cytokine production and/or proliferation of spleen cells, lymph node cells or thymocytes include, without limitation, those described in: Polyclonal T cell stimulation, Kruisbeek, A. M. and Shevach, Vol 1 pp. 3.12.1-3.12.14, and Measurement of mouse and human Interferon γ, Schreiber, R. D. Vol 1 pp. 6.8.1-6.8.8. In Current Protocols in Immunology. E. M. Coligan eds. John Wiley and Sons, Toronto. 1994; Coligan eds., John Wiley and Sons, Toronto, 1994.
Assays for proliferation and differentiation of hematopoietic and lymphopoietic cells include, without limitation, those described in: Measurement of Human and Murine Interleukin 2 and Interleukin 4, Bottomly *et al.*, In Current Protocols in Immunology. Coligan eds. Vol 1 pp. 6.3.1-6.3.12, John Wiley and Sons, Toronto. 1991; de Vries *et al.,* J. Exp. Med. 173:1205, 1991; Moreau *et al.,* Nature 336:690, 1988; Greenberger *et al.,* Proc. Natl. Acad. Sci. U.S.A. 80:2931, 1983; Measurement of mouse and human interleukin 6, Nordan, R. In Current Protocols in Immunology. Coligan eds. Vol 1 pp. 6.6.1-6.6.5, John Wiley and Sons, Toronto. 1991; Smith *et al.,* Proc. Natl. Acad. Sci. U.S.A. 83:1857, 1986; Measurement of human Interleukin 11, Bennett *et al.,* In Current Protocols in Immunology. Coligan eds. Vol 1 pp. 6.15.1 John Wiley and Sons, Toronto. 1991; Measurement of mouse and human Interleukin 9, Ciarletta *et al.,* In Current Protocols in Immunology. Coligan eds. Vol 1 pp. 6.13.1, John Wiley and Sons, Toronto. 1991.
Assays for T-cell clone responses to antigens (which will identify, among others, polypeptides that affect APC-T cell interactions as well as direct T-cell effects by measuring proliferation and cytokine production) include, without limitation, those described in: Current Protocols in Immunology, Coligan eds., Pub. Greene Publishing Associates and Wiley-Interscience (Chapter 3, *In vitro* assays for Mouse Lymphocyte Function; Chapter 6, Cytokines and their cellular receptors; Chapter 7, Immunologic studies in Humans); Weinberger *et al.,* Proc. Natl. Acad. Sci. USA 77:6091, 1980; Weinberger *et al.,* Eur. J. Immun. 11:405, 1981; Takai *et al.,* J. Immunol. 137:3494, 1986; Takai *et al.,* J. Immunol. 140:508, 1988.
Assays for thymocyte or splenocyte cytotoxicity include, without limitation, Current Protocols in Immunology, Coligan eds., Pub. Greene Publishing Associates and Wiley-Interscience (*In vitro* assays for Mouse Lymphocyte Function pp. 3.1-3.19; Chapter 7, Immunologic studies in Humans); Herrmann *et al.,* Proc. Natl. Acad. Sci. USA 78:2488, 1981; Herrmann *et al.,* J.Immunol. 128:1968, 1982; Handa *et al.,* J.Immunol. 135:1564, 1985; Takai *et al.,* J.Immunol. 137:3494, 1986; Takai *et al.,* J.Immunol. 140:508, 1988; Bowman *et al.,* J.Virol. 61:1992; Bertagnolli *et al.,* Cell. Imm. 133:327, 1991; Brown *et al.,* J.Immun. 153:3079, 1994.
Assays for T-cell-dependent IgG responses and isotype switching (which will identify, among others, polypeptides that modulate T-cell dependent antibody responses and that affect Thl/Th2 profiles) include, without limitation, those described in: Maliszewski, J. Immunol. 144:3028, 1990; and Assays for B cell function: *In vitro* antibody production, Mond, J. J. and Brunswick, M. In Current Protocols in Immunology. Coligan eds. Vol 1 pp. 3.8.1-3.8.16, Wiley and Sons, Toronto. 1994.
Mixed lymphocyte reaction (MLR) assays (which will identify, among others, polypeptides that generate predominantly Th1 and CTL responses) include, without limitation, those described in: Current Protocols in Immunology, Coligan eds., Pub. Greene Publishing Associates and Wiley-Interscience (*In vitro* assays for Mouse Lymphocyte Function pp 3.1-3.19; Chapter 7, Immunologic studies in Humans); Takai *et al.*, 1986, *supra*; Takai *et al*., 1988, *supra*; Bertagnolli *et al.,* J. Immunol. 149:3778, 1992.
Dendritic cell-dependent assays (which will identify, among others, polypeptides expressed by dendritic cells that activate naive T-cells) include, without limitation, those described in: Guery *et al.,* J. Immunol. 134:536, 1995; Inaba *et al.,* J. of Exp. Med. 173:549, 1991; Macatonia *et al*., J. Immunol. 154:5071, 1995; Porgador *et al.,* J. of Exp. Med. 182:255, 1995; Nair *et al.,* J. Virol. 67:4062, 1993; Huang *et al.,* Science 264:961, 1994; Macatonia *et al*., J. of Exp. Med. 169:1255, 1989; Bhardwaj *et al.,* J. Clin. Invest. 94:797, 1994; and Inaba *et al*., J. of Exp. Med. 172:631, 1990.
Assays for lymphocyte survival/apoptosis (which will identify, among others, polypeptides that prevent apoptosis after superantigen induction and polypeptides that regulate lymphocyte homeostasis) include, without limitation, those described in: Darzynkiewicz *et al.,* Cytometry 13:795, 1992; Gorczyca *et al.,* Leukemia 7:659, 1993; Gorczyca *et al.,* Cancer Research 53:1945, 1993; Itoh *et al.,* Cell 66:233, 1991; Zacharchuk, J. Immunol. 145:4037, 1990; Zamai *et al.,* Cytometry 14:891, 1993; Gorczyca *et al.,* Int. J. of Oncology 1:639, 1992.
Assays for polypeptides that influence early steps of T-cell commitment and development include, without limitation, those described in: Antica *et al.,* Blood 84:111, 1994; Fine *et al.,* Cell. Immunol. 155:111, 1994; Galy *et al.,* Blood 85:2770, 1995; Toki *et al.,* Proc. Nat. Acad Sci. USA 88:7548, 1991.
Assays for embryonic stem cell differentiation (which will identify, among others, polypeptides that influence embryonic differentiation hematopoiesis) include, without limitation, those described in: Johansson *et al* Cell. Biol. 15:141, 1995; Keller *et al.,* Mol. and Cell. Biol. 13:473, 1993; McClanahan *et al.,* Blood 81:2903, 1993.
Assays for stem cell survival and differentiation (which will identify, among others, polypeptides that regulate lympho-hematopoiesis) include, without limitation, those described in: Methylcellulose colony forming assays, Freshney, M. G. In Culture of Hematopoietic Cells. R. I. Freshney, *et al.* eds. Vol pp. 265-268, Wiley-Liss, Inc., New York, N.Y. 1994; Hirayama *et al.,* Proc. Natl. Acad. Sci. USA 89:5907-5911, 1992; Primitive hematopoietic colony forming cells with high proliferative potential, McNiece, I. K. and Briddell, R. A. In Culture of Hematopoietic Cells. R. I. Freshney, *et al.* eds. Vol pp. 23-39, Wiley-Liss, Inc., New York, N.Y. 1994; Neben *et al.,* Exp. Hematol. 22:353, 1994; Cobblestone area forming cell assay, Ploemacher, In Culture of Hematopoietic Cells. Freshney, *et al.* eds. Vol pp. 1-21, Wiley-Liss, Inc., New York, N.Y. 1994; Long term bone marrow cultures in the presence of stromal cells, Spooncer *et al.* In Culture of Hematopoietic Cells. Freshney, *et al*. eds. Vol pp. 163-179, Wiley-Liss, Inc., New York, N.Y. 1994; Long term culture initiating cell assay, Sutherland, In Culture of Hematopoietic Cells. Freshney, *et al.* eds. Vol pp. 139-162, Wiley-Liss, Inc., New York, N.Y. 1994.
Assays for tissue generation activity include, without limitation, those described in: Patent Publication No. WO95/16035 (bone, cartilage, tendon); Patent Publication No. WO95/05846 (nerve, neuronal); Patent Publication No. WO91/07491 (skin, endothelium). Assays for wound healing activity include, without limitation, those described in: Winter, Epidermal Wound Healing, pps. 71-112 (Maibach, and Rovee, eds.), Year Book Medical Publishers, Inc., Chicago, as modified by Eaglstein and Mertz, J. Invest. Dermatol 71:382-84 (1978).
Assays for activin/inhibin activity include, without limitation, those described in: Vale *et al.,* Endocrinol. 91:562, 1972; Ling *et al.,* Nature 321:779, 1986; Vale *et al.,* Nature 321:776, 1986; Mason *et al.,* Nature 318:659, 1985; Forage *et al.,* Proc. Natl. Acad. Sci. USA 83:3091, 1986.
Assays for cell movement and adhesion include, without limitation, those described in: Current Protocols in Immunology, Coligan eds., Pub. Greene Publishing Associates and Wiley-Interscience (Chapter 6.12, Measurement of α and β Chemokines 6.12.1-6.12.38; Taub *et al.* J. Clin. Invest. 95:1370-1376, 1995; Lind *et al.* APMIS 103:140, 1995; Muller *et al.* Eur. J. Immunol. 25: 1744; Gruber *et al.* J. Immunol. 152:5860, 1994; Johnston *et al.* J. Immunol. 153: 1762, 1994.
Assay for hemostatic and thrombolytic activity include, without limitation, those described in: Linet *et al.,* J. Clin. Pharmacol. 26:131, 1986; Burdick *et al.,* Thrombosis Res. 45:413,1987; Humphrey *et al.,* Fibrinolysis 5:71, 1991; Schaub, Prostaglandins 35:467, 1988.
Assays for receptor-ligand activity include, without limitation, those described in: Current Protocols in Immunology, Coligan eds., Pub. Greene Publishing Associates and Wiley-Interscience (Chapter 7.28, Measurement of Cellular Adhesion under static conditions 7.28.1-7.28.22), Takai *et al.,* Proc. Natl. Acad. Sci. USA 84:6864, 1987; Bierer *et al.,* J. Exp. Med. 168:1145, 1988; Rosenstein *et al.,* J. Exp. Med. 169:149, 1989; Stoltenborg *et al.,* J. Immunol. Methods 175:59, 1994; Stitt *et al.,* Cell 80:661, 1995.
Assays for cadherin adhesive and invasive suppressor activity include, without limitation, those described in: Hortsch *et al.* J Biol. Chem. 270(32):18809, 1995; Miyaki *et al.* Oncogene 11: 2547, 1995; Ozawa *et al.* Cell 63:1033, 1990.
A polynucleotide encoding a polypeptide having an ADAM-H9 sequence provided by the invention can be used for numerous diagnostic or other useful purposes. A polynucleotide of the invention (*e.g.*, SEQ ID Nos:2, 5, 7, or 9) can be used as markers for tissues in which the corresponding polypeptide is preferentially expressed, as molecular weight markers on Southern gels, as chromosome markers or tags to identify chromosomes or to map related gene positions, to compare with endogenous DNA sequences in subjects to identify potential genetic disorders, as probes to hybridize and thus discover novel related polynucleotides, as a source of information to derive PCR primers for genetic fingerprinting, as a probe to "subtract-out" known polynucleotides in the process of discovering other novel nucleic acids, as an antigen to raise anti-DNA antibodies or elicit another immune response, and for gene therapy.
Probes and Primers. Among the uses of the disclosed ADAM-H9 polynucleotides, and combinations of fragments thereof, is the use of fragments as probes or primers. Such fragments generally comprise at least about 17 contiguous nucleotides of a DNA sequence. In other embodiments, a DNA fragment comprises at least 30, or at least 60 contiguous nucleotides of a DNA sequence. The basic parameters affecting the choice of hybridization conditions and guidance for devising suitable conditions are set forth by Sambrook *et al.,* 1989 and are described in detail above. Using knowledge of the genetic code in combination with the amino acid sequences set forth above, sets of degenerate oligonucleotides can be prepared. Such oligonucleotides are useful as primers, *e.g.*, in polymerase chain reactions (PCR), whereby DNA fragments are isolated and amplified. In certain embodiments, degenerate primers can be used as probes for non-human genetic libraries. Such libraries would include but are not limited to cDNA libraries, genomic libraries, and even electronic EST (express sequence tag) or DNA libraries. Homologous sequences identified by this method would then be used as probes to identify non-human homologues of the ADAM-H9 sequence identified herein.
Chromosome Mapping. The polynucleotides encoding ADAM-H9 polypeptides, and the disclosed fragments and combinations of these polynucleotides, can be used by those skilled in the art using known techniques to identify the human chromosome to which these sequences map. Useful techniques include, but are not limited to, using the sequence or portions, including oligonucleotides, as a probe in various known techniques such as radiation hybrid mapping (high resolution), *in situ* hybridization to chromosome spreads (moderate resolution), and Southern blot hybridization to hybrid cell lines containing individual human chromosomes (low resolution). The following web site provides additional information about radiation hybrid mapping: www-genome.wi.mit.edu/ftp/distribution/human_STS_releases/july97/07-97.INTRO.html.

A polynucleotide encoding a polypeptide having an ADAM-H9 sequence of the invention, and the disclosed fragments and combinations of these polynucleotides can be used to analyze abnormalities associated with the genes corresponding to ADAM-H9 polypeptides. This enables one to distinguish conditions in which this marker is rearranged or deleted. In addition, polynucleotides of the invention or a fragment thereof can be used as a positional marker to map other genes of unknown location. The polynucleotide can be used in developing treatments for any disorder mediated (directly or indirectly) by defective, or insufficient amounts of, genes (*e.g.*, an ADAM-H9-associated disorder) corresponding to the polynucleotides of the invention. The polynucleotides and associated sequences disclosed herein permit the detection of defective genes, and the replacement thereof with normal genes. Defective genes can be detected in *in vitro* diagnostic assays, and by comparison of the polynucleotide sequences disclosed herein with that of a gene derived from a subject suspected of harboring a defect in this gene or having an ADAM-H9-associated disorder.

Uses of ADAM-H9 polypeptides and peptide fragments thereof include, but are not limited to, the following: delivery agents; therapeutic and research reagents; molecular weight and isoelectric focusing markers; controls for peptide fragmentation; identification of unknown polypeptides; and preparation of antibodies.

The ADAMS-H9 polypeptides described and/or claimed herein (*e*.*g*., SEQ ID Nos:1, 3, 4, 6, 8, or 10) of the invention can be used as polypeptide purification reagents. For example, ADAM-H9 polypeptides can be attached to a solid support material and used to purify its binding partners (*e.g.*, an integrin molecule) by affinity chromatography. In particular embodiments, a polypeptide is attached to a solid support by conventional procedures. As one example, chromatography columns containing functional groups that will react with amino acid side chains of polypeptides are available (Pharmacia Biotech, Inc., Piscataway, NJ). In an alternative, an ADAM-H9-Fc polypeptide is attached to Polypeptide A- or Polypeptide G-containing chromatography columns through interaction with the Fc moiety. The polypeptide also finds use in purifying or identifying cells that express a binding partner on the cell surface. Polypeptides are bound to a solid phase such as a column chromatography matrix or a similar suitable substrate. For example, magnetic microspheres can be coated with the polypeptides and held in an incubation vessel through a magnetic field. Suspensions of cell mixtures containing the binding partner expressing cells are contacted with the solid phase having the polypeptides thereon. Cells expressing the binding partner on the cell surface bind to the polypeptides on the solid phase, and unbound cells then are washed away. Alternatively, the polypeptides can be conjugated to a detectable moiety, then incubated with cells to be tested for binding partner expression. After incubation, unbound-labeled matter is removed and the presence or absence of the detectable moiety on the cells is determined.

Carriers and Delivery Agents. The polypeptides also find use as carriers for delivering agents attached thereto to cells bearing identified binding partners (*e.g.*, an integrin). The polypeptides thus can be used to deliver diagnostic or therapeutic agents to such cells in *in vitro* or *in vivo* procedures. Detectable (diagnostic) and therapeutic agents that can be attached to a polypeptide include, but are not limited to, toxins, other cytotoxic agents, drugs, radionuclides, chromophores, enzymes that catalyze a colorimetric or fluorometric reaction, and the like, with the particular agent being chosen according to the intended application. Among the toxins are ricin, abrin, diphtheria toxin, *Pseudomonas aeruginosa* exotoxin A, ribosomal inactivating polypeptides, mycotoxins such as trichothecenes, and derivatives and fragments (*e.g.*, single chains) thereof. Radionuclides suitable for diagnostic use include, but are not limited to, ¹²³I, ¹³¹I, ^{99m}Tc, ¹¹¹In, and ⁷⁶Br. Examples of radionuclides suitable for therapeutic use are ¹³¹I, ²¹¹At, ⁷⁷Br, ¹⁸⁶Re, ¹⁸⁸Re, ²¹²Pb, ²¹²Bi, ¹⁰⁹Pd, ⁶⁴Cu, and ⁶⁷Cu. Such agents can be attached to the polypeptide by any suitable conventional procedure. The polypeptide comprises functional groups on amino acid side chains that can be reacted with functional groups on a desired agent to form covalent bonds, for example. Alternatively, the polypeptide or agent can be derivatized to generate or attach a desired reactive functional group. The derivatization can involve attachment of one of the bifunctional coupling reagents available for attaching various molecules to polypeptides (Pierce Chemical Company, Rockford, Illinois). Of particular interest are soluble ADAM-H9 disintegrins that can be used to target cells expressing a binding partner for the ADAM-H9 disintegrin moiety (*e.g.,* an integrin). Such soluble ADAM-H9 disintegrins can be used to target reagents to cells expressing, for example, the disintegrin's cognate integrin. Similarly, and as discussed more fully below, antibodies specific for an ADAM-H9 polypeptide can be labeled with a diagnostic or therapeutic agent and used to target the diagnostic or therapeutic to cells expressing an ADAM-H9 polypeptide.

ADAM-H9 polypeptides and ADAM-H9 fragments (*e.g*., fragments having disintegrin activity) can be employed in modulating a biological activity of an ADAM polypeptide, particularly ADAM-H9 polypeptide, in *in vitro* or *in vivo* procedures. Encompassed within the invention are domains of ADAM-H9 polypeptides that act as modulators of native ADAM polypeptide function, including native ADAM-H9 activity, when expressed as fragments or as components of fusion polypeptides. For example, a substantially purified polypeptide domain of the invention can be used to inhibit binding of an ADAM-H9 polypeptide to endogenous binding partners. Such use effectively would block ADAM-H9 interactions and inhibit ADAM-H9 activities. In still another aspect of the invention, a soluble form of an ADAM-H9 binding partner (*e.g.*, a soluble integrin domain) is used to bind to, and competitively inhibit activation of the endogenous ADAM-H9 polypeptide.

In another embodiment, the invention is directed to methods of inhibiting the binding of an integrin to its ligand, and thereby inhibiting the biological activity of the integrin, comprising contacting the integrin with an effective amount of an ADAM-H9dis polypeptide. The invention is further directed to methods of inhibiting endothelial cell migration and methods of inhibiting angiogenesis comprising administering an effective amount of an ADAM-H9dis polypeptide. In some embodiments the ADAM-H9dis polypeptide is in the form of a multimer, preferably a leucine zipper multimer or Fc polypeptide. Alternatively, substantially purified or modified ADAM-H9 polypeptides of the invention can be administered to modulate interactions between ADAM-H9 polypeptides and ADAM-H9 binding partners that are not membrane-bound.

Antibodies that bind to ADAM-H9 polypeptides can inhibit ADAM-H9 polypeptide activity and may act as antagonists. For example, antibodies that specifically bind to one or more epitopes of an ADAM-H9 polypeptide, or epitope of conserved variants of ADAM-H9 polypeptides, or fragments can be used to inhibit ADAM-H9 activity. By "specifically bind" means that an antibody to an ADAM-H9 polypeptide or fragment thereof will not cross-react with unrelated polypeptides. Preferably such an antibody will not cross-react with other members of the ADAM family.

In an alternative aspect, the invention further encompasses the use of agonists of ADAM-H9 activity to treat or ameliorate the symptoms of a disease for which increased disintegrin activity is beneficial. In a preferred aspect, the invention entails administering compositions comprising an ADAM-H9 polynucleotide (*e.g*., comprising SEQ ID Nos 5, 7, or 9) or fragment thereof or a polypeptide comprising an ADAM-H9 amino acid sequence (*e.g.,* SEQ ID Nos 6, 8, or 10) or fragment thereof. The administering may be to cells *in vitro,* to cells *ex vivo,* to cells *in vivo,* and/or to a multicellular organism. Preferred therapeutic forms include soluble forms of an ADAM-H9 having disintegrin activity. Such a soluble ADAM-H9 disintegrin will bind to its binding partner (*e.g.*, an integrin) and stimulate a biological activity associated with the binding partner.

In still another aspect of the invention, the compositions comprise administering a polynucleotide encoding an ADAM-H9 polypeptide for expression in a host organism for treatment of disease. Particularly preferred in this regard is expression in a human subject for treatment of a dysfunction associated with aberrant (*e.g.*, decreased) endogenous activity of an ADAM-H9 polypeptide. Furthermore, the invention encompasses the administration of compounds found to increase the endogenous activity of polypeptides having an ADAM-H9 amino acid sequence to cells and/or organisms. One example of compounds that increase ADAM-H9 polypeptide activity are antibodies that bind to ADAM-H9 polypeptides, preferably monoclonal antibodies, and increase or stimulate ADAM-H9 polypeptide activity by causing constitutive intracellular signaling (or "ligand mimicking"), or by preventing the binding of a native inhibitor of ADAM-H9 polypeptide activity.

Due to the multiplicity and interconnectedness of biological pathways and interactions, an ADAM-H9 polypeptide, fragment, variant, antagonist, agonist, antibody, and binding partner of the invention can be useful for treating medical conditions and diseases associated with cell-cell and cell matrix interactions (*e.g.*, integrin-mediated disorders), endothelial migration, angiogenesis, inflammation, cancer, allergy, reproductive, neurological and vascular conditions as described further herein. The therapeutic molecule or molecules to be used will depend on the etiology of the condition to be treated and the biological pathways involved, and will consider that different variants, antagonists, and binding partners of ADAM-H9 polypeptides may have similar or different effects. For example, an ADAM-H9 polypeptide or fragment thereof may act as an antagonist of a protein processing function of metalloproteinases (*e.g.*, from other members of the ADAM family of polypeptides) by interacting with an ADAM binding partner and preventing the activity of the metalloproteinase upon its substrate. Accordingly, an ADAM-H9 may modulate protein processing, such as release of growth factors, adhesion proteins, and inflammatory factors.

The disclosed ADAM-H9 polypeptides, fragments thereof, antibodies, compositions and combination therapies described herein are useful in medicines for treating bacterial, viral or protozoal infections, and complications resulting therefrom. Cardiovascular disorders are treatable with the disclosed ADAM-H9 polypeptides, fragments thereof, antibodies, pharmaceutical compositions or combination therapies, including aortic aneurysms; arteritis; vascular occlusion; complications of coronary by-pass surgery; ischemia/reperfusion injury; heart disease; heart failure; and myocardial infarction. In addition, the ADAM-H9 polypeptides, fragments thereof, antibodies, compositions and combination therapies of the invention can be used to treat chronic pain conditions, to treat various disorders of the endocrine system, conditions of the gastrointestinal system, disorders of the genitourinary system, and anemias and hematological disorders.

Due to the role of integrins (*e.g.*, αᵥβ3, αᵥβ₅, β₁, β₄, α₁, α₂, α₃, α₄, α₅, and α₆ integrins) in cell proliferative disorders, including cancer and cancer cell metastasis, also provided herein are methods for using ADAM-H9 polypeptides, fragments thereof (particularly comprising an ADAM-H9dis domain, *e.g.*, ADAM-H9dis-Fc oligomers), antibodies, compositions or combination therapies to treat various hematologic and oncologic disorders. For example, soluble ADAM-H9 disintegrin domains can be used to treat various forms of cancer, including acute myelogenous leukemia, Epstein-Barr virus-positive nasopharyngeal carcinoma, glioma, colon, stomach, prostate, renal cell, cervical and ovarian cancers, lung cancer (SCLC and NSCLC), including cancer-associated cachexia, fatigue, asthenia, paraneoplastic syndrome of cachexia, and hypercalcemia by modulating integrin-associated interactions.

Additional diseases treatable with the polypeptides, fragments, antibodies, compositions or combination therapies of the invention are solid tumors, including sarcoma, osteosarcoma, and carcinoma, such as adenocarcinoma (*e.g.*, breast cancer) and squamous cell carcinoma. Administration of a soluble ADAM-H9 disintegrin domain can modulate cell-cell and cell-matrix interactions of such tumor cells and/or modulate the angiogenesis and blood supply to such tumors.

In addition, the ADAM-H9 polypeptides, fragments thereof, compositions or combination therapies are useful for treating leukemia, including acute myelogenous leukemia, chronic or acute lymphoblastic leukemia and hairy cell leukemia. Other malignancies with invasive metastatic potential that can be treated with the ADAM-H9 polypeptides, fragments, antibodies, compositions and combination therapies, include multiple myeloma, various lymphoproliferative disorders such as autoimmune lymphoproliferative syndrome (ALPS), chronic lymphoblastic leukemia, hairy cell leukemia, chronic lymphatic leukemia, peripheral T-cell lymphoma, small lymphocytic lymphoma, mantle cell lymphoma, follicular lymphoma, Burkitt's lymphoma, Epstein-Barr virus-positive T cell lymphoma, histiocytic lymphoma, Hodgkin's disease, diffuse aggressive lymphoma, acute lymphatic leukemias, T gamma lymphoproliferative disease, cutaneous B cell lymphoma, cutaneous T cell lymphoma (*i.e.,* mycosis fungoides), and Sézary syndrome.

A combination of at least one ADAM-H9 polypeptide, fragment thereof, or antibody, and one or more additional anti-angiogenesis factors or other therapeutic agent(s) may be administered to the subject. The additional therapeutic agent(s) may be administered prior to, concurrently with, or following the administration of the ADAM-H9 polypeptide, fragments thereof (particularly comprising an ADAM-H9dis domain, *e*.*g*., ADAM-H9dis-Fc oligomers), or antibody. The use of more than one therapeutic agent is particularly advantageous when the subject that is being treated has a solid tumor. In some embodiments of the invention, the treatment further comprises treating the mammal with radiation. Radiation, including brachytherapy and teletherapy, may be administered prior to, concurrently with, or following the administration of the ADAM-H9 polypeptide, fragment, antibody, or ADAM-H9 binding partner and/or additional therapeutic agent(s).

The method includes the administration of, in addition to a ADAM-H9 polypeptide, fragments thereof (particularly comprising an ADAM-H9dis domain, *e.g.,* ADAM-H9dis-Fc oligomers), or antibody, one or more therapeutics selected from the group consisting of alkylating agents, antimetabolites, vinca alkaloids and other plant-derived chemotherapeutics, antitumor antibiotics, antitumor enzymes, topoisomerase inhibitors, platinum analogs, adrenocortical suppressants, hormones and antihormones, antibodies, immunotherapeutics, radiotherapeutics, and biological response modifiers.

The method further includes administration of, in addition to an ADAM-H9 polypeptide, fragments thereof (particularly comprising an ADAM-H9dis domain, *e.g.,* ADAM-H9dis-Fc oligomers), or antibody, one or more therapeutics selected from the group consisting of cisplatin, cyclophosphamide, mechloretamine, melphalan, bleomycin, carboplatin, fluorouracil, 5-fluorodeoxyuridine, methotrexate, taxol, asparaginase, vincristine, and vinblastine, lymphokines and cytokines such as interleukins, interferons (α, β or δ) and TNF, chlorambucil, busulfan, carmustine, lomustine, semustine, streptozocin, dacarbazine, cytarabine, mercaptopurine, thioguanine, vindesine, etoposide, teniposide, dactinomycin, daunorubicin, doxorubicin, bleomycin, plicamycin, mitomycin, L-asparaginase, hydroxyurea, methylhydrazine, mitotane, tamoxifen, fluoxymesterone, IL-8 inhibitors, angiostatin, endostatin, kringle 5, angiopoietin-2 or other antagonists of angiopoietin-1, antagonists of platelet-activating factor, antagonists of basic fibroblast growth factor, and COX-2 inhibitors.

The method also includes administration of, in addition to an ADAM-H9 polypeptide, fragments thereof (particularly comprising an ADAM-H9dis domain, *e.g.*, ADAM-H9dis-Fc oligomers), or antibody, one or more therapeutic polypeptides, including soluble forms thereof, selected from the group consisting of Flt3 ligand (see, U.S. Patent No. 5,554,512), CD40 ligand (see, U.S. Patent No. 5,716,805), IL-2, IL-12, 4-1BB ligand (see, U.S. Patent No. 5,674,704), anti-4-1BB antibodies, TRAIL (see, U.S. Patent No. 5,763,223), TNF antagonists and TNF receptor (TNFR) antagonists including TNFR/Fc, Tek antagonists (see, PCT Publication No. WO 00/75323, 14 December 2000), TWEAK antagonists and TWEAK-R (see, U.S. Serial Numbers 60/172,878 and 60/203,347 and Feng *et al.,* Am. J. Pathol. 156(4):1253) antagonists including TWEAK-R/Fc, VEGF antagonists including anti-VEGF antibodies, VEGF receptor (including VEGF-R1 and VEGF-R2, also known as Flt1 and Flkl or KDR) antagonists, CD148 (also referred to as DEP-1, ECRTP, and PTPRJ, see Takahashi *et al.,* J. Am. Soc. Nephrol. 10:2135-45, 1999; and PCT Publication No. WO 00/15258, 23 March 2000) binding proteins, and nectin-3 (see, Satoh-Horikawa *et al.,* J. Biol. Chem. 275(14):10291, 2000; GenBank accession numbers of human nectin-3 nucleic acid and polypeptide sequences are AF282874 and AAF97597, respectively) antagonists.

In some preferred embodiments an ADAM-H9 polypeptide, fragments thereof (particularly comprising an ADAM-H9dis domain, *e.g.*, ADAM-H9dis-Fc oligomers), or antibody of the invention is used as a component of, or in combination with, "metronomic therapy," such as that described by Browder *et al.* and Klement *et al.* (Cancer Research 60:1478, 2000; J. Clin. Invest. 105(8):R15, 2000; see also Barinaga, Science 288:245, 2000).

Described and/or claimed herein are compounds, compositions, and methods for treating a subject, preferably a mammalian subject, and most preferably a human subject, who is suffering from a medical disorder, and in particular an ADAM-H9-associated disorder. Such ADAM-H9-associated disorders include conditions caused (directly or indirectly) or exacerbated by binding between a polypeptide having an ADAM-H9 sequence (*e.g*., SEQ ID Nos:1, 3, 4, 6, 8, or 10) and its binding partner (*e.g.*, an integrin). For purposes of this disclosure, the terms ''illness,'' "disease," "disorder," "medical condition," "abnormal condition" and the like are used interchangeably with the term "medical disorder." The terms "treat", "treating", and "treatment" used herein include curative, preventative (*e.g*., prophylactic) and palliative or ameliorative treatment. For such therapeutic uses, ADAM-H9 polypeptides and fragments, ADAM-H9 polynucleotides encoding an ADAM-H9 polypeptide, and/or agonists or antagonists of the ADAM-H9 polypeptide such as antibodies can be administered to the subject in need through known means. Compositions of the invention can contain a polypeptide in any form described herein, such as native polypeptides, variants, derivatives, oligomers, and biologically active fragments. In particular embodiments, the composition comprises a soluble polypeptide or an oligomer comprising soluble ADAM-H9 polypeptides (*e.g.,* a soluble ADAM-H9 disintegrin domain).

In practicing the method of treatment or use of the invention, a therapeutically effective amount of a therapeutic agent of the invention is administered to a subject having a condition to be treated, preferably to treat or ameliorate diseases associated with the activity of an ADAM-H9 polypeptide. "Therapeutic agent" includes without limitation any ADAM-H9 polypeptide, fragment, and variant; polynucleotide encoding an ADAM-H9 polypeptide, fragment, and variant; agonists or antagonists of the an ADAM-H9 polypeptide such as antibodies; an ADAM-H9 polypeptide binding partner; complexes formed from an ADAM-H9 polypeptide, fragment, variant, and binding partner, and the like. As used herein, the term "therapeutically effective amount" means the total amount of each therapeutic agent or other active component of the pharmaceutical composition or method that is sufficient to show a meaningful subject benefit, *e.g.*, treatment, healing, prevention or amelioration of the relevant medical condition, or an increase in rate of treatment, healing, prevention or amelioration of such conditions. When applied to an individual therapeutic agent or active ingredient, administered alone, the term refers to that ingredient alone. When applied to a combination, the term refers to combined amounts of the ingredients that result in the therapeutic effect, whether administered in combination, serially or simultaneously. As used herein, the phrase "administering a therapeutically effective amount" of a therapeutic agent means that the subject is treated with said therapeutic agent in an amount and for a time sufficient to induce an improvement, and preferably a sustained improvement, in at least one indicator that reflects the severity of the disorder. An improvement is considered "sustained" if the subject exhibits the improvement on at least two occasions separated by one or more weeks. The degree of improvement is determined based on signs or symptoms, and determinations may also employ questionnaires that are administered to the subject, such as quality-of-life questionnaires. Various indicators that reflect the extent of the subject's illness may be assessed for determining whether the amount and time of the treatment is sufficient. The baseline value for the chosen indicator or indicators is established by examination of the subject prior to administration of the first dose of the therapeutic agent. Preferably, the baseline examination is done within about 60 days of administering the first dose. If the therapeutic agent is being administered to treat acute symptoms, the first dose is administered as soon as practically possible after the injury has occurred. Improvement is induced by administering therapeutic agents such as an ADAM-H9 polypeptide, fragment, antibody, or ADAM-H9 binding partner until the subject manifests an improvement over baseline for the chosen indicator or indicators. In treating chronic conditions, this degree of improvement is obtained by repeatedly administering this medicament over a period of at least a month or more, *e.g.,* for one, two, or three months or longer, or indefinitely. A period of one to six weeks, or even a single dose, often is sufficient for treating acute conditions. Although the extent of the subject's illness after treatment may appear improved according to one or more indicators, treatment may be continued indefinitely at the same level or at a reduced dose or frequency. Once treatment has been reduced or discontinued, it later may be resumed at the original level if symptoms should reappear.

One skilled in the art will recognize that suitable dosages will vary, depending upon such factors as the nature and severity of the disorder to be treated, the subject's body weight, age, general condition, and prior illnesses and/or treatments, and the route of administration. Preliminary doses can be determined according to animal tests, and the scaling of dosages for human administration is performed according to art-accepted practices such as standard dosing trials. For example, the therapeutically effective dose can be estimated initially from cell culture assays. The dosage will depend on the specific activity of the compound and can be readily determined by routine experimentation. A dose may be formulated in animal models to achieve a circulating plasma concentration range that includes the IC₅₀ (*i.e.,* the concentration of the test compound which achieves a half-maximal inhibition of symptoms) as determined in cell culture, while minimizing tonicities. Such information can be used to more accurately determine useful doses in humans. Ultimately, the attending physician will decide the amount of polypeptide of the invention with which to treat each individual subject. Initially, the attending physician will administer low doses of polypeptide of the invention and observe the subject's response. Larger doses of polypeptide of the invention may be administered until the optimal therapeutic effect is obtained for the subject, and at that point the dosage is not increased further. It is contemplated that the various pharmaceutical compositions used to practice the method of the invention should contain about 0.01 ng to about 100 mg (preferably about 0.1 ng to about 10 mg, more preferably about 0.1 microgram to about 1 mg) of a polypeptide of the invention per kg body weight. In one embodiment of the invention, an ADAM-H9 polypeptide, fragment, antibody, or ADAM-H9 binding partner is administered one time per week to treat the various medical disorders disclosed herein. In another embodiment polypeptide, fragment, antibody, or ADAM-H9 binding partner is administered at least two times per week and in another embodiment at least three times per week. If injected, the effective amount of an ADAM-H9 polypeptide, fragment, antibody, or ADAM-H9 binding partner per adult dose ranges from 1-20 mg/m², and preferably is about 5-12 mg/m². Alternatively, a flat dose may be administered whose amount may range from 5-100 mg/dose. Exemplary dose ranges for a flat dose to be administered by subcutaneous injection are 5-25 mg/dose, 25-50 mg/dose and 50-100 mg/dose. In one embodiment of the invention, the various indications described herein are treated by administering a preparation acceptable for injection containing an ADAM-H9 polypeptide, fragment, antibody, or ADAM-H9 binding partner at 25 mg/dose, or alternatively, containing 50 mg per dose. The 25 mg or 50 mg dose may be administered repeatedly, particularly for chronic conditions. If a route of administration other than injection is used, the dose is appropriately adjusted in accord with standard medical practices. In many instances, an improvement in a subject's condition will be obtained by injecting a dose of about 25 mg of an ADAM-H9 polypeptide, fragment, antibody, or ADAM-H9 binding partner one to three times per week over a period of at least three weeks, or a dose of 50 mg of an ADAM-H9 polypeptide, fragment, antibody, or ADAM-H9 binding partner one or two times per week for at least three weeks (a treatment for longer periods may be necessary to induce the desired degree of improvement). For incurable chronic conditions, the regimen may be continued indefinitely, with adjustments being made to dose and frequency if such are deemed necessary by the subject's physician. The foregoing doses are examples for an adult subject who is a person who is 18 years of age or older. For pediatric subjects (age 4-17), a suitable regimen involves the subcutaneous injection of 0.4 mg/kg, up to a maximum dose of 25 mg of an ADAM-H9 polypeptide, fragment, antibody, or ADAM-H9 binding partner, administered by subcutaneous injection one or more times per week. If an antibody against an ADAM-H9 polypeptide is used as an ADAM-H9 polypeptide antagonist, a preferred dose range is 0.1 to 20 mg/kg, and more preferably is 1-10 mg/kg. Another preferred dose range for an anti-ADAM-H9 polypeptide antibody is 0.75 to 7.5 mg/kg of body weight. Humanized antibodies are preferred. Such antibodies may be injected or administered intravenously.

Compositions comprising an effective amount of an ADAM-H9 polypeptide of the invention (from whatever source derived, including without limitation from recombinant and non-recombinant sources), in combination with other components such as a physiologically acceptable diluent, carrier, or excipient, are provided herein. The term "pharmaceutically acceptable" means a non-toxic material that does not interfere with the effectiveness of the biological activity of the active ingredient(s). Formulations suitable for administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The polypeptides can be formulated according to known methods used to prepare pharmaceutically useful compositions. They can be combined in admixture, either as the sole active material or with other known active materials suitable for a given indication, with pharmaceutically acceptable diluents (*e.g.*, saline, Tris-HCl, acetate, and phosphate buffered solutions), preservatives (*e.g.*, thimerosal, benzyl alcohol, parabens), emulsifiers, solubilizers, adjuvants and/or carriers. Suitable formulations for pharmaceutical compositions include those described in *Remington's Pharmaceutical Sciences,* 16th ed. 1980, Mack Publishing Company, Easton, PA. In some embodiments the polypeptide may undergo pegylation to assist in adsorption or uptake. For example, such compositions can be complexed with polyethylene glycol (PEG), metal ions, or incorporated into polymeric compounds such as polyacetic acid, polyglycolic acid, hydrogels, dextran, and the like, or incorporated into liposomes, microemulsions, micelles, unilamellar or multilamellar vesicles, erythrocyte ghosts or spheroblasts. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Pat. No. 4,235,871; U.S. Pat. No. 4,501,728; U.S. Pat. No. 4,837,028; and U.S. Pat. No. 4,737,323, all of which are incorporated herein by reference. Such compositions will influence the physical state, solubility, stability, rate of *in vivo* release, and rate of *in vivo* clearance, and are thus chosen according to the intended application, so that the characteristics of the carrier will depend on the selected route of administration. In one preferred embodiment of the invention, sustained-release forms of an ADAM-H9 polypeptide are used. Sustained-release forms suitable for use in the disclosed methods include, but are not limited to, an ADAM-H9 polypeptide that is encapsulated in a slowly-dissolving biocompatible polymer (such as the alginate microparticles described in U.S. Pat. No. 6,036,978), admixed with such a polymer (including topically applied hydrogels), and or encased in a biocompatible semi-permeable implant.

An ADAM-H9 polypeptide of the invention may be active in multimers (*e.g.*, heterodimers or homodimers) or complexes with itself or other polypeptides. As a result, pharmaceutical compositions of the invention may comprise a polypeptide of the invention in such multimeric or complexed form. The pharmaceutical composition of the invention may be in the form of a complex of the polypeptide(s) of invention. The invention further includes the administration of an ADAM-H9 polypeptide, fragment, antibody, or ADAM-H9 binding partner concurrently with one or more other drugs that are administered to the same subject in combination, each drug being administered according to a regimen suitable for that medicament. "Concurrent administration" encompasses simultaneous or sequential treatment with the components of the combination, as well as regimens in which the drugs are alternated, or wherein one component is administered long-term and the other(s) are administered intermittently. Components may be administered in the same or in separate compositions, and by the same or different routes of administration. Examples of components that may be included in the pharmaceutical composition of the invention are cytokines, lymphokines, or other hematopoietic factors such as: M-CSF, GM-CSF, TNF, IL-1, IL-2, IL-3, IL4, IL-5, IL-6, IL-7, IL-8, IL-9, IL-10, IL,-11, IL-12, IL-13, IL-14, IL-15, IL-17, IL-18, IFN, TNF0, TNF1, TNF2, G-CSF, Meg-CSF, thrombopoietin, stem cell factor, and erythropoietin. The pharmaceutical composition may further contain other agents that either enhance the activity of the polypeptide or compliment its activity or use in treatment. Such additional factors and/or agents may be included in the pharmaceutical composition to produce a synergistic effect with a polypeptide of the invention, or to minimize side effects. Conversely, an ADAM-H9 polypeptide, fragment, antibody, or ADAM-H9 binding partner of the invention may be included in formulations with a particular cytokine, lymphokine, other hematopoietic factor, thrombolytic or anti-thrombotic factor, or anti-inflammatory agent to minimize side effects of the cytokine, lymphokine, other hematopoietic factor, thrombolytic or anti-thrombotic factor, or anti-inflammatory agent. Additional examples of drugs to be administered concurrently include but are not limited to antivirals, antibiotics, analgesics, corticosteroids, antagonists of inflammatory cytokines, non-steroidal antiinflammatories, pentoxifylline, thalidomide, and disease-modifying antirheumatic drugs (DMARDs) such as azathioprine, cyclophosphamide, cyclosporine, hydroxychloroquine sulfate, methotrexate, leflunomide, minocycline, penicillamine, sulfasalazine and gold compounds such as oral gold, gold sodium thiomalate, and aurothioglucose. Additionally, an ADAM-H9 polypeptide, fragment, antibody, or ADAM-H9 binding partner may be combined with a second ADAM-H9 polypeptide, antibody against an ADAM-H9 polypeptide, or an ADAM-H9 polypeptide-derived peptide that acts as a competitive inhibitor of a native an ADAM-H9 polypeptide.

Any efficacious route of administration may be used to therapeutically administer an ADAM-H9 polypeptide, fragment, antibody, or ADAM-H9 binding partner thereof, including those compositions comprising ADAM-H9 polynucleotides. Parenteral administration includes injection, for example, via intra-articular, intravenous, intramuscular, intralesional, intraperitoneal or subcutaneous routes by bolus injection or by continuous infusion. Other routes include localized administration, *e.g.*, at a site of disease or injury. Other suitable means of administration include sustained release from implants; aerosol inhalation and/or insufflation; eyedrops; vaginal or rectal suppositories; buccal preparations; oral preparations, including pills, syrups, lozenges or chewing gum; and topical preparations such as lotions, gels, sprays, ointments or other suitable techniques. Alternatively, ADAM-H9 polypeptide, fragment, antibody, or ADAM-H9 binding partner may be delivered by implanting cells that express the polypeptide, for example, by implanting cells that express an ADAM-H9 polypeptide, fragment, antibody, or ADAM-H9 binding partner. Cells may also be cultured *ex vivo* in the presence of polypeptides of the invention in order to proliferate or to produce a desired effect on or activity in such cells. Treated cells can then be introduced *in vivo* for therapeutic purposes. In another embodiment, the subject's own cells are induced to produce an ADAM-H9 polypeptide, fragment, antibody, or ADAM-H9 binding partner by transfection *in vivo* or *ex vivo* with a polynucleotide that encodes an ADAM-H9 polypeptide, fragment, antibody, or ADAM-H9 binding partner. The polynucleotide can be introduced into the subject's cells, for example, by injecting naked DNA or liposome-encapsulated DNA that encodes an ADAM-H9 polypeptide, fragment, antibody, or ADAM-H9 binding partner, or by other means of transfection. Polynucleotides of the invention may also be administered to subjects by other known methods for introduction of nucleic acids into a cell or organism (including, without limitation, in the form of viral vectors).

When a therapeutically effective amount of an ADAM-H9 polypeptide, fragment thereof, antibody, or binding partner of the invention is administered orally, the polypeptide will typically be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% a polypeptide of the invention, and preferably from about 25 to 90% a polypeptide of the invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of a polypeptide of the invention, and preferably from about 1 to 50% a polypeptide of the invention.

When a therapeutically effective amount of an ADAM-H9 polypeptide, fragment, antibody, or binding agent of the invention is administered by intravenous, cutaneous or subcutaneous injection, the polypeptide will be in the form of a pyrogen-free, parenterally acceptable aqueous solution. The preparation of such parenterally acceptable polypeptide solutions, having due regard to pH, isotonicity, stability, and the like, is within the skill in the art. A preferred pharmaceutical composition for intravenous, cutaneous, or subcutaneous injection should contain, in addition to a polypeptide of the invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the invention may also contain stabilizers, preservatives, buffers, antioxidants, or other additives known to those of skill in the art. The duration of intravenous therapy using the pharmaceutical composition of the invention will vary, depending on the severity of the disease being treated and the condition and potential idiosyncratic response of each individual subject. It is contemplated that the duration of each application of a polypeptide of the invention will be in the range of 12 to 24 hours of continuous intravenous administration. Ultimately the attending physician will decide on the appropriate duration of intravenous therapy.

For compositions of the invention which are useful for tissue repair or regeneration, the therapeutic method includes administering a pyrogen-free, physiologically acceptable form of the composition topically, systematically, locally or in association with an implant or device. Further, the composition may desirably be encapsulated or injected in a viscous form for delivery to the site tissue damage. Additional useful agents may also optionally be included in the composition, as described above, or may be administered simultaneously or sequentially with the composition in the methods of the invention. The compositions can include a matrix capable of delivering the polypeptide-containing composition to the site tissue damage, providing a structure for the developing tissue and optimally capable of being resorbed into the body. The choice of matrix material is based on biocompatibility, biodegradability, mechanical properties, cosmetic appearance and interface properties. Potential matrices for the compositions include calcium sulfate, tricalciumphosphate, hydroxyapatite, polylactic acid, polyglycolic acid and polyanhydrides. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. Progress can be monitored by periodic assessment of tissue/bone growth and/or repair, for example, X-rays, histomorphometric determinations and tetracycline labeling.

In addition to human subjects, compositions comprising an ADAM-H9 polypeptide, fragment, antibody, or ADAM-H9 binding partner is useful in the treatment of disease conditions in non-human animals, such as pets (dogs, cats, birds, primates, and the like), domestic farm animals (horses cattle, sheep, pigs, birds, and the like). In such instances, an appropriate dose may be determined according to the animal's body weight. For example, a dose of 0.2-1 mg/kg may be used. Alternatively, the dose is determined according to the animal's surface area, an exemplary dose ranging from 0.1-20 mg/m², or more preferably, from 5-12 mg/m². For small animals, such as dogs or cats, a suitable dose is 0.4 mg/kg. In a one embodiment, an ADAM-H9 polypeptide, fragment, antibody, or ADAM-H9 binding partner (preferably constructed from genes derived from the same species as the subject), is administered by injection or other suitable route one or more times per week until the animal's condition is improved, or it may be administered indefinitely.

The invention also relates to the use an ADAM-H9 polypeptide, fragment, and variant; polynucleotide encoding an ADAM-H9 polypeptide, fragment, and variant; agonists or antagonists of an ADAM-H9 polypeptide such as antibodies; an ADAM-H9 polypeptide binding partner; complexes formed from an ADAM-H9 polypeptide, fragment, variant, and binding partner, and the like, in the manufacture of a medicament for the prevention or therapeutic treatment of a disease or disorder.

Further encompassed by the invention are systems and methods for analyzing ADAM-H9 polypeptides comprising identifying and/or characterizing one or more ADAM-H9 polypeptides, encoding nucleic acids, and corresponding genes, these systems and methods preferably comprising a data set representing a set of one or more ADAM-H9 molecules, or the use thereof. Accordingly, described herein is a computer readable medium having stored thereon a member selected from the group consisting of a polynucleotide comprising a sequence as set forth in SEQ ID Nos:2, 5, 7, or 9; a polypeptide comprising a sequence as set forth in SEQ ID Nos:1, 3, 4, 6, 8, or 10; a set of polynucleotide sequences wherein at least one of said sequences comprises a sequence as set forth in SEQ ID Nos:2, 5, 7, or 9; and a set of polypeptide sequences wherein at least one of said sequences comprises a sequence as set forth in SEQ ID Nos:1, 3, 4, 6, 8, or 10.

Described herein is furthermore a computing environment and a plurality of algorithms selectively executed to analyze a polypeptide or polynucleotide of the invention. Examples of analyses of an ADAM polypeptide include, without limitation, displaying the amino acid sequence of a polypeptide in the set, comparing the amino acid sequence of one polypeptide in the set to the amino acid sequence of another polypeptide in the set, predicting the structure of a polypeptide in the set, determining the nucleotide sequences of nucleic acids encoding a polypeptide in the set, and identifying a gene corresponding to a polypeptide in the set.

Unless otherwise defined, all technical and scientific terms used herein have the same meaning as commonly understood by one of ordinary skill in the art to which this invention belongs. All headings and subheading provided herein are solely for ease of reading and should not be construed to limit the invention. The terms "a", "an" and "the" as used herein are meant to encompass the plural unless the context clearly dictates the singular form. Although methods and materials similar or equivalent to those described herein can be used in the practice or testing of the invention, suitable methods and materials are described below. In case of conflict, the present specification, including definitions, will control. In addition, the materials, methods, and examples are illustrative only and not intended to be limiting. The following examples are intended to illustrate particular embodiments and not to limit the scope of the invention.

### EXAMPLE 1

### Identification of ADAM-H9, a New Member of the ADAM Family of Polypeptides

A data set was received from Celera Genomics (Rockville, Maryland) containing amino acid sequences predicted to be encoded by the human genome. This data set was searched using a BLAST algorithm to identify ADAM family polypeptides. An amino acid sequence as set forth in SEQ ID NO:1 was identified as comprising partial amino acid sequences of a new human ADAM family polypeptide. This amino acid sequence was used to identify a first exon of about 194 bp. This first exon was used to identify a clone containing a continguous polynucleotide containing the first exon sequence. A second exon was identified by analyzing the contiguous sequence upstream of the first exon until substantial homology was found to the coding sequence for ADAM9 (SEQ ID NO:23). This region of substantial homology was identified as a possible second exon and was subsequently PCR amplified from a cDNA library of lymph node cells. The PCR product (SEQ ID NO:2) encoded a partial sequence of an ADAM-H9 polypeptide having the amino acid sequence shown in SEQ ID NO:3 and SEQ ID NO:4 (from residue 17 to 67). The first 14 amino acids of SEQ ID NO:3 represent the translation of a second exon of about 42 bp (approximately 1.3kb upstream of the first identified exon) and both exons were confirmed by PCR amplification and analysis of cDNA from a variety of human tissues including placenta, liver, kidney, pancreas, spleen, testis, stomach, bone marrow, lymph node, heart, skeletal muscle, brain, lung, colon, prostate, thymus, ovary, small intestine, skin, and esophagus. cDNA from some related tissue of fetal origin were also analyzed. The amino acid sequences presented in Figure 2 are presented in standard 1-letter amino acid code, where "A" represents alanine, "C" represents cysteine, and the like.

### EXAMPLE 2

### RACE Analysis

Additional polynucleotides encoding an ADAM-H9 polypeptide were identified by rapid amplification of cDNA ends (RACE) analysis. All RACE products were cloned into vectors and sequenced. Sequence analysis of the RACE products identified a number of clones having substantially identical sequences. RACE Analysis kits are available from a number of companies including Roche Molecular Systems. Primers were designed based upon consensus sequences found by RACE product comparison.

A primer pair comprising nucleotides 25-49 and 1434-1464 of SEQ ID NO:5 was used to PCR amplify a cDNA library from lymph node cells and bone marrow cells. The resulting PCR products were cloned and sequenced using standard protocols. The polynucleotides contained in these clones are presented in SEQ ID Nos:5, 7, and 9, and encode the polypeptides having a sequence as set forth in SEQ ID Nos:6, 8, and 10, respectively. Two of the three cloned sequences include predicted transmembrane anchors and cytoplasmic domains.

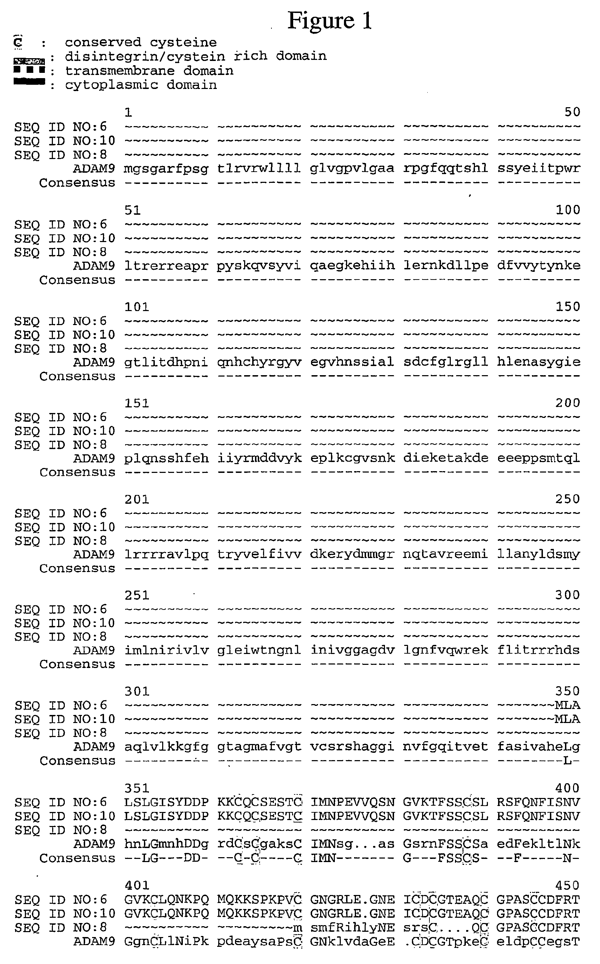

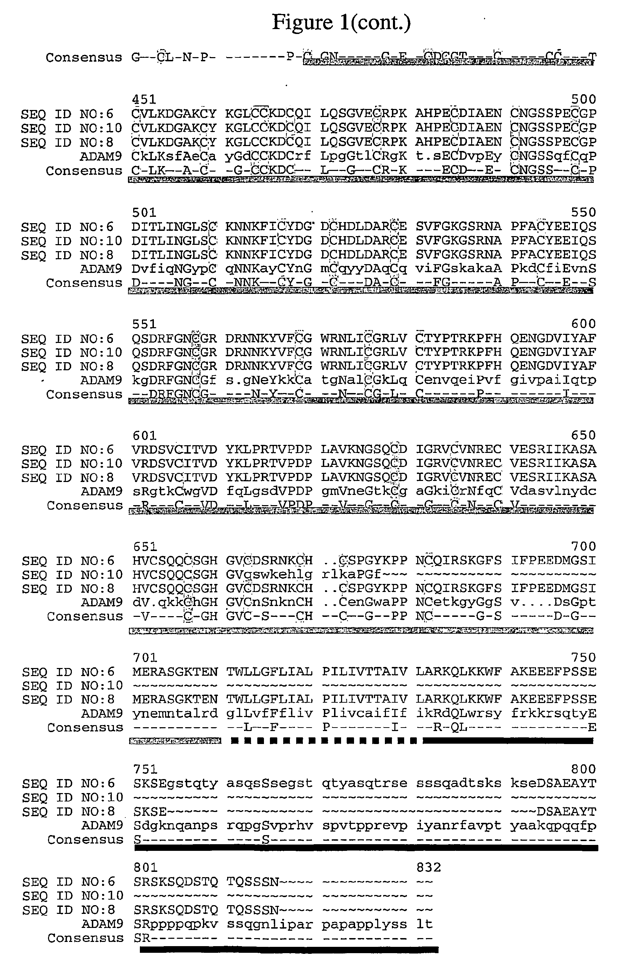

An analysis of the ADAM-H9 sequence demonstrates that SEQ ID Nos: 1, 3, 4, 6, 8, and 10 all contain a region of amino acids having homology to the disintegrin domain of the ADAM family of polypeptides. A disintegrin domain of the invention may include a sequence beginning with a highly conserved CGN sequence beginning at residue 73 and continuing to about residue 360 to 362 of SEQ ID NO:6; may include a sequence from about residue 1 or 16 to about residue 285 to 287 of SEQ ID NO:8; or may include a sequence from residue 1 or 73 (or any residue therebetween) to about 314 or 329 (or any residue therebetween) of SEQ ID NO:10. The analysis also identified a transmembrane sequence present in SEQ ID Nos:6 and 8 beginning at about residue 361 and continuing to residue 382 of SEQ ID NO:6 or from about residue 286 to residue 307 of SEQ ID NO:8. Accordingly, a polypeptide lacking a transmembrane domain, (*e.g.*, fragments of SEQ ID Nos:6 and 8 having the transmembrane domain missing or deleted) are predicted to be soluble polypeptides having disintegrin activity. Also identified by the invention is a naturally occurring variant of an ADAM-H9 polypeptide which lacks a transmembrane domain (see, *e.g.*, SEQ ID NO:10). In addition, a comparison of the ADAM-H9 polypeptide sequences of SEQ ID Nos:6, 8, and 10 with that of the human ADAM9 sequence demonstrates a number of conserved cysteine residues in the disintegrin and cysteine rich domains consistent with ADAM family polypeptides. An alignment of the ADAM9 sequence with SEQ ID Nos:6, 8, and 10 is presented in Figure 1. In Figure 1 the gray bars represent an approximation of the disintegrin and cysteine rich domain and the dashed line the approximate transmembrane domain. Conserved cysteine residues are highlighted and capitalized.

The polynucleotide sequences encoding a portion or all of the polypeptide sequences of ADAM-H9 (SEQ ID Nos: 1, 3, 4, 6, 8, or 10) are provided in Figure 3. The bolded ATG in SEQ ID Nos:5, 7, and 9 above represent the start codon or methionine at position 1 of SEQ ID Nos:6, 8, and 10. An analysis of the coding sequence of SEQ ID Nos:6, 8, and 10, as depicted in SEQ ID Nos: 5, 7, and 9, respectively, demonstrate that the domain having disintegrin activity corresponds to about nucleotide 248 to about nucleotide 1111 of SEQ ID NO:5; about nucleotide 82 or 127 (or any nucleotide therebetween) to about nucleotide 936 of SEQ ID NO:7; or about nucleotide 32 or 248 (or any nucleotide therebetween) to about nucleotide 973 or 1018 (or any nucleotide therebetween) of SEQ ID NO:9. Accordingly, a polynucleotide comprising fragments of the nucleic acids sequences above represent a coding sequence for a soluble polypeptide having disintegrin activity. Furthermore, the coding sequence for the transmembrane domain of SEQ ID Nos:6 and 8 correspond to about nucleotides 1112 to about 1177 or about nucleotides 937 to about 1002 of SEQ ID Nos:5 and 7, respectively. As discussed herein the cytoplasmic domains of SEQ ID Nos:6, 8, and 10 differ and potentially represent splice variants.

Variants of the ADAM-H9 polypeptide sequences can be identified based upon the sequences provided herein. A number of variants are provided herein (as described more fully below) and are included within the scope of the invention. For example, SEQ ID NO:1 is present in SEQ ID Nos:3, 4, 6, 8, and 10, however, SEQ ID NO:8 lacks a stretch of 39 amino acids in the predicted cytoplasmic domain of the polypeptide compared to SEQ ID NO:6. Amino acid substitutions and other alterations (deletions, insertions, and the like) to ADAM-H9 amino acid sequences are predicted to be more likely to alter or disrupt ADAM-H9 polypeptide activities if they result in changes to the conserved residues of the amino acid sequences as shown in Figure 1, and particularly if those changes do not substitute an amino acid of similar structure (such as substitution of any one of the aliphatic residues - Ala, Gly; Leu, Ile, or Val - for another aliphatic residue). Conversely, if a change is made to an ADAM-H9 amino acid sequence resulting in substitution of the residue at that position in the alignment from one of the other ADAM-H9 polypeptide sequences, it is less likely that such an alteration will affect the function of the altered ADAM-H9 polypeptide.

### EXAMPLE 3

### Monoclonal Antibodies That Bind Polypeptides of the Invention

A substantially purified ADAM-H9 polypeptide can be used to generate monoclonal antibodies immunoreactive therewith, using conventional techniques such as those described in U.S. Patent 4,411,993. Mice are immunized with an ADAM-H9 polypeptide immunogen emulsified in complete Freund's adjuvant, and injected in amounts ranging from 10-100 µg subcutaneously or intraperitoneally. Ten to twelve days later, the immunized animals are boosted with additional ADAM-H9 polypeptide emulsified in incomplete Freund's adjuvant. Mice are periodically boosted thereafter on a weekly to bi-weekly immunization schedule. Serum samples are periodically taken by retro-orbital bleeding or tail-tip excision to test for an ADAM-H9 polypeptide antibody by dot blot assay, ELISA (Enzyme-Linked Immunosorbent Assay) or inhibition of binding of an ADAM-H9 polypeptide to an ADAM-H9 polypeptide binding partner.

Following detection of an appropriate antibody titer, positive animals are provided one last intravenous injection of an ADAM-H9 polypeptide in saline. Three to four days later, the animals are sacrificed, spleen cells harvested, and spleen cells are fused to a murine myeloma cell line, *e.g.,* NS1 or preferably P3x63Ag8.653 (ATCC CRL 1580). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against a substantially pure ADAM-H9 polypeptide by adaptations of the techniques disclosed in Engvall *et al.,* (*Immunochem.* 8:871, 1971) and in U.S. Patent 4,703,004. A preferred screening technique is the antibody capture technique described in Beckmann *et al.,* (*J. Immunol.* 144:4212, 1990). Positive hybridoma cells can be injected intraperitoneally into syngeneic BALB/c mice to produce ascites containing high concentrations of anti-ADAM-H9 monoclonal antibody. Alternatively, hybridoma cells can be grown *in vitro* in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to Polypeptide A or Polypeptide G can also be used, as can chromatography based upon binding to ADAM-H9 polypeptide.

### EXAMPLE 4

### Chromosome mapping

The gene corresponding to an ADAM-H9 polypeptide is mapped using PCR-based mapping strategies. Initial human chromosomal assignments are made using an ADAM-H9-specific PCR primers such as those described above in Example 1 and a BIOS Somatic Cell Hybrid PCRable DNA kit from BIOS Laboratories (New Haven, CT), following the manufacturer's instructions. More detailed mapping is performed using a Genebridge 4 Radiation Hybrid Panel (Research Genetics, Huntsville, AL (see, *e.g.,* Walter, MA *et al.,* Nature Genetics 7:22-28, 1994). Data from this analysis is then submitted electronically to the MIT Radiation Hybrid Mapper (http://www-genome.wi.mit.edu/cgi-bin/condg/rhmapper.pl) following the instructions contained therein. This analysis yields specific genetic marker names which, when submitted electronically to NCBI: (www-ncbi.nlm.nih.gov/genemap/map.cgi?CHR=8), yield the specific chromosome interval. The predicted chromosomal location is on chromosome 8 at approximately 8p11.1.

### EXAMPLE 5

### Generation of ADAM-H9dis.Fc and Activity of ADAM Disintegrin Domain Polypeptides In a Corneal Pocket Assay

To construct a polynucleotide encoding the ADAM-H9 extracellular domain fused to an Fc, a nucleic acid encoding amino acids residues 73 to 362 from SEQ ID NO:6, was joined to a nucleic acid encoding an F_{c} portion from human IgG1. The polypeptide encoded by this construct is shown in SEQ ID NO:25. This construct uses the igKappa leader, which is cleaved by the signal peptidase after the C-terminal G (Glycine) amino acid at position 20 of SEQ ID NO:25. The soluble form of the molecule is then predicted to start at amino acid 21 of SEQ ID NO:25. The TS (Threonine-Serine) sequence (amino acids 21 and 22 of SEQ ID NO:25) are a consequence of the restriction site used to link a disintegrin domain of SEQ ID NO:6 (amino acids 73 to 362 of SEQ ID NO:6) to the Fc domain. A disintegrin domain of SEQ ID NO:6 begins at amino acid 23 and continues to amino acid 312 of SEQ ID NO:25. The RS (Arginine-Serine) sequence at amino acids 313-314 is a consequence of the restriction site used to link a disintegrin domain of SEQ ID NO:6 (amino acids 73 to 362 of SEQ ID NO:6) to the Fc domain. The Fc sequence begins at amino acid 315 and continues to the in frame stop codon following residue 542.

A mouse corneal pocket assay is used to quantitate the inhibition of angiogenesis by ADAM-H9dis-Fc polypeptides *in vivo.* In this assay, agents to be tested for angiogenic or anti-angiogenic activity are immobilized in a slow release form in a hydron pellet, which is implanted into micropockets created in the corneal epithelium of anesthetized mice. Vascularization is measured as the appearance, density, and extent of vessel in growth from the vascularized corneal limbus into the normally avascular cornea.

Hydron pellets, as described in Kenyon *et al.,* Invest Opthamol. & Visual Science 37:1625, 1996, incorporate sucralfate with bFGF (90 ng/pellet), bFGF and IgG (11 µg/pellet, control), or bFGF and a range of concentrations of the agent to be tested (*e.g.*, ADAM-H9dis-Fc polypeptide). The pellets are surgically implanted into corneal stromal micropockets created by micro-dissection 1 mm medial to the lateral corneal limbus of 6-8 week old male C57BL mice. After five days, at the peak of neovascular response to bFGF, the corneas are photographed using a Zeiss slit lamp at an incipient angle of 35-50° from the polar axis in the meridian containing the pellet. Images are digitized and processed by subtractive color filters (Adobe Photoshop 4.0) to delineate established microvessels by hemoglobin content. Image analysis software (Bioquant, Nashville, TN) is used to calculate the fraction of the corneal image that is vascularized, the vessel density within the vascularized area, and the vessel density within the total cornea. The inhibition of bFGF-induced corneal angiogenesis, as a function of the dose of ADAM-H9 disintegrin-Fc polypeptide, is determined.

### EXAMPLE 6

### Inhibition of Neovascularization by ADAM Disintegrin Domain Polypeptides in a Murine Transplant Model

Survival of heterotopically transplanted cardiac tissue from one mouse donor to the ear skin of another genetically similar mouse requires adequate neovascularization by the transplanted heart and the surrounding tissue, to promote survival and energy for cardiac muscle function. Inadequate vasculature at the site of transplant causes excessive ischemia to the heart, tissue damage, and failure of the tissue to engraft. Agents that antagonize factors involved in endothelial cell migration and vessel formation can decrease angiogenesis at the site of transplant, thereby limiting graft tissue function and ultimately engraftment itself. A murine heterotopic cardiac isograft model is used to demonstrate the antagonistic effects of ADAM-H9dis-Fc polypeptides on neovascularization.

Female BALB/c (≈12 weeks of age) recipients are given neonatal heart grafts from donor mice of the same strain. The donor heart tissue is grafted into the left ear pinnae of the recipient on day 0 and the mice are divided into two groups. The control group receives human IgG (Hu IgG) while the other group receives ADAM-H9dis-Fc, both intraperitoneally. The treatments are continued for five consecutive days. The functionality of the grafts is determined by monitoring visible pulsatile activity on days 7 and 14 post-engraftment. The inhibition of functional engraftment, as a function of the dose of ADAM-H9dis-Fc, is determined. The histology of the transplanted hearts is examined is order to visualize the effects of ADAM-H9dis-Fc on edema at the site of transplant and host and donor tissue vasculature (using, *e.g.,* Factor VIII staining).

### EXAMPLE 7

### Treatment of Tumors with ADAM-H9 Disintegrin (ADAM-H9dis) Domain Polypeptides

ADAM-H9dis-Fc is tested in animal models of solid tumors. The effect of the ADAMdis-Fc is determined by measuring tumor frequency and tumor growth. The biological activity of ADAM-H9dis-Fc is also demonstrated in other *in vitro, ex vivo,* and *in vivo* assays known in the art, such as calcium mobilization assays and assays to measure platelet activation, recruitment, or aggregation.

## Claims

1. A polypeptide selected from the group consisting of:
(a) a polypeptide comprising an amino acid sequence of SEQ ID NO: 8 or SEQ ID NO:10;
(b) soluble fragments of the polypeptide of SEQ ID NO :6, SEQ ID NO: 8, or SEQ ID NO:10 having at least one activity selected from the group consisting of integrin binding activity, inhibition of endothelial cell migration, and inhibition of angiogenesis;
(c) fragments of the polypeptide of SEQ ID NO:6, SEQ ID NO:8, or SEQ ID NO:10 comprising a disintegrin domain amino acid sequence, said sequence corresponding to the sequence from amino acid residue 73±5 to any one of amino acid residues 360±5 to 362±5 of SEQ ID NO:6, from any one of amino acid residues 1 to 16±5 to any one of amino acid residues 285±5 to 287±5 of SEQ ID NO:8, and from any one of amino acid residues 1 to 73±5 to any one of amino acid residues 314±5 to 329±5, respectively;
(d) SEQ ID NO:6 from amino acid residue 73±5 to any one of amino acid residues 360±5 to 362±5;
(e) SEQ ID NO:8 from any one of amino acid residues 1 to 16±5 to any one of amino acid residues 285±5 to 287±5;
(f) SEQ ID NO:10 from any one of amino acid residues 1 to 73±5 to any one of amino acid residues 314±5 to 329±5;
(g) polypeptides having amino acid sequences sharing amino acid identity across the length of the amino acid sequences of SEQ ID NO: 8 or SEQ ID NO:10, wherein the percent amino acid identity is selected from the group consisting of at least 97.5%, at least 99%, and at least 99.5%; and
(h) a polypeptide comprising an amino acid sequence of SEQ ID NO:25.

2. A polypeptide according to claim 1 linked to a second polypeptide, wherein the second polypeptide is a leucine zipper polypeptide, an Fc polypeptide, or a peptide linker.

3. An isolated polynucleotide encoding a polypeptide of claim 1 or 2.

4. An isolated polynucleotide selected from the group consisting of
a) a polynucleotide comprising a sequence of SEQ ID NO:7 or SEQ ID NO:9;
b) SEQ ID NO:5 from nucleotide 248 to nucleotide 1111 of SEQ ID NO:5;
c) a polynucleotide comprising a sequence of SEQ ID NO:7 from a nucleotide between 82 and 127 to nucleotide 936;
d) a polynucleotide comprising a sequence of SEQ ID NO:9 from a nucleotide between 32 and 248 to a nucleotide between 973 and 1018;
(e) a nucleotide sequence complementary to a sequence of SEQ ID NO:7 or SEQ ID NO:9; and
(f) any of nucleotide sequences of a) to e) wherein T can also be U.

5. An isolated polynucleotide comprising a sequence of claim 4 operably linked to a polynucleotide encoding a polypeptide of interest.

6. An expression vector comprising a polynucleotide of claim 3, 4, or 5.

7. A recombinant host cell comprising a polynucleotide of claim 3, 4, or 5.

8. A method for producing a polypeptide of claim 1 or 2, comprising culturing the host cell of claim 7 under conditions promoting expression of the polypeptide of claims 1 or 2.

9. The method of claim 8, further comprising purifying the polypeptide.

10. A disintegrin polypeptide produced by culturing the host cell of claim 7 under conditions to promote expression of the polypeptide.

11. An antibody that specifically binds to a polypeptide of claim 1.

12. The antibody of claim 11, wherein the antibody is a monoclonal antibody.

13. The antibody of claim 11, wherein the antibody is a human or humanized antibody.

14. The antibody of claim 11, wherein the antibody inhibits the biological activity of the polypeptide of claim 1.

15. A method for identifying an agent that modulates an activity of a polypeptide of claim 1, comprising:
(a) contacting the agent with a polypeptide of claim 1 under conditions such that the agent and polypeptide interact; and
(b) determining the activity of the polypeptide in the presence of the agent compared to a control, wherein a change in activity is indicative of an agent that modulates the polypeptide's activity.

16. The method of claim 15, wherein the agent is selected from the group consisting of an antibody or a peptide.

17. A soluble ADAM-H9 disintegrin domain polypeptide comprising a fragment of the polypeptide of SEQ ID NO:6, SEQ ID NO:8, or SEQ ID NO:10 having at least one activity selected from the group consisting of integrin binding activity, inhibition of endothelial cell migration, and inhibition of angiogenesis, for use in therapy.

18. Use of a soluble ADAM-H9 disintegrin domain polypeptide comprising a fragment of the polypeptide of SEQ ID N0:6, SEQ ID NO:8, or SEQ ID NO:10 having at least one activity selected from the group consisting of integrin binding activity, inhibition of endothelial cell migration, and inhibition of angiogenesis, for the preparation of a pharmaceutical composition for inhibiting angiogenesis in a mammal in need of such treatment.

19. An *in vitro* method for modulating angiogenesis in a tissue, comprising contacting the tissue with a polypeptide of claim 1.

20. A polypeptide of claim 1 for use in therapy.

21. The use of a polypeptide of claim 1 for the preparation of a pharmaceutical composition for modulating angiogenesis in a tissue.

22. An *in vitro* method for modulating endothelial cell migration, comprising contacting an endothelial cell with a polypeptide of claim 1.

23. The use of a polypeptide of claim 1 for the preparation of a pharmaceutical composition for modulating endothelial cell migration.

24. An *in vitro* method of inhibiting the binding of an integrin to a ligand comprising contacting a cell that expresses the integrin with an effective amount of a soluble ADAM-H9 disintegrin domain polypeptide comprising a fragment of the polypeptide of SEQ ID NO: 6, SEQ ID NO: 8, or SEQ ID NO:10 having at least one activity selected from the group consisting of integrin binding activity, inhibition of endothelial cell migration, and inhibition of angiogenesis.

25. Use of a soluble ADAM-H9 disintegrin domain polypeptide comprising a fragment of the polypeptide of SEQ ID NO:6, SEQ ID NO:8, or SEQ ID NO:10 having at least one activity selected from the group consisting of integrin binding activity, inhibition of endothelial cell migration, and inhibition of angiogenesis, for the preparation of a pharmaceutical composition for treating a condition selected from the group consisting of ocular disorders; malignant and metastatic conditions; inflammatory diseases; osteoporosis, accelerated bone resorption disorders; restenosis; inappropriate platelet activation, recruitment, or aggregation; thrombosis; and a condition requiring tissue repair or wound healing.

26. The soluble ADAM-H9 disintegrin domain polypeptide of claim 17 for the use specified therein,wherein the soluble ADAM-H9 disintegrin domain polypeptide comprises a sequence selected from the group consisting of:
(a) SEQ ID NO:6 from amino acid residue 73±5 to any one of amino acid residues 360±5 to 362±5;
(b) SEQ ID NO:8 from any one of amino acid residues 1 to 16±5 to any one of amino acid residues 285±5 to or 287±5; and
(c) SEQ ID NO:10 from any one of amino acid residues 1 to 73±5 to any one of amino acid residues 314±5 to 329±5.

27. The soluble ADAM-H9 disintegrin domain polypeptide of claim 26, wherein the soluble ADAM-H9 disintegrin domain is in the form of a multimer.

28. The soluble ADAM-H9 disintegrin domain polypeptide of claim 27, wherein the multimer is a dimer or trimer.

29. The soluble ADAM-H9 disintegrin domain polypeptide of claims 27 or 28, wherein the multimer comprises an Fc polypeptide, a leucine zipper, or a peptide linker.

30. The soluble ADAM-H9 disintegrin domain polypeptide of claim 29, wherein the multimer comprises a sequence as set forth in SEQ ID NO:25.

31. The use of claims 18 or 25, wherein the soluble ADAM-H9 disintegrin domain polypeptide comprises a sequence selected from the group consisting of:
(a) SEQ ID NO:6 from amino acid residue 73±5 to any one of amino acid residues 360±5 to 362±5;
(b) SEQ ID NO:8 from any one of amino acid residues 1 to 16±5 to any one of amino acid residues 285±5 to or 287±5; and
(c) SEQ ID NO: 10 from any one of amino acid residues 1 to 73±5 to any one of amino acid residues 314±5 to 329±5.

32. The use of claim 31, wherein the soluble ADAM-H9 disintegrin domain is in the form of a multimer.

33. The use of claim 32, wherein the multimer is a dimer or trimer.

34. The use of claims 32 or 33, wherein the multimer comprises an Fc polypeptide, a leucine zipper, or a peptide linker.

35. The use of claim 34, wherein the multimer comprises a sequence as set forth in SEQ ID NO:25.

36. The method of claim 24, wherein the soluble ADAM-H9 disintegrin domain polypeptide comprises a sequence selected from the group consisting of:
(a) SEQ ID NO:6 from amino acid residue 73±5 to any one of amino acid residues 360±5 to 362±5;
(b) SEQ ID NO:8 from any one of amino acid residues 1 to 16±5 to any one of amino acid residues 285±5 to or 287±5; and
(c) SEQ ID NO:10 from any one of amino acid residues 1 to 73±5 to any one of amino acid residues 314±5 to 329±5.

37. The method of claim 36, wherein the soluble ADAM-H9 disintegrin domain is in the form of a multimer.

38. The method of claim 37, wherein the multimer is a dimer or trimer.

39. The method of claims 37 or 38, wherein the multimer comprises an Fc polypeptide, a leucine zipper, or a peptide linker.

40. The method of claim 39, wherein the multimer comprises a sequence as set forth in SEQ ID NO:25.

## Patentansprüche

1. Ein Polypeptid augewählt aus der Gruppe bestehend aus:
(a) einem Polypeptid umfassend eine Aminosäuresequenz SEQ ID NO:8 oder SEQ ID NO:10;
(b) löslichen Fragmenten des Polypeptids der SEQ ID NO:6, SEQ ID NO:8, oder SEQ ID NO:10, welche mindestens eine Aktivität ausgewählt aus der Gruppe bestehend aus Integrin-Bindungsaktivität, Hemmung der Endothelialzellmigration und Hemmung der Angiogenese besitzen;
(c) Fragmenten des Polypeptids der SEQ ID NO:6, SEQ ID NO:8, oder SEQ ID NO:10 umfassend eine Disintegrindomäne-Aminosäuresequenz, wobei diese Sequenz einer Sequenz von Aminosäurerest 73±5 bis zu einem der Aminosäurereste 360±5 bis 362±5 der SEQ ID NO:6 entspricht, von einem der Aminosäurereste 1 bis 16±5 bis zu einem der Aminosäurereste 285±5 bis 287±5 der SEQ ID NO:8, oder von einem der Aminosäurereste 1 bis 73±5 bis zu einem der Aminosäurereste 314±5 bis 329±5;
(d) SEQ ID NO:6 von Aminosäurerest 73±5 bis zu einem der Aminosäurereste 360±5 bis 362±5;
(e) SEQ ID NO:8 von einem der Aminosäurereste 1 bis 16±5 bis zu einem der Aminosäurereste 285±5 bis 287±5;
(f) SEQ ID NO:10 von einem der Aminosäurereste 1 bis 73±5 bis zu einem der Aminosäurereste 314±5 bis 329±5;
(g) Polypeptiden von Aminosäuresequenzen, die die Aminosäureidentität über die Länge der Aminosäuresequenz der SEQ ID NO:8 oder SEQ ID NO: 10 teilen, wobei die Prozente Aminosäureidentität ausgewählt sind aus der Gruppe bestehend aus mindestens 97,5%, mindestens 99%, und mindestens 99,5%; und
(h) einem Polypeptid umfassend eine Aminosäuresequenz der SEQ ID NO:25.

2. Ein Polypeptid gemäß Anspruch 1, welches mit einem zweiten Polypeptid verbunden ist, wobei das zweite Polypeptide ein Leucin-Zipper-Polypeptid, ein Fc-Polypeptid, oder ein Peptid-Linker ist.

3. Ein isoliertes Polynukleotid, welches für ein Polypeptid gemäß Anspruch 1 oder 2 kodiert.

4. Ein isoliertes Polynukleotid ausgewählt aus der Gruppe bestehend aus:
a) einem Polynukleotid umfassend eine Sequenz der SEQ ID NO:7 oder SEQ ID NO:9;
b) SEQ ID NO:5 von Nukleotid 248 bis Nukleotid 1111 der SEQ ID NO:5;
c) einem Polynukleotid umfassend eine Sequenz der SEQ ID NO:7 von einem Nukleotid zwischen 82 und 127 bis Nukleotid 936;
d) einem Polynukleotid umfassend eine Sequenz der SEQ ID NO:9 von einem Nukleotid zwischen 32 und 248 bis zu einem Nukleotid zwischen 973 und 1018;
(e) einer Nukleotid-Sequenz, die komplementär ist zu einer Sequenz der SEQ ID NO:7 oder SEQ ID NO:9; und
(f) einer der Nukleotid-Sequenzen von a) bis e), wobei T auch U sein kann.

5. Ein isoliertes Polynukleotid umfassend die Sequenz gemäß Anspruch 4, welches funktionell mit einem Polynukleotid verbunden ist, welches für ein Polynukleotid von Interesse kodiert.

6. Ein Expressionsvektor umfassend ein Polynukleotid gemäß Anspruch 3, 4 oder 5.

7. Eine rekombinante Wirtszelle umfassend ein Polynukleotid gemäß Anspruch 3, 4 oder 5.

8. Ein Verfahren zur Herstellung eines Polypeptids gemäß Anspruch 1 oder 2, umfassend das Kultivieren der Wirtszelle gemäß Anspruch 7 unter Bedingungen, welche die Expression der Polypeptide gemäß Ansprüchen 1 oder 2 fördern.

9. Das Verfahren gemäß Anspruch 8, des weiteren umfassend das Reinigen des Polypeptids.

10. Ein Disintegrin-Polypeptid, welches durch das Kultivieren der Wirtszelle gemäß Anspruch 7 unter Bedingungen hergestellt wurde, welche die Expression des Polypeptids fördern.

11. Ein Antikörper, der spezifisch an ein Polypeptid gemäß Anspruch 1 bindet.

12. Der Antikörper gemäß Anspruch 11, wobei der Antikörper ein monoklonaler Antikörper ist.

13. Der Antikörper gemäß Anspruch 11, wobei der Antikörper ein menschlicher oder vermenschlichter Antikörper ist.

14. Der Antikörper gemäß Anspruch 11, wobei der Antikörper die biologische Aktivität des Polypeptids gemäß Anspruch 1 hemmt.

15. Ein Verfahren zur Identifizierung eines Mittels, das die Aktivität eines Polypeptids gemäß Anspruch 1 moduliert, umfassend:
(a) Kontaktieren des Mittels mit einem Polypeptid gemäß Anspruch 1 unter Bedingungen, dass das Mittel und das Polypeptid interagieren; und
(b) Feststellen der Aktivität des Polypeptides in Anwesenheit des Mittels im Vergleich zu einer Kontrolle, wobei eine Änderung der Aktivität ein Hinweis auf ein Mittel ist, das die Aktivität des Polypeptides moduliert.

16. Das Verfahren gemäß Anspruch 15, wobei das Mittel gewählt ist aus der Gruppe bestehend aus einem Antikörper oder einem Peptid.

17. Ein lösliches ADAM-H9 Disintegrindomäne-Polypeptid umfassend ein Fragment des Polypeptids der SEQ ID NO:6, SEQ ID NO:8, oder SEQ ID NO:10, welches mindestens eine Aktivität ausgewählt aus der Gruppe bestehend aus Integrin-Bindungsaktivität, Hemmung der Endothelialzellmigration und Hemmung der Angiogenese besitzt, zur Verwendung in der Therapie.

18. Verwendung eines löslichen ADAM-H9 Disintegrindomäne-Polypeptids umfassend ein Fragment des Polypeptids der SEQ ID NO:6, SEQ ID NO:8, oder SEQ ID NO: 10, welches mindestens eine Aktivität ausgewählt aus der Gruppe bestehend aus Integrin-Bindungsaktivität, Hemmung der Endothelialzellmigration und Hemmung der Angiogenese besitzt, zur Herstellung einer pharmazeutischen Zusammensetzung zur Hemmung der Angiogenese in einem Säugetier mit Bedarf an einer solchen Behandlung.

19. Ein *in vitro* Verfahren zur Modulierung der Angiogenese in einem Gewebe, umfassend Kontaktieren des Gewebes mit einem Polypeptid gemäß Anspruch 1.

20. Ein Polypeptid gemäß Anspruch 1 zur Verwendung in der Therapie.

21. Die Verwendung eines Polypeptids gemäß Anspruch 1 zur Herstellung einer pharmazeutischen Zusammensetzung zur Modulierung der Angiogenese in einem Gewebe.

22. Ein *in vitro* Verfahren zur Modulierung der Endothelialzellmigration, umfassend das Kontaktieren der Endothelialzelle mit einem Polypeptid gemäß Anspruch 1.

23. Die Verwendung eines Polypeptids gemäß Anspruch 1 zur Herstllung einer pharmazeutischen Zusammensetzung zur Modulierung der Endothelialzellmigration.

24. Ein *in vitro* Verfahren zur Hemmung der Bindung eines Integrins an einen Liganden, umfassend das in Kontaktbringen einer Zelle, welche das Integrin exprimiert, mit einer effektiven Menge eines löslichen ADAM-H9 Disintegrindomäne-Polypeptids umfassend ein Fragment des Polypeptids SEQ ID NO:6, SEQ ID NO:8, oder SEQ ID NO:10, welches mindestens eine Aktivität ausgewählt aus der Gruppe bestehend aus Integrin-Bindungsaktivität, Hemmung der Endothelialzellmigration und Hemmung der Angiogenese besitzt.

25. Verwendung eines löslichen ADAM-H9 Disintegrindomäne-Polypeptids umfassend ein Fragment des Polypeptids SEQ ID NO:6, SEQ ID NO:8, oder SEQ ID NO: 10, welches mindestens eine Aktivität ausgewählt aus der Gruppe bestehend aus Integrin-Bindungsaktivität, Hemmung der Endothelialzellmigration und Hemmung der Angiogenese besitzt, zur Herstellung einer pharmazeutischen Zusammensetzung zur Behandlung eines Zustandes ausgewählt aus der Gruppe bestehend aus Augenerkrankungen; malignen und metastasierenden Zuständen; entzündlichen Erkrankungen; Osteoporose, beschleunigte Kochenresorptionserkrankungen; Restenose; unangemessene Thrombozyten-Aktivierung, -Rekrutierung oder -Aggregation; Thrombose; und einem Zustand, welcher der Gewebe-Wiederherstellung oder Wundheilung bedarf.

26. Das lösliche ADAM-H9 Disintegrindomäne-Polypeptid gemäß Anspruch 17 zu der darin spezifizierten Verwendung, wobei das lösliche ADAM-H9 Disintegrindomäne-Polypeptid eine Sequenz umfasst ausgewählt aus der Gruppe bestehend aus:
(a) SEQ ID NO:6 von Aminosäurerest 73±5 bis zu einem der Aminosäurereste 360±5 bis 362±5;
(b) SEQ ID NO:8 von einem der Aminosäurereste 1 bis 16±5 bis zu einem der Aminosäurereste 285±5 bis oder 287±5; und
(c) SEQ ID NO:10 von einem der Aminosäurereste 1 bis 73±5 bis zu einem der Aminosäurereste 314±5 bis 329±5.

27. Das lösliche ADAM-H9 Disintegrindomäne-Polypeptid gemäß Anspruch 26, wobei das lösliche ADAM-H9 Disintegrindomäne-Polypeptid in Form eines Multimers vorliegt.

28. Das lösliche ADAM-H9 Disintegrindomäne-Polypeptid gemäß Anspruch 27, wobei das Multimer ein Dimeres oder Trimeres ist.

29. Das lösliche ADAM-H9 Disintegrindomäne-Polypeptid gemäß Ansprüchen 27 oder 28, wobei das Multimer ein Fc-Polypeptid, einen Leucin-Zipper, oder einen Peptid-Linker umfasst.

30. Das lösliche ADAM-H9 Disintegrindomäne-Polypeptid gemäß Anspruch 29, wobei das Multimer eine Sequenz wie in SEQ ID NO:25 dargelegt umfasst.

31. Die Verwendung gemäß Ansprüchen 18 oder 25, wobei das lösliche ADAM-H9 Disintegrindomäne-Polypeptid eine Sequenz umfasst, ausgewählt aus der Gruppe bestehend aus:
(a) SEQ ID NO:6 von Aminosäurerest 73±5 bis zu einem der Aminosäurereste 360±5 bis 362±5;
(b) SEQ ID NO:8 von einem der Aminosäurereste 1 bis 16±5 bis zu einem der Aminosäurereste 285±5 bis oder 287±5; und
(c) SEQ ID NO:10 von einem der Aminosäurereste 1 bis 73±5 bis zu einem der Aminosäurereste 314±5 bis 329±5.

32. Die Verwendung gemäß Anspruch 31, wobei die lösliche ADAM-H9 Disintegrindomäne in Form eines Multimers vorliegt.

33. Die Verwendung gemäß Anspruch 32, wobei das Multimer ein Dimeres oder Trimeres ist.

34. Die Verwendung gemäß Ansprüchen 32 oder 33, wobei das Multimer ein Fc-Polypeptid, einen Leucin-Zipper, oder einen Peptid-Linker umfasst.

35. Die Verwendung gemäß Anspruch 34, wobei das Multimer eine Sequenz wie in SEQ ID NO:25 dargelegt umfasst.

36. Das Verfahren gemäß Anspruch 24, wobei das lösliche ADAM-H9 Disintegrindomäne-Polypeptid eine Sequenz umfasst ausgewählt aus der Gruppe bestehend:
(a) SEQ ID NO:6 von Aminosäurerest 73±5 bis zu einem der Aminosäurereste 360±5 bis 362±5;
(b) SEQ ID NO:8 von einem der Aminosäurereste 1 bis 16±5 bis zu einem der Aminosäurereste 285±5 bis oder 287±5; und
(c) SEQ ID NO:10 von einem der Aminosäurereste 1 bis 73±5 bis zu einem der Aminosäurereste 314±5 bis 329±5.

37. Das Verfahren gemäß Anspruch 36, wobei die lösliche ADAM-H9 Disintegrindomäne in Form eines Multimers vorliegt.

38. Das Verfahren gemäß Anspruch 37, wobei das Multimer ein Dimeres oder Trimeres ist.

39. Das Verfahren gemäß Ansprüchen 37 oder 38, wobei das Multimer ein Fc-Polypeptid, einen Leucin-Zipper, oder einen Peptid-Linker umfasst.

40. Das Verfahren gemäß Anspruch 39, wobei das Multimer eine Sequenz wie in SEQ ID NO:25 dargelegt umfasst.

## Revendications

1. Polypeptide choisi dans le groupe constitué par :
(a) un polypeptide comprenant une séquence d'aminoacides de SEQ ID NO : 8 ou de SEQ ID NO : 10 ;
(b) des fragments solubles du polypeptide de SEQ ID NO : 6, de SEQ ID NO : 8, ou de SEQ ID NO : 10 ayant au moins une activité choisie dans le groupe constitué par l'activité de liaison à l'intégrine, l'inhibition de la migration des cellules endothéliales, et l'inhibition de l'angiogenèse ;
(c) des fragments du polypeptide de SEQ ID NO : 6, de SEQ ID NO : 8, ou de SEQ ID NO : 10 comprenant une séquence d'aminoacides de domaine désintégrine, ladite séquence correspondant à la séquence du résidu d'aminoacide 73 + 5 à l'un quelconque des résidus d'aminoacides 360 ± 5 à 362 ± 5 de SEQ ID NO : 6, de l'un quelconque des résidus d'aminoacides 1 à 16 ± 5 à l'un quelconque des résidus d'aminoacides 285 ± 5 à 287 ± 5 de SEQ ID NO : 8, et de l'un quelconque des résidus d'aminoacides 1 à 73 ± 5 à l'un quelconque des résidus d'aminoacides 314 ± 5 à 329 ± 5, respectivement ;
(d) SEQ ID NO : 6, du résidu d'aminoacide 73 ± 5 à l'un quelconque des résidus d'aminoacides 360 ± 5 à 362 ± 5 ;
(e) SEQ ID NO : 8, de l'un quelconque des résidus d'aminoacides 1 à 16 ± 5 à l'un quelconque des résidus d'aminoacides 285 ± 5 à 287 ± 5 ;
(f) SEQ ID NO : 10, de l'un quelconque des résidus d'aminoacides 1 à 73 ± 5 à l'un quelconque des résidus d'aminoacides 314 ± 5 à 329 ± 5 ;
(g) des polypeptides ayant des séquences d'aminoacides partageant une identité d'aminoacides sur la longueur des séquences d'aminoacides de SEQ ID NO : 8 ou de SEQ ID NO : 10, dans lesquelles le pourcentage d'identité d'aminoacides est choisi dans le groupe constitué par au moins 97,5 %, au moins 99 % et au moins 99,5 % ; et
(h) un polypeptide comprenant une séquence d'aminoacides de SEQ ID NO : 25.

2. Polypeptide selon la revendication 1, lié à un second polypeptide, ledit second polypeptide étant un polypeptide de fermeture éclair à leucine, un polypeptide Fc, ou un segment peptidique de liaison.

3. Polynucléotide isolé codant pour un polypeptide selon la revendication 1 ou 2.

4. Polynucléotide isolé choisi dans le groupe constitué par :
(a) un polynucléotide comprenant une séquence de SEQ ID NO : 7 ou de SEQ ID NO : 9 ;
(b) SEQ ID NO : 5, du nucléotide 248 au nucléotide 1111 de SEQ ID NO : 5 ;
(c) un polynucléotide comprenant une séquence de SEQ ID NO : 7 d'un nucléotide compris entre 82 et 127 jusqu'au nucléotide 936 ;
(d) un polynucléotide comprenant une séquence de SEQ ID NO : 9 d'un nucléotide compris entre 32 et 248 jusqu' à un nucléotide compris entre 973 et 1018 ;
(e) une séquence de nucléotides complémentaire d'une séquence de SEQ ID NO : 7 ou de SEQ ID NO : 9 ; et
(f) l'une quelconque des séquences de nucléotides a) à e) dans laquelle T peut également être U.

5. Polynucléotide isolé comprenant une séquence selon la revendication 4 opérationnellement liée à un polynucléotide codant pour un polypeptide d'intérêt.

6. Vecteur d'expression comprenant un polynucléotide selon la revendication 3, 4 ou 5.

7. Cellule hôte recombinante comprenant un polynucléotide selon la revendication 3, 4 ou 5.

8. Procédé pour la production d'un polypeptide selon la revendication 1 ou 2, comprenant la culture de la cellule hôte selon la revendication 7 dans des conditions favorisant l'expression du polypeptide selon les revendications 1 ou 2.

9. Procédé selon la revendication 8, comprenant en outre la purification du polypeptide.

10. Polypeptide désintégrine produit en cultivant la cellule hôte selon la revendication 7 dans des conditions visant à favoriser l'expression du polypeptide.

11. Anticorps qui se lie spécifiquement à un polypeptide selon la revendication 1.

12. Anticorps selon la revendication 11, ledit anticorps étant un anticorps monoclonal.

13. Anticorps selon la revendication 11, ledit anticorps étant un anticorps humain ou humanisé.

14. Anticorps selon la revendication 11, ledit anticorps inhibant l'activité biologique du polypeptide selon la revendication 1.

15. Procédé pour identifier un agent qui module une activité d'un polypeptide selon la revendication 1, comprenant :
(a) la mise en contact de l'agent avec un polypeptide selon la revendication 1 dans des conditions telles que l'agent et le polypeptide interagissent ; et
(b) la détermination de l'activité du polypeptide en présence de l'agent comparativement à un témoin, procédé dans lequel une modification dans l'activité est indicatrice d'un agent qui module l'activité du polypeptide.

16. Procédé selon la revendication 15, dans lequel l'agent est choisi dans le groupe constitué par un anticorps ou un peptide.

17. Polypeptide de domaine désintégrine ADAM-H9 soluble comprenant un fragment du polypeptide de SEQ ID NO : 6, de SEQ ID NO : 8, ou de SEQ ID NO : 10 ayant au moins une activité choisie dans le groupe constitué par l'activité de liaison à l'intégrine, l'inhibition de la migration des cellules endothéliales, et l'inhibition de l'angiogenèse, pour une utilisation en thérapie.

18. Utilisation d'un polypeptide de domaine désintégrine ADAM-H9 soluble comprenant un fragment du polypeptide de SEQ ID NO : 6, de SEQ ID NO : 8, ou de SEQ ID NO : 10 ayant au moins une activité choisie dans le groupe constitué par l'activité de liaison à l'intégrine, l'inhibition de la migration des cellules endothéliales, et l'inhibition de l'angiogenèse, pour la préparation d'une composition pharmaceutique destinée à inhiber l'angiogenèse chez un mammifère ayant besoin d'un tel traitement.

19. Procédé *in vitro* pour moduler l'angiogenèse dans un tissu, comprenant la mise en contact du tissu avec un polypeptide selon la revendication 1.

20. Polypeptide selon la revendication 1, pour une utilisation en thérapie.

21. Utilisation d'un polypeptide selon la revendication 1, pour la préparation d'une composition pharmaceutique destinée à moduler l'angiogenèse dans un tissu.

22. Procédé *in vitro* pour moduler la migration des cellules endothéliales, comprenant la mise en contact d'une cellule endothéliale avec un polypeptide selon la revendication 1.

23. Utilisation d'un polypeptide selon la revendication 1 pour la préparation d'une composition pharmaceutique destinée à moduler la migration des cellules endothéliales.

24. Procédé *in vitro* d'inhibition de la liaison d'une intégrine à un ligand comprenant la mise en contact d'une cellule qui exprime l'intégrine avec une quantité efficace d'un polypeptide de domaine désintégrine ADAM-H9 soluble comprenant un fragment du polypeptide de SEQ ID NO : 6, de SEQ ID NO : 8, ou de SEQ ID NO : 10 ayant au moins une activité choisie dans le groupe constitué par l'activité de liaison à l'intégrine, l'inhibition de la migration des cellules endothéliales, et l'inhibition de l'angiogenèse.

25. Utilisation d'un polypeptide de domaine désintégrine ADAM-H9 soluble comprenant un fragment du polypeptide de SEQ ID NO : 6, de SEQ ID NO : 8, ou de SEQ ID NO : 10 ayant au moins une activité choisie dans le groupe constitué par l'activité de liaison à l'intégrine, l'inhibition de la migration des cellules endothéliales, et l'inhibition de l'angiogenèse, pour la préparation d'une composition pharmaceutique destinée à traiter un état choisi dans le groupe constitué par des troubles oculaires ; des états malins et métastasiques ; des maladies inflammatoires ; l'ostéoporose, des troubles de résorption osseuse accélérée ; la resténose ; l'activation, le recrutement ou l'agrégation inapproprié(e) des plaquettes ; une thrombose ; et un état nécessitant une réparation tissulaire ou une cicatrisation.

26. Polypeptide de domaine désintégrine ADAM-H9 soluble selon la revendication 17, destiné à l'utilisation spécifiée ici, ledit polypeptide de domaine désintégrine ADAM-H9 soluble comprenant une séquence choisie dans le groupe constitué par :
(a) SEQ ID NO : 6, du résidu d'aminoacide 73 ± 5 jusqu'à l'un quelconque des résidus d'aminoacides 360 ± 5 à 362 ± 5 ;
(b) SEQ ID NO : 8, de l'un quelconque des résidus d'aminoacides 1 à 16 ± 5 jusqu'à l'un quelconque des résidus d'aminoacides 285 ± 5 à 287 ± 5 ;
(c) SEQ ID NO : 10, de l'un quelconque des résidus d'aminoacides 1 à 73 ± 5 jusqu'à l'un quelconque des résidus d'aminoacides 314 ± 5 à 329 ± 5.

27. Polypeptide de domaine désintégrine ADAM-H9 soluble selon la revendication 26, ledit domaine désintégrine ADAM-H9 soluble étant sous la forme d'un multimère.

28. Polypeptide de domaine désintégrine ADAM-H9 soluble selon la revendication 27, dans lequel le multimère est un dimère ou un trimère.

29. Polypeptide de domaine désintégrine ADAM-H9 soluble selon la revendication 27 ou 28, dans lequel le multimère comprend un polypeptide Fc, une fermeture éclair à leucine, ou un segment peptidique de liaison.

30. Polypeptide de domaine désintégrine ADAM-H9 soluble selon la revendication 29, dans lequel le multimère comprend une séquence comme établi dans SEQ ID NO : 25.

31. Utilisation selon les revendications 18 ou 25, dans laquelle le polypeptide de domaine désintégrine ADAM-H9 soluble comprend une séquence choisie dans le groupe constitué par :
(a) SEQ ID NO : 6, du résidu d'aminoacide 73 ± 5 jusqu'à l'un quelconque des résidus d'aminoacides 360 ± 5 à 362 ± 5 ;
(b) SEQ ID NO : 8, de l'un quelconque des résidus d'aminoacides 1 à 16 ± 5 jusqu'à l'un quelconque des résidus d'aminoacides 285 ± 5 à 287 ± 5 ; et
(c) SEQ ID NO : 10, de l'un quelconque des résidus d'aminoacides 1 à 73 ± 5 jusqu'à l'un quelconque des résidus d'aminoacides 314 ± 5 à 329 ± 5.

32. Utilisation selon la revendication 31, dans laquelle le domaine désintégrine ADAM-H9 soluble est sous la forme d'un multimère.

33. Utilisation selon la revendication 32, dans laquelle le multimère est un dimère ou un trimère.

34. Utilisation selon la revendication 32 ou 33, dans laquelle le multimère comprend un polypeptide Fc, une fermeture éclair à leucine, ou un segment peptidique de liaison.

35. Utilisation selon la revendication 34, dans laquelle le multimère comprend une séquence comme établi dans SEQ ID NO : 25.

36. Procédé selon la revendication 24, dans lequel le polypeptide de domaine désintégrine ADAM-H9 soluble comprend une séquence choisie dans le groupe constitué par :
(a) SEQ ID NO : 6, du résidu d'aminoacide 73 ± 5 jusqu'à l'un quelconque des résidus d'aminoacides 360 ± 5 à 362 ± 5 ;
(b) SEQ ID NO : 8, de l'un quelconque des résidus d'aminoacides 1 à 16 ± 5 jusqu'à l'un quelconque des résidus d'aminoacides 285 ± 5 à 287 ± 5 ; et
(c) SEQ ID NO : 10, de l'un quelconque des résidus d'aminoacides 1 à 73 ± 5 jusqu'à l'un quelconque des résidus d'aminoacides 314 ± 5 à 329 ± 5.

37. Procédé selon la revendication 36, dans lequel le domaine désintégrine ADAM-H9 soluble est sous la forme d'un multimère.

38. Procédé selon la revendication 37, dans lequel le multimère est un dimère ou un trimère.

39. Procédé selon les revendications 37 ou 38, dans lequel le multimère comprend un polypeptide Fc, une fermeture éclair à leucine, ou un segment peptidique de liaison.

40. Procédé selon la revendication 39, dans lequel le multimère comprend une séquence comme établi dans SEQ ID NO : 25.
